(19) Europäisches Patentamt
European Patent Office
Office européen des brevets

(11) EP 4 610 358 A1

(12) EUROPEAN PATENT APPLICATION
published in accordance with Art. 153(4) EPC

(43) Date of publication:
**03.09.2025 Bulletin 2025/36**

(21) Application number: **23882764.6**

(22) Date of filing: **27.10.2023**

(51) International Patent Classification (IPC):
***C12N 15/53*** *(2006.01)* ***C12M 1/40*** *(2006.01)*
***C12N 1/15*** *(2006.01)* ***C12N 1/19*** *(2006.01)*
***C12N 1/21*** *(2006.01)* ***C12N 5/10*** *(2006.01)*
***C12N 9/06*** *(2006.01)* ***C12N 11/00*** *(2006.01)*
***C12N 15/63*** *(2006.01)* ***C12Q 1/00*** *(2006.01)*
***C12Q 1/26*** *(2006.01)*

(52) Cooperative Patent Classification (CPC):
**C12M 1/40; C12N 5/10; C12N 9/0004; C12N 11/00; C12N 15/63; C12N 15/74; C12N 15/80; C12N 15/81; C12Q 1/00; C12Q 1/26**

(86) International application number:
**PCT/JP2023/038908**

(87) International publication number:
**WO 2024/090562 (02.05.2024 Gazette 2024/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.10.2022 JP 2022172293**

(71) Applicant: **Kikkoman Corporation**
**Noda-shi, Chiba 278-8601 (JP)**

(72) Inventors:
- **KUZE Keitaro**
  **Noda-shi, Chiba 278-8601 (JP)**
- **TODA Keita**
  **Noda-shi, Chiba 278-8601 (JP)**
- **ICHIYANAGI Atsushi**
  **Noda-shi, Chiba 278-8601 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) FAD-DEPENDENT GLUTAMATE DEHYDROGENASE

(57) Provided is a method for producing a glutamate dehydrogenase using FAD as a coenzyme. Also provided is an FAD type glutamate dehydrogenase comprising a predetermined amino acid substitution.

EP 4 610 358 A1

## Description

Technical Field

The present invention relates to an FAD type glutamate dehydrogenase, a method for producing the same, a method for using the same, and a composition or kit comprising the FAD type glutamate dehydrogenase.

Background Art

Previously, L-glutamate measurement was performed using L-glutamate decarboxylase and L-glutamate dehydrogenase. Glutamate dehydrogenases known so far are enzymes that use nicotinamide adenine dinucleotide (NAD) as coenzyme and are enzymes that generate ammonia. Conventional glutamate dehydrogenases are generally referred to as GLDH in academic literatures. In the present specification, for convenience, such conventional glutamate dehydrogenase (so-called GLDH), may be referred to as a NAD type glutamate dehydrogenase or NAD-GLDH since NAD is the coenzyme. The coenzyme NAD was necessary for measuring L-glutamic acid when using NAD-GLDH. NAD-GLDH is generally expressed as an apoenzyme. As such, NAD is usually externally added to the expressed NAD-GLDH.

Subsequently, a L-glutamate oxidase that acts only on L-glutamate from solid culture of *Actinomyces* was reported (Non Patent Literature 1). L-glutamate oxidase is generally referred to as GLOD. L-glutamate oxidase is encoded as one polypeptide having $\alpha$, y, and $\beta$ chains and two such polypeptides form a homodimer. This homodimer is cleaved by a protease and becomes the mature form. The mature form is a heterohexamer constituted by $\alpha_2\beta_2\gamma_2$.

A recombinantly expressed homodimer had weak activity as a L-glutamate oxidase, was inferior in substrate affinity to the original L-glutamate oxidase of *Actinomyces,* and was also unstable against heat. When subjected to protease treatment, this recombinantly expressed homodimer enzyme, was converted into the same structure as that of the original *Actinomyces* L-glutamate oxidase and also acquired enzyme chemical properties equivalent thereto (Non Patent Literature 2). A method of producing this precursor on a large scale using recombinant *Escherichia coli* and subjecting the precursor to protease treatment for maturation is used.

Patent Literature 1 describes an L-glutamate oxidase mutant. Patent Literature 1 has no description regarding the thermal stability of the prepared L-glutamate oxidase mutant.

Patent Literature 2 describes a dry composition of a L-glutamate oxidase comprising a disaccharide lactose.

Problems of detecting L-glutamic acid using a L-glutamate oxidase include protease treatment being necessary for the expression of the L-glutamate oxidase; the L-glutamate oxidase does not necessarily have sufficient stability, for example, thermal stability; and the system may be influenced by dissolved oxygen present therein. Furthermore, although the L-glutamate oxidase generates hydrogen peroxide by transferring an electron to an oxygen molecule, electron transfer was difficult at a potential lower than the redox potential of hydrogen peroxide.

Citation List

Patent Literature

Patent Literature 1: WO 2021/193598
Patent Literature 2: JP Patent Publication (Kokai) No. 2019-097420 A (JP Patent No. 6349452)

Non Patent Literature

Non Patent Literature 1: Kusakabe H et al., Agric. Biol. Chem., 47, 1323-1328 (1983)
Non Patent Literature 2: Arima J et al., J. Biochem., 134, 805-812 (2003)

Summary of Invention

Technical Problem

L-glutamate oxidase, because of using oxygen as an electron acceptor, is influenced by dissolved oxygen. As such, a method for measuring L-glutamic acid, which is not influenced or resistant to being influenced by dissolved oxygen is needed. Further, an enzyme for L-glutamic acid measurement having high thermal stability is needed. Further, a

convenient method of producing an enzyme for L-glutamic acid measurement without protease treatment is needed. Further, an enzyme for L-glutamic acid measurement capable of transferring an electron at a potential lower than the redox potential of hydrogen peroxide is needed.

An object of the present disclosure is to at least in part solve the problems described above.

Solution to the Problem

The present inventors carried out concentrated studies. As a result, the present inventors prepared, as one example, a glutamate dehydrogenase by introducing the amino acid substitution of the present disclosure into a glutamate oxidase, thereby completing the present invention including this as one embodiment thereof. The present inventors also prepared, as one example, a freeze-dried composition comprising GLOD or FAD-glutamate dehydrogenase and a trisaccharide, thereby completing the present invention including this as one embodiment thereof.

The present disclosure provides the following embodiments.

[1] A flavin adenine dinucleotide (FAD) type glutamate dehydrogenase, wherein the amino acid at the position corresponding to the 109th and/or 545th position of SEQ ID NO: 58 is substituted and the ratio (OD/DH) of oxidase activity (OD) to dehydrogenase activity (DH) of the polypeptide after the amino acid substitution is reduced compared to the polypeptide prior to the amino acid substitution.

[2] The FAD type glutamate dehydrogenase according to Embodiment 1, wherein

the amino acid at the position corresponding to the 109th position of SEQ ID NO: 58 is substituted with an amino acid residue selected from the group consisting of serine, tryptophan, glutamine, alanine, aspartic acid, glutamic acid, phenylalanine, arginine, histidine, lysine, threonine, asparagine, cysteine, glycine, proline, valine, leucine, isoleucine, and tyrosine, and/or

the amino acid at the position corresponding to the 545th position of SEQ ID NO: 58 is substituted with an amino acid residue selected from the group consisting of alanine, aspartic acid, glutamic acid, phenylalanine, arginine, histidine, lysine, serine, threonine, asparagine, glutamine, cysteine, glycine, proline, valine, isoleucine, methionine, tyrosine and tryptophan.

[3] The FAD type glutamate dehydrogenase according to Embodiment 1 or 2, wherein the amino acid at the position corresponding to the 425th position of SEQ ID NO: 58 is further substituted with an amino acid residue selected from the group consisting of glutamine, alanine, aspartic acid, glutamic acid, phenylalanine, arginine, histidine, lysine, serine, threonine, asparagine, cysteine, glycine, proline, valine, isoleucine, methionine, tyrosine and tryptophan.

[4] The FAD type glutamate dehydrogenase according to any of Embodiments 1 to 3, wherein the amino acid sequence of the region corresponding to the 459th to 507th positions of SEQ ID NO: 58 is further deleted and the FAD type glutamate dehydrogenase comprises glutamate dehydrogenase activity.

[5] The FAD type glutamate dehydrogenase according to any of Embodiments 1 to 4, wherein the amino acid sequence of the polypeptide prior to the amino acid substitution comprises an amino acid sequence identity of 90% or more with the amino acid sequence of SEQ ID NO: 58, SEQ ID NO: 59, SEQ ID NO: 12 or SEQ ID NO: 13.

[6] The FAD type glutamate dehydrogenase according to any of Embodiments 1 to 5, wherein

the FAD type glutamate dehydrogenase further comprises an amino acid substitution compared to the amino acid sequence of SEQ ID NO: 58, at one or more positions selected from the group consisting of
the position corresponding to the 186th position of SEQ ID NO: 58,
the position corresponding to the 87th position of SEQ ID NO: 58,
the position corresponding to the 103rd position of SEQ ID NO: 58,
the position corresponding to the 133rd position of SEQ ID NO: 58,
the position corresponding to the 297th position of SEQ ID NO: 58,
the position corresponding to the 376th position of SEQ ID NO: 58,
the position corresponding to the 393rd position of SEQ ID NO: 58,
the position corresponding to the 428th position of SEQ ID NO: 58,
the position corresponding to the 516th position of SEQ ID NO: 58,
the position corresponding to the 566th position of SEQ ID NO: 58,
the position corresponding to the 568th position of SEQ ID NO: 58,
the position corresponding to the 585th position of SEQ ID NO: 58, and
the position corresponding to the 615th position of SEQ ID NO: 58.

[7] The FAD type glutamate dehydrogenase according to Embodiment 6, wherein

the amino acid substitution at the position corresponding to the 186th position of SEQ ID NO: 58 is substitution with an amino acid selected from the group consisting of glycine, alanine, cysteine, leucine, methionine, phenylalanine, tyrosine, serine, threonine, asparagine, glutamine, histidine, aspartic acid and glutamic acid,
the amino acid substitution at the position corresponding to the 87th position of SEQ ID NO: 58 is substitution with tyrosine,
the amino acid substitution at the position corresponding to the 103rd position of SEQ ID NO: 58 is substitution with an amino acid selected from the group consisting of phenylalanine, leucine, valine, isoleucine and methionine,
the amino acid substitution at the position corresponding to the 133rd position of SEQ ID NO: 58 is substitution with an amino acid selected from the group consisting of leucine and tyrosine,
the amino acid substitution at the position corresponding to the 297th position of SEQ ID NO: 58 is substitution with an amino acid selected from the group consisting of leucine, valine, isoleucine and methionine,
the amino acid substitution at the position corresponding to the 376th position of SEQ ID NO: 58 is substitution with an amino acid selected from the group consisting of phenylalanine, leucine, isoleucine and methionine,
the amino acid substitution at the position corresponding to the 393rd position of SEQ ID NO: 58 is substitution with an amino acid selected from the group consisting of leucine, valine, isoleucine and methionine,
the amino acid substitution at the position corresponding to the 428th position of SEQ ID NO: 58 is substitution with an amino acid selected from the group consisting of tyrosine and methionine,
the amino acid substitution at the position corresponding to the 516th position of SEQ ID NO: 58 is substitution with phenylalanine,
the amino acid substitution at the position corresponding to the 566th position of SEQ ID NO: 58 is substitution with an amino acid selected from the group consisting of phenylalanine, leucine, valine and methionine,
the amino acid substitution at the position corresponding to the 568th position of SEQ ID NO: 58 is substitution with an amino acid selected from the group consisting of isoleucine and methionine,
the amino acid substitution at the position corresponding to the 585th position of SEQ ID NO: 58 is substitution with an amino acid selected from the group consisting of leucine and methionine, or
the amino acid substitution at the position corresponding to the 615th position of SEQ ID NO: 58 is substitution with leucine.

[8] The FAD type glutamate dehydrogenase according to Embodiment 7, wherein

the FAD type glutamate dehydrogenase comprises an amino acid sequence identity of 90% or more with the amino acid sequence of SEQ ID NO: 60,
the amino acid sequence of the region corresponding to the 459th to 507th positions of SEQ ID NO: 58 is deleted, and
the FAD type glutamate dehydrogenase comprises an amino acid substitution compared to the amino acid sequence of SEQ ID NO: 58, at one or more positions selected from the group consisting of
the position corresponding to the 186th position of SEQ ID NO: 58,
the position corresponding to the 87th position of SEQ ID NO: 58,
the position corresponding to the 103rd position of SEQ ID NO: 58,
the position corresponding to the 133rd position of SEQ ID NO: 58,
the position corresponding to the 297th position of SEQ ID NO: 58,
the position corresponding to the 376th position of SEQ ID NO: 58,
the position corresponding to the 393rd position of SEQ ID NO: 58,
the position corresponding to the 428th position of SEQ ID NO: 58,
the position corresponding to the 516th position of SEQ ID NO: 58,
the position corresponding to the 566th position of SEQ ID NO: 58,
the position corresponding to the 568th position of SEQ ID NO: 58,
the position corresponding to the 585th position of SEQ ID NO: 58, and
the position corresponding to the 615th position of SEQ ID NO: 58,
the amino acid substitution being any of the amino acid substitutions described in Embodiment 7, and
wherein the FAD type glutamate dehydrogenase comprises glutamate dehydrogenase activity.

[9] The FAD type glutamate dehydrogenase according to any of Embodiments 1 to 8, wherein the amino acid sequence of the region corresponding to the 671st to 690th positions of SEQ ID NO: 58 is further deleted.
[10] A composition, reagent, electrode, sensor or kit comprising the glutamate dehydrogenase according to any of Embodiments 1 to 9.
[11] A polynucleotide encoding the glutamate dehydrogenase according to any of Embodiments 1 to 9.

[12] A vector comprising the polynucleotide according to Embodiment 11.
[13] A host cell comprising the vector according to Embodiment 12.
[14] A method for producing a glutamate dehydrogenase comprising the steps of:

culturing the host cell according to Embodiment 13 to produce the glutamate dehydrogenase according to any of Embodiments 1 to 9, and
obtaining the produced glutamate dehydrogenase.

[15] A method for bringing the glutamate dehydrogenase according to any of Embodiments 1 to 9 or the composition, reagent, electrode, sensor or kit according to Embodiment 10 into contact with a sample containing glutamic acid to oxidize the glutamic acid contained in the sample.
[16] The method according to Embodiment 15, comprising detecting glutamic acid.
[17] An FAD type glutamate dehydrogenase, wherein

the amino acid at the position corresponding to the 109th, 545th and/or 425th position of SEQ ID NO: 58 is substituted and the ratio (OD/DH) of oxidase activity (OD) to dehydrogenase activity (DH) of the polypeptide after the amino acid substitution is reduced compared to the polypeptide prior to the amino acid substitution,
wherein the FAD type glutamate dehydrogenase further comprises a thermal stability improving mutation, whereby the thermal stability of the polypeptide after the amino acid substitution is improved compared to the polypeptide before the substitution, and
the FAD type glutamate dehydrogenase meets a condition selected from the group consisting of the following:

(i) when the amino acid sequence of the glutamate dehydrogenase is aligned with the amino acid sequence of SEQ ID NO: 58, the glutamate dehydrogenase comprises a substitution of the amino acid at the position corresponding to a position selected from the group consisting of the 186th, 87th, 103rd, 133rd, 297th, 376th, 393rd, 428th, 516th, 566th, 568th, 585th, and 615th positions of SEQ ID NO: 58,
(ii) the glutamate dehydrogenase of said (i), wherein the glutamate dehydrogenase consists of an amino acid sequence comprising a substitution, deletion or addition of one or several amino acids at a position other than the positions corresponding to the 186th, 87th, 103rd, 133rd, 297th, 376th, 393rd, 428th, 516th, 566th, 568th, 585th, and 615th positions of SEQ ID NO: 58,
(iii) with regard to the glutamate dehydrogenase of said (i) or (ii), the full length amino acid sequence thereof comprises an amino acid sequence identity of 70% or more, 80% or more, or 90% or more with the amino acid sequence of SEQ ID NO: 58 or SEQ ID NO: 1, and
(iv) with regard to the glutamate dehydrogenase of said (i) or (ii), the full length amino acid sequence thereof comprises an amino acid sequence identity of 70% or more, 80% or more, or 90% or more with the amino acid sequence of SEQ ID NO: 58 or SEQ ID NO: 1, the amino acid at the position corresponding to the 291st position of SEQ ID NO: 58 in the glutamate dehydrogenase is arginine, and the amino acid sequence of the positions corresponding to the 51st to 56th positions of SEQ ID NO: 58 is Gly-Xaa-Gly-Xaa-Xaa-Gly (wherein Xaa represents any amino acid).

[18] The FAD type glutamate dehydrogenase according to Embodiment 17, wherein

the amino acid at the position corresponding to the 109th position of SEQ ID NO: 58 is substituted with an amino acid residue selected from the group consisting of serine, tryptophan, glutamine, alanine, aspartic acid, glutamic acid, phenylalanine, arginine, histidine, lysine, threonine, asparagine, cysteine, glycine, proline, valine, leucine, isoleucine, and tyrosine, and/or
the amino acid at the position corresponding to the 545th position of SEQ ID NO: 58 is substituted with an amino acid residue selected from the group consisting of alanine, aspartic acid, glutamic acid, phenylalanine, arginine, histidine, lysine, serine, threonine, asparagine, glutamine, cysteine, glycine, proline, valine, isoleucine, methionine, tyrosine and tryptophan.

[19] The FAD type glutamate dehydrogenase according to Embodiment 17 or 18, wherein the amino acid at the position corresponding to the 425th position of SEQ ID NO: 58 is further substituted with an amino acid residue selected from the group consisting of glutamine, alanine, aspartic acid, glutamic acid, phenylalanine, arginine, histidine, lysine, serine, threonine, asparagine, cysteine, glycine, proline, valine, isoleucine, methionine, tyrosine and tryptophan.
[20] The FAD type glutamate dehydrogenase according to any of Embodiments 17 to 19, wherein

the amino acid substitution at the position corresponding to the 186th position of SEQ ID NO: 58 is substitution with an amino acid selected from the group consisting of glycine, alanine, cysteine, leucine, methionine, phenylalanine, tyrosine, serine, threonine, asparagine, glutamine, histidine, aspartic acid and glutamic acid,
the amino acid substitution at the position corresponding to the 87th position of SEQ ID NO: 58 is substitution with tyrosine,
the amino acid substitution at the position corresponding to the 103rd position of SEQ ID NO: 58 is substitution with an amino acid selected from the group consisting of phenylalanine, leucine, valine, isoleucine and methionine,
the amino acid substitution at the position corresponding to the 133rd position of SEQ ID NO: 58 is substitution with an amino acid selected from the group consisting of leucine and tyrosine,
the amino acid substitution at the position corresponding to the 297th position of SEQ ID NO: 58 is substitution with an amino acid selected from the group consisting of methionine, leucine, valine, and isoleucine,
the amino acid substitution at the position corresponding to the 376th position of SEQ ID NO: 58 is substitution with an amino acid selected from the group consisting of phenylalanine, leucine, isoleucine and methionine,
the amino acid substitution at the position corresponding to the 393rd position of SEQ ID NO: 58 is substitution with an amino acid selected from the group consisting of leucine, valine, isoleucine and methionine,
the amino acid substitution at the position corresponding to the 428th position of SEQ ID NO: 58 is substitution with an amino acid selected from the group consisting of tyrosine and methionine,
the amino acid substitution at the position corresponding to the 516th position of SEQ ID NO: 58 is substitution with phenylalanine,
the amino acid substitution at the position corresponding to the 566th position of SEQ ID NO: 58 is substitution with an amino acid selected from the group consisting of methionine, phenylalanine, leucine, and valine,
the amino acid substitution at the position corresponding to the 568th position of SEQ ID NO: 58 is substitution with an amino acid selected from the group consisting of isoleucine and methionine,
the amino acid substitution at the position corresponding to the 585th position of SEQ ID NO: 58 is substitution with an amino acid selected from the group consisting of leucine and methionine, and
the amino acid substitution at the position corresponding to the 615th position of SEQ ID NO: 58 is substitution with leucine.

[21] A dry composition comprising an FAD type glutamate dehydrogenase or GLOD and a trisaccharide.
[22] The dry composition according to Embodiment 21, wherein the trisaccharide content per 100 U of the FAD type glutamate dehydrogenase or GLOD is 0.10 mg or more and less than 50 mg.
[23] The dry composition according to Embodiment 21, wherein the amount of the trisaccharide contained in the dry composition is 1% by mass or more and less than 90% by mass.
[24] The dry composition according to any of Embodiments 21 to 23, wherein the trisaccharide is raffinose.

The present specification encompasses the contents disclosed in JP Patent Application No. 2022-172293, which is a priority document of the present application.

Advantages of the Invention

In a particular embodiment, one effect of the present invention is that a glutamate dehydrogenase using FAD as a coenzyme can be obtained.

Brief Description of Drawings

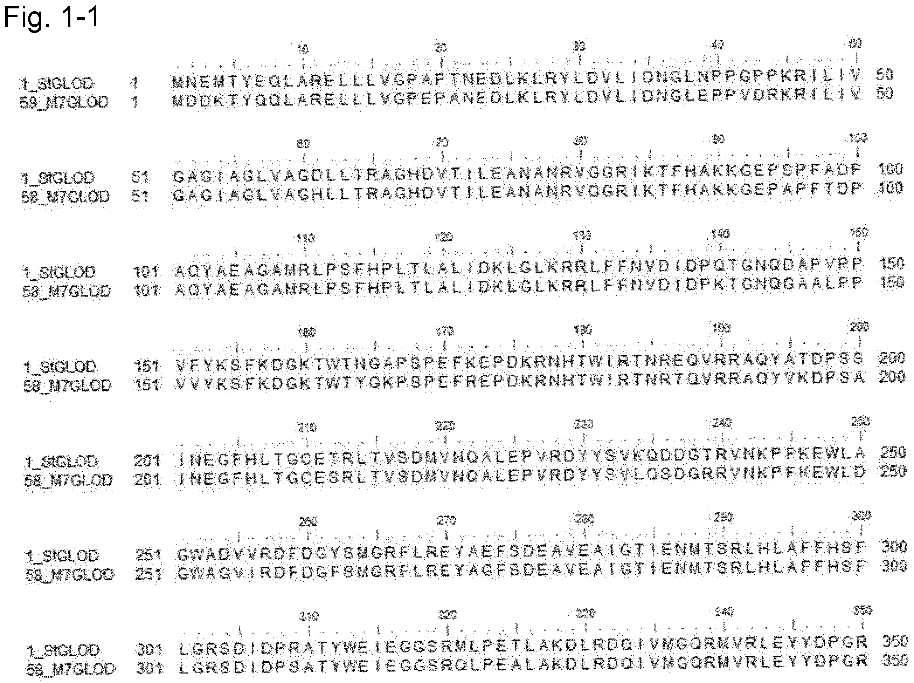

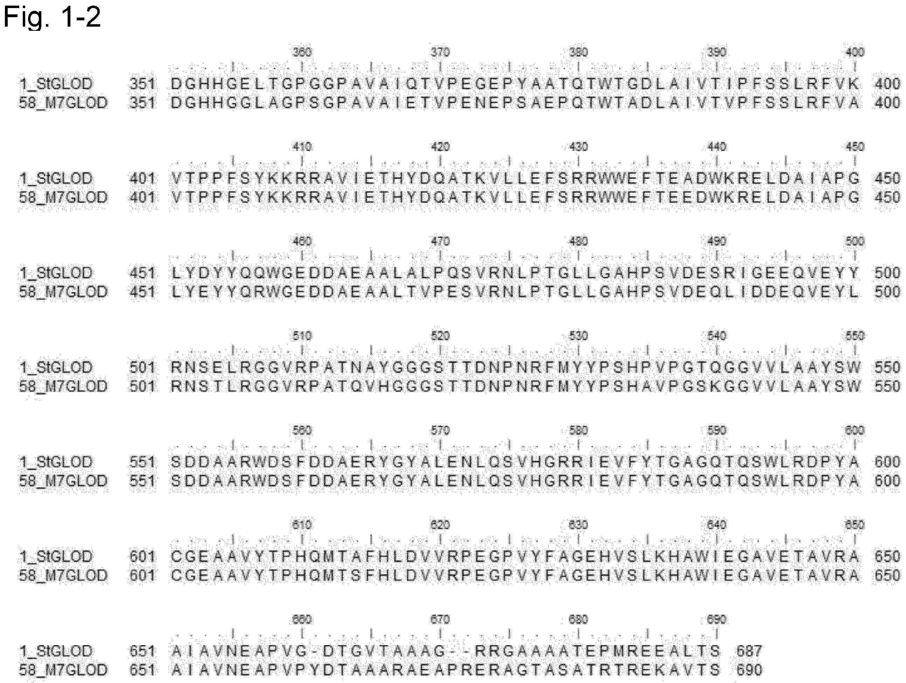

[Figure 1-1] Figure 1-1 is a diagram in which StGLOD of SEQ ID NO: 1 and M7GLOD of SEQ ID NO: 58 are aligned.
[Figure 1-2] Figure 1-2 is a diagram in which StGLOD of SEQ ID NO: 1 and M7GLOD of SEQ ID NO: 58 are aligned.
[Figure 2] Figure 2 shows the oxidase activity of GLOD and the dehydrogenase activity of FAD type GLDH.
[Figure 3] Figure 3 shows the amino acid sequence of GLOD of SEQ ID NO: 58. For convenience of explanation, individual units are hyphened. $\alpha$ chain (MDDK ... ENEP)-y chain (SAEP ... AEAA)-region 1 that is excised by a protease (LTVP ... STLR)-$\beta$ chain (GGVR ... RAEA)-region 2 that is excised by a protease (PRERAGTASATRTREKAVTS) is linked in this order.

Description of Embodiments

L-glutamate oxidase is encoded as one polypeptide having $\alpha$, $\gamma$, and $\beta$ chains by a GLOD gene and two such polypeptides form a homodimer. In nature, this homodimer is cleaved by a protease into the mature form. The mature form is a heterohexamer composed of $\alpha_2\beta_2\gamma_2$.

Unless specified otherwise, in the present specification, the term GLOD refers to L-glutamate oxidase. L-glutamate oxidase is an oxidoreductase classified as EC 1.4.3.11 and catalyzes the following chemical reaction.

[Formula 1] L-glutamate $+O_2 + H_2O \rightarrow$ 2-oxoglutaric acid + $NH_3 + H_2O_2$

GLOD uses flavin adenine dinucleotide (FAD) as a coenzyme. GLOD does not require externally adding FAD in order to exert enzyme activity. If FAD is present in a GLOD expression system, active GLOD is generated through incorporation of FAD when the polypeptide is folded. For example, in the case of recombinantly expressing GLOD in *Escherichia coli,* FAD from *Escherichia coli* is incorporated and active GLOD is obtained. Oxidoreductases using FAD as a coenzyme are known to have an FAD binding motif sequence, for example, a Gly-Xaa-Gly-Xaa-Xaa-Gly motif (wherein Xaa is any amino acid). For example, when taking SEQ ID NO: 58 as the standard sequence, "Gly-Ala-Gly-Ile-Ala-Gly", the amino acid sequence of the 51st to 56th positions of SEQ ID NO: 58, amounts to the FAD binding motif sequence represented by Gly-Xaa-Gly-Xaa-Xaa-Gly. In one embodiment, with regard to the mutant of the present disclosure, Gly at the positions corresponding to the 51st, 53rd, and 56th positions, respectively, when taking SEQ ID NO: 58 as the standard sequence, need not be subjected to amino acid substitution. In one embodiment, Gly at the positions corresponding to the 51st, 53rd, and 56th positions of SEQ ID NO: 58 in the L-glutamate oxidase are not subjected to amino acid substitution. For convenience, GLOD as used herein may also be referred to as FAD-GLOD. Unless specified otherwise, GLOD as used herein refers to GLOD using FAD as a coenzyme.

A GLOD is known to recognize the substrate glutamate through an arginine residue. For example, Arg (R) at the 291st position with reference to SEQ ID NO: 58 is an important position for recognizing glutamate. In an embodiment, with regard to the mutant of the present disclosure, the position corresponding to the 291st position of SEQ ID NO: 58 need not undergo an amino acid substitution. In an embodiment, with regard to the mutant of the present disclosure, when the amino acid at the position corresponding to the 291st position of SEQ ID NO: 58 is Arg (R), this amino acid is not substituted.

(Standard sequence)

In the present specification, for convenience, each position of an enzyme is defined by taking SEQ ID NO: 58 as the standard sequence. SEQ ID NO: 58 is a putative glutamate oxidase from *Streptomyces* sp. MOE7 (M7GLOD). However, the N terminal secretory signal sequence (MNDNGNGIRGEHTGVQR) of the naturally occurring sequence is removed. Further, in SEQ ID NO: 58, the 1st amino acid of the $\alpha$ chain region immediately after cleavage of the secretory signal sequence in wild type is altered from the wild type Ala to Met. The position of this Met is defined as the position corresponding to the 1st position (position 1) of SEQ ID NO: 58 in the present specification.

(Each subunit of GLOD)

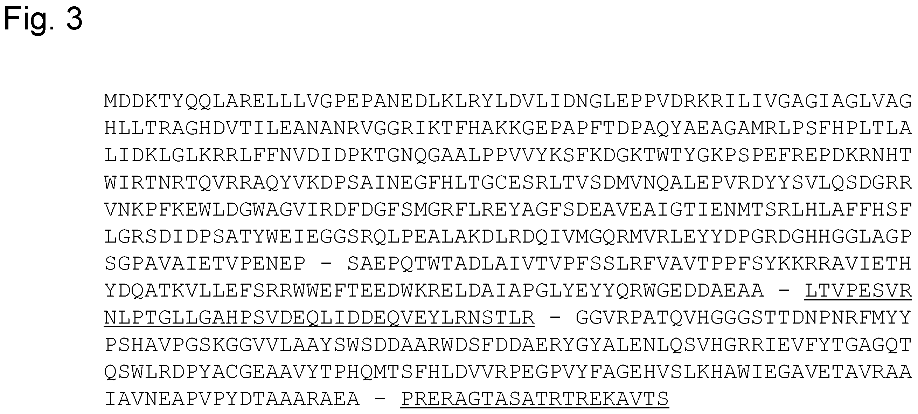

In the present specification, for convenience, the $\alpha$ chain of the GLOD is defined as the polypeptide constituted of 375 amino acid residues from the 1st to 375th positions (MDDK ... ENEP) with reference to SEQ ID NO: 58. In the present specification, for convenience, the y chain of the GLOD is defined as the polypeptide constituted of 91 amino acids from the 376th to 466th positions (SAEP ... AEAA) with reference to SEQ ID NO: 58. In the present specification, for convenience, the $\beta$ chain of the GLOD is defined as the polypeptide constituted of 164 amino acids from the 507th to 670th positions (GGVR ... RAEA) with reference to SEQ ID NO: 58. The region from the 467th to 506th positions with reference to SEQ ID NO: 58 is excised by a protease in nature (LTVP ... STLR). The C terminal sequence from the 671st to 690th positions (PRERAGTASATRTREKAVTS), with reference to SEQ ID NO: 58, is a region that is excised by a protease. See Figure 3.

The present inventors found that a glutamate dehydrogenase can be prepared by introducing the amino acid substitution of the present disclosure into a glutamate oxidase (GLOD). In the present specification, such a glutamate dehydrogenase may also be referred to as an FAD-GLDH or FAD type glutamate dehydrogenase. FAD-GLDH catalyzes the following chemical reaction.

[Formula 2] L-glutamate + oxidized form mediator + $H_2O \rightarrow$ 2-oxoglutaric acid + $NH_3$ + reduced form mediator

Examples of mediators include quinones, anthraquinones, phenazines, viologens, cytochromes, phenoxazines, phenylenediamines, phenothiazines, ferricyanides, ferricyanide such as potassium ferricyanide, ferredoxins, metal

complexes, ferrocene, ferrocenes, osmium complexes and derivatives thereof. Examples of phenazine compounds include, but are not limited to, phenazine methosulfate (PMS), methoxy PMS, PES (ethylphenazinium ethyl sulfate), thionines, naphthoquinones, benzoquinones, phthalocyanines, viologens, and benzylviologen. Examples of phenylenediamines include, but are not limited to, N,N'-diphenyl-p-phenylenediamine (DPPD), 4-isopropylaminodiphenylamine (IPPD), (1,3-dimethylbutyl)-N'-phenyl-p-phenylenediamine (6PPD), N,N,N',N'-tetramethyl-1,4-phenylenediamine, 2,3,5,6-tetramethyl-1,4-phenylenediamine, and N,N-dimethyl-p-phenylenediamine. Examples of mediators include, but are not limited to, brilliant cresyl blue, gallocyanin, safranine-O, resorufin, alizarine brilliant blue, phenothiazine, phenazine ethosulfate, dichlorophenol indophenol, ferrocene, benzoquinone, and phthalocyanine. In one embodiment, with regard to the mediator, a substrate can be quantified, for example, by adding 2,6-dichlorophenol indophenol (DCPIP) as an electron acceptor and monitoring a decrease in absorbance at 600 nm. The mediator may also be referred to as an artificial electron mediator, artificial electron acceptor, or electron mediator.

The FAD-GLDH may be prepared based on a GLOD. As the GLOD, a conventional GLOD or mutant thereof, for example, amino acid sequence deletion type GLOD mutant or thermally stable mutant, may be used.

(Amino acid substitutions of the present disclosure)

Amino acid substitutions of the present disclosure comprise a substitution of the amino acid residue at the position corresponding to the 545th position of SEQ ID NO: 58. In an embodiment, the position corresponding to the 545th position of SEQ ID NO: 58 may be substituted with alanine, aspartic acid, glutamic acid, phenylalanine, arginine, histidine, lysine, serine, threonine, asparagine, glutamine, cysteine, glycine, proline, valine, isoleucine, methionine, tyrosine or tryptophan. Since the 545th position of SEQ ID NO: 58 is L, for convenience, a substitution at this position with A may also be referred to L545A with regard to M7GLOD. The 545th position of SEQ ID NO: 58 corresponds to the 545th position of SEQ ID NO: 1.

Amino acid substitutions of the present disclosure comprise a substitution of the amino acid residue at the position corresponding to the 425th position of SEQ ID NO: 58. In one embodiment, the position corresponding to the 425th position of SEQ ID NO: 58 may be substituted with glutamine, alanine, aspartic acid, glutamic acid, phenylalanine, arginine, histidine, lysine, serine, threonine, asparagine, cysteine, glycine, proline, valine, isoleucine, methionine, tyrosine or tryptophan. Since the 425th position of SEQ ID NO: 58 is L, for convenience, a substitution at this position with Q may also be referred to L425Q with regard to M7GLOD. The 425th position of SEQ ID NO: 58 corresponds to the 425th position of SEQ ID NO: 1.

Amino acid substitutions of the present disclosure comprise a substitution of the amino acid residue at the position corresponding to the 109th position of SEQ ID NO: 58. In one embodiment, the position corresponding to the 109th position of SEQ ID NO: 58 may be substituted with serine, tryptophan, glutamine, alanine, aspartic acid, glutamic acid, phenylalanine, arginine, histidine, lysine, threonine, asparagine, cysteine, glycine, proline, valine, leucine, isoleucine, or tyrosine. Since the 109th position of SEQ ID NO: 58 is M, for convenience, a substitution at this position with S may also be referred to M109S with regard to M7GLOD. The 109th position of SEQ ID NO: 58 corresponds to the 109th position of SEQ ID NO: 1.

(Ratio of oxidase activity to dehydrogenase activity)

The ratio (DH/OD) of dehydrogenase activity (DH) to oxidase activity (OD) of the FAD-GLDH may be increased compared to the polypeptide before introduction of the amino acid substitution of the present disclosure. In one embodiment, the DH/OD of the FAD-GLDH may be increased by 5% or more, for example, 10% or more, 20% or more, 30% or more, 40% or more, 50% or more, 100% or more, 150% or more, 200% or more, 300% or more, 400% or more, 500% or more, 600% or more, 700% or more, 800% or more, 900% or more, 1000% or more, 1100% or more, 1200% or more, 1300% or more, 1400% or more, 1500% or more, 1600% or more, 1700% or more, 1800% or more, 1900% or more, 2000% or more, 3000% or more, 4000% or more, 5000% or more, 6000% or more, 8000% or more, 10000% or more, 13000% or more, 16000% or more, 19000% or more, 22000% or more, 25000% or more, 28000% or more, 32000% or more, 36000% or more, 41000% or more, 46000% or more, 50000% or more, 60000% or more, 70000% or more, 80000% or more, 100000% or more, 120000% or more, 140000% or more, 160000% or more, 180000% or more, 200000% or more, 220000% or more, for example, 250000% or more, compared to a polypeptide before introduction of the amino acid substitution of the present disclosure (DH/OD = 100%). Here, the phrase "increased by 5%" means that the DH/OD of a polypeptide after the amino acid substitution is 105% when the DH/OD of a polypeptide before the amino acid substitution is defined as 100%. In other words, the ratio (OD/DH) of oxidase activity (OD) to dehydrogenase activity (DH) of the FAD-GLDH may be decreased compared to a polypeptide before introduction of the amino acid substitution of the present disclosure. In one embodiment, the OD/DH of the FAD-GLDH may be decreased to 95% or less, 90% or less, 80% or less, 70% or less, 60% or less, 50% or less, 40% or less, 30% or less, 20% or less, 10% or less, 9% or less, 8% or less, 7% or less, 6% or less, 5% or less, 4% or less, 3% or less, 2% or less, for example, 1% or less, compared to a polypeptide before

introduction of the amino acid substitution of the present disclosure (OD/DH = 100%). Here, the phrase "decreased to 1% or less" means that the OD/DH of a polypeptide after the amino acid substitution is 1% when the OD/DH of a polypeptide before the amino acid substitution is defined as 100%. In one embodiment, no OD activity is observed in the FAD-GLDH having the amino acid substitution of the present disclosure introduced therein. As such, the OD/DH of this FAD-GLDH may be decreased to 0.1% or less, 0.01% or less, for example, 0%, compared to a polypeptide before introduction of the amino acid substitution of the present disclosure (OD/DH = 100%).

(Amino acid sequence deletion type GLOD mutant)

It was believed that in the amino acid sequence of the GLOD, the region to be excised by a protease interferes with exhibition of GLOD function. Accordingly, a GLOD mutant sequence, in which the amino acid sequence between the γ and β regions is deleted, was prepared and this was expressed. For example, when deleting the region to be excised by a protease from the GLOD of SEQ ID NO: 58, the last 8 amino acids (GEDDAEAA, the 459th to 466th positions) of the γ region were also removed from the mutant. Further, G at the 507th position, which is the first amino acid residue of the β chain, was also removed from the mutant. As such, the boundary between the γ chain region and β chain region of the prepared mutant was a sequence represented by γ chain region ... QRW-GVRP ... β chain region in which the C terminal QRW of the γ chain was connected to the N terminal GVRP of the β chain. As a result, a mutant exhibiting GLOD activity was obtained. This amino acid sequence deletion type GLOD mutant does not requires any protease treatment to exhibit activity. In an embodiment, the amino acid sequence deletion type GLOD mutant is not subjected to protease treatment when recombinantly expressed. FAD-GLDH may be prepared using such GLOD.

The present inventors further prepared connection region substituted type mutants by replacing said boundary sequence (y chain region ... QRW-GVRP ... β chain region) with various amino acid sequences. More specifically, a mutant M7GLODΔ49C having the amino acid sequence of SEQ ID NO: 59, a mutant StGLODΔ49Ai having the amino acid sequence of SEQ ID NO: 12, or a mutant StGLODΔ49C having the amino acid sequence of SEQ ID NO: 13 was prepared as a mutant comprising a modification of said boundary sequence (y chain region ... QRW-GVRP ... β chain region), and recombinant expression was carried out and the activity of the mutants were confirmed. These amino acid sequence deletion type GLOD mutants also did not require any protease treatment to exhibit activity. FAD-GLDH may be prepared using such GLOD.

In an embodiment, the present disclosure provides a glutamate dehydrogenase wherein the region corresponding to the 459th to 507th positions of SEQ ID NO: 58 is deleted and the glutamate dehydrogenase comprises an amino acid substitution at the position corresponding to the 545th, 425th and/or 109th position of SEQ ID NO: 58. In a particular embodiment, in the FAD-GLDH, the region corresponding to the 671st to 690th positions may be further deleted.

(Thermal stability improving effect)

In the case of subjecting these amino acid sequence deletion type FAD-GLDHs to heat treatment, the residual activities may be improved compared to their corresponding enzymes before the amino acid sequence deletion. Residual activity refers to the value of activity of an FAD-GLDH sample after heat treatment when the activity of a refrigerated (for example, 4°C) FAD-GLDH sample is defined as 1. The residual activity may be a value of 0 or more and 1 or less but also may be a value that exceeds 1 when the FAD-GLDH is activated by heat treatment. In the present specification, mutants comprising the mutation of the present disclosure and comprising a deletion of the amino acid sequence between the γ and β chains may also be referred to as inter-γ-β chain amino acid sequence deletion type FAD-GLDH mutants or simply as amino acid sequence deletion type FAD-GLDH mutants. The amino acid sequence deletion type FAD-GLDH mutant may also be referred to as "FAD-GLDHΔ". The number of deleted amino acid residues, XX, may be recited following the FAD-GLDHΔ (e.g., FAD-GLDHΔXX). For example, an FAD-GLDH mutant in which 49 amino acid residues are deleted may also be referred to as FAD-GLDHΔ49. As used herein, the term amino acid sequence deletion type FAD-GLDH mutant encompasses not only StFAD-GLDH and M7FAD-GLDH but also their variants, and FAD-GLDH mutants based on GLODs of other origins in which the region corresponding to the deleted region of SEQ ID NO: 1, SEQ ID NO: 58, or SEQ ID NO: 12 or 13 is deleted. The amino acid sequence deletion type GLOD mutant may also be referred to as "GLODΔ". The number of deleted amino acid residues, XX, may be recited following the GLODΔ (e.g., GLODΔXX). For example, a GLOD mutant in which 49 amino acid residues are deleted may also be referred to as GLODΔ49. In the present specification, the term amino acid sequence deletion type GLOD mutant encompasses not only StGLOD and M7GLOD but also their variants and GLOD mutants of other origins in which the region corresponding to the deleted region of SEQ ID NO: 1, SEQ ID NO: 58, or SEQ ID NO: 12 or 13 is deleted.

In an embodiment, with regard to the FAD-GLDH mutant of the present disclosure, the residual activity after heat treatment of the modified FAD-GLDH mutant may be improved by, for example, 10% or more, 20% or more, 30% or more, 40% or more, 50% or more, 60% or more, 70% or more, 80% or more, 90% or more, 100% or more, 150% or more, 200% or more, 250% or more, 300% or more, 350% or more, for example, 400% or more when the residual activity after heat

treatment of an FAD-GLDH before modification is defined as 100%. Here, the phrase "the residual activity is improved by 10%" means that the residual activity (dehydrogenase activity) after heat treatment of the modified enzyme is 110% when the residual activity (dehydrogenase activity) after heat treatment of an enzyme before modification is defined as 100%.

In one embodiment, with regard to the GLOD, the residual activity after heat treatment of the modified GLOD may be improved by, for example, 10% or more, 20% or more, 30% or more, 40% or more, 50% or more, 60% or more, 70% or more, 80% or more, 90% or more, 100% or more, 150% or more, 200% or more, 250% or more, 300% or more, 350% or more, for example, 400% or more when the residual activity after heat treatment of a GLOD before modification is defined as 100%. Here, the phrase "the residual activity is improved by 10%" means that the residual activity (oxidase activity) after heat treatment of the modified enzyme is 110% when the residual activity (oxidase activity) after heat treatment of an enzyme before modification is defined as 100%.

In one embodiment, thermal stability may be evaluated from residual activity after heat treatment at 45°C, 50°C, 60°C, 65°C or 70°C for 30 minutes or 35 minutes. In one embodiment, the residual activity (dehydrogenase activity) of an FAD-GLDHΔ mutant in which the region corresponding to the 459th to 507th positions of SEQ ID NO: 58 is deleted after being subjected to heat treatment at 45°C, 50°C, 60°C, 65°C or 70°C for 30 minutes or 35 minutes may be 105% or more, 110% or more, 120% or more, 130% or more, 140% or more, 150% or more, 200% or more, 300% or more, for example, 400% or more when the residual activity of an FAD-GLDH in which all or part of the 459th to 507th positions of SEQ ID NO: 58 is not deleted after being subjected to the same treatment as above is defined as 100%. In another embodiment, the residual activity (dehydrogenase activity) of an FAD-GLDHΔ mutant in which the region corresponding to the 459th to 507th positions of SEQ ID NO: 58 is deleted after being subjected to heat treatment at 45°C, 50°C, 60°C, 65°C, or 70°C for 30 minutes or 35 minutes may be 105% or more, 110% or more, 120% or more, 130% or more, 140% or more, 150% or more, 200% or more, 300% or more, for example, 400% or more when the residual activity of an FAD-GLDH in which the amino acid sequence from the 459th to 466th positions of SEQ ID NO: 58 is not deleted after being subjected to the same treatment as above is defined as 100%. In a particular embodiment, in the mutant, the region corresponding to the 671st to 690th positions of SEQ ID NO: 58 may be further deleted.

The thermal stability of an FAD-GLDHΔ mutant in which the region corresponding to the 459th to 507th positions of SEQ ID NO: 58 is deleted, may be improved compared to an enzyme before modification. Those skilled in the art shall appreciate that the thermal stability of FAD-GLDHs of other origins or FAD-GLDHs based on GLODs of other origins will also be improved by deleting the amino acid sequence corresponding to the 459th to 507th positions of SEQ ID NO: 58 and these Δ mutants can be used in various reactions. The same applies to the region corresponding to the 671st to 690th positions of SEQ ID NO: 58.

In one embodiment, the present disclosure provides an FAD-GLDHΔ having an amino acid sequence identity of 90% or more, for example, 91% or more, 92% or more, 93% or more, 94% or more, 95% or more, 96% or more, 97% or more, 98% or more, for example, 99% or more with the amino acid sequence of SEQ ID NO: 59, 12, or 13, comprising a deletion of the region corresponding to the 459th to 507th positions of SEQ ID NO: 58, comprising an amino acid substitution at the position corresponding to the 545th, 425th and/or 109th position of SEQ ID NO: 58, and having glutamate dehydrogenase activity. In this FAD-GLDHΔ, the region corresponding to the 671st to 690th positions of SEQ ID NO: 58 may be further deleted.

The FAD-GLDH may be prepared based on the GLOD. GLODs are widely distributed in nature and can be obtained by searching for enzymes from sources of microorganisms, animals, or plants. With regard to microorganisms, GLODs can be obtained from, for example, *Actinomyces,* filamentous fungi, yeast, or bacteria. In the present specification, the origin of GLOD is not particularly limited, and GLOD means a GLOD from, for example, a microorganism belonging to the genus *Streptomyces* such as *Streptomyces* sp. X-119-6 and *Streptomyces* sp. MOE7, the genus *Azotobacter,* the genus *Embleya,* the genus *Kitasatospora,* the genus *Saccharothrix,* the genus *Alloactinosynnema,* the genus *Streptoalloteichus,* the genus *Actinoalloteichus,* the genus *Catenulispora,* the genus *Nannocystis,* the genus *Actinobacteria,* the genus *Actinophytocola,* the genus *Sphaerisporangium,* the genus Microbispora, the genus *Streptosporangium,* the genus *Phytohabitans,* the genus *Haliangium,* the genus *Archangium,* the genus *Streptacidiphilus,* the genus *Saccharothrix* or the genus *Trichoderma,* and unless specified otherwise, includes all wild type and variants thereof.

(Obtaining a gene encoding GLOD)

In order to obtain a gene encoding a GLOD (hereinafter, also simply referred to as a "GLOD gene"), gene cloning methods carried out in the art in general can be used. For example, chromosomal DNA or mRNA can be extracted from microbial cells or various cells having GLOD productivity by routine methods. Further, cDNA can be synthesized using mRNA as the template. Using chromosomal DNA or cDNA thus obtained, a library of chromosomal DNA or cDNA can be prepared.

Next, an appropriate probe DNA can be synthesized based on the amino acid sequence of the GLOD above and a GLOD gene can be screened from the chromosomal DNA or cDNA library by using the probe DNA or, alternatively, appropriate primer DNA(s) can be prepared based on the amino acid sequence above and the DNA containing the gene

fragment of interest encoding the GLOD can be amplified with an appropriate polymerase chain reaction (PCR method) such as the 5'RACE method and the 3'RACE method, and then, these DNA fragments can be linked to obtain a DNA containing the full-length GLOD gene of interest.

Examples of the GLOD gene include, but are not limited to, a GLOD gene from *Streptomyces* sp. X-119-6 and a GLOD gene from *Streptomyces* sp. MOE7.

The GLOD gene may be linked to a vector. Vectors include any vector, such as a plasmid, bacteriophage, or cosmid vector, and an example thereof is pBluescriptII SK+ (manufactured by Stratagene Corporation). A plasmid may be obtained using conventional techniques. For example, a plasmid comprising a GLOD gene may be extracted and purified with the use of the GenElute Plasmid Miniprep Kit (manufactured by Sigma-Aldrich). The obtained GLOD gene may be engineered to prepare a GLOD mutant gene or a purified enzyme can be obtained.

Subsequently, the GLOD gene may be engineered to obtain an FAD-GLDH gene. In one embodiment, the GLOD gene may be engineered so that an amino acid substitution (for example, but not limited to, an amino acid substitution of Met with Ser) is introduced to the position corresponding to the 109th position of SEQ ID NO: 58 to prepare an FAD-GLDH gene. In one embodiment, the GLOD gene may be engineered so that an amino acid substitution (for example, but not limited to, an amino acid substitution of Leu with Ala) is introduced to the position corresponding to the 545th position of SEQ ID NO: 58 to prepare an FAD-GLDH gene. In one embodiment, the GLOD gene may be engineered so that an amino acid substitution (for example, but not limited to, an amino acid substitution of Leu with Gln) is introduced to the position corresponding to the 425th position of SEQ ID NO: 58 to prepare an FAD-GLDH gene.

(Treatment for inducing mutation of FAD-GLDH gene)

Mutation of the FAD-GLDH gene can be performed by any known method depending on the intended form of mutation. That is, methods of bringing a chemical agent serving as a mutagen into contact with and allowing to act on an FAD-GLDH gene or recombinant DNA comprising said gene integrated therein; ultraviolet irradiation methods; genetic engineering techniques; methods of employing protein engineering procedures can be used extensively.

Examples of the chemical agent serving as the mutagen used in the above mutation treatment include hydroxylamine, N-methyl-N'-nitro-N-nitrosoguanidine, nitrous acid, sulfurous acid, hydrazine, formic acid or 5-bromouracil and the like.

The conditions for allowing a chemical agent to contact and act can be determined depending on the type of chemical agent being used and the like and the conditions are not particularly limited as long as the mutation of interest can actually be induced in the FAD-GLDH gene. A mutation of interest can be induced usually by allowing a chemical agent preferably having a concentration of 0.5 to 12 M to contact and act on (the gene) at a reaction temperature of 20 to 80°C for 10 minutes or more and preferably 10 to 180 minutes. In the case of ultraviolet irradiation, a mutation can be induced with a routine method as mentioned above.

As a method of employing protein engineering procedures, in general, the method known as Site-Specific Mutagenesis can be used. Examples thereof include the Kramer method (Nucleic Acids Res., 12, 9441-9456 (1984)), the Eckstein method (Nucleic Acids Res., 13, 8749 -8764 (1985):Nucleic Acids Res., 13, 8765 (1985): Nucleic Acids Res, 14, 9679 (1986)) and the Kunkel method (Proc. Natl. Acid. Sci. U.S.A., 82, 488-492 (1985)).

Incidentally, apart from the above gene modifying methods, a modified FAD-GLDH gene of interest can be directly synthesized using organic synthesis methods or enzyme synthesis methods.

The nucleotide sequence of the FAD-GLDH gene may be confirmed using, for example, multi-capillary DNA analysis system Applied Biosystems 3730xlDNA analyzer (manufactured by Thermo Fisher Scientific Inc.).

(Transformation or transduction)

The FAD-GLDH gene can be integrated into a vector such as a bacteriophage, cosmid, or a plasmid for transformation of prokaryote cells or eukaryote cells according to routine methods. Using the resultant, the host cell corresponding to the vector may be transformed or transduced with routine methods.

In one embodiment, FAD-GLDH may be expressed using prokaryote cells, for example, microorganisms belonging to the genus *Escherichia* such as *Escherichia coli,* microorganisms belonging to the genus *Brevibacillus* such as *Brevibacillus choshinensis,* microorganisms belonging to the genus *Corynebacterium* such as *Corynebacterium glutamicum,* or microorganisms belonging to the genus *Streptomyces* such as *Streptomyces violaceoruber.* Examples of the *Escherichia coli* host include, but are not limited to, various *Escherichia coli* strains such as K-12 strain, JM109 strain, DH5$\alpha$ strain, BL21 strain, JM109(DE3) strain, DH5$\alpha$(DE3) strain, BL21(DE3) strain, TG1 strain, 1100 strain, W3110 strain and C600 strain. The host can be transformed or transduced to obtain host cells having the FAD-GLDH gene introduced therein (transformants). As a method for introducing a recombinant vector into a host cell, if the host cell is a microorganism belonging to *Escherichia coli,* a method of transferring recombinant DNA in the presence of a calcium ion can be employed. Furthermore, an electroporation method may be used. Moreover, commercially available competent cells (for example,

ECOS Competent *Escherichia coli* BL21 (DE3); manufactured by Nippon Gene Co., Ltd.) may be used. The GLOD gene may be a codon-optimized gene depending on the expression host.

In one embodiment, FAD-GLDH may be expressed using eukaryote cells. Examples of the eukaryotic host cell include yeast. Examples of microorganisms classified as yeast include yeasts belonging to the genus *Zygosaccharomyces,* the genus *Schizosaccharomyces,* the genus *Saccharomyces,* the genus *Pichia* and the genus *Candida.* The gene insert may contain a marker gene which enables selecting transformed cells. Examples of the marker gene include genes which compensate auxotrophy of a host cell, such as URA3 and TRP1. The gene insert may desirably contain a promoter enabling expression of the gene of interest in a host cell or other regulatory sequences (for example, enhancer sequence, terminator sequence, polyadenylation sequence and the like). Specific examples of the promoter include GAL1 promoter and ADH1 promoter. As methods for transforming yeast, known methods such as the method of using lithium acetate as well as electroporation can suitably be used although the transformation method is not limited thereto and various methods including the spheroplast method and glass bead method can be used for transformation.

Other examples of the eukaryotic host cell include mold cells (including filamentous fungi) such as those of the genus *Aspergillus* and the genus *Trichoderma.* The method for preparing a transformant of a mold cell is not particularly limited and includes, for example, a method of inserting a gene encoding an FAD-GLDH into a host filamentous fungus with routine methods such that the gene encoding the FAD-GLDH is expressed. More specifically, a DNA construct is prepared by inserting a gene encoding an FAD-GLDH between an expression inducing promoter and a terminator; and then, a host filamentous fungus is transformed with the DNA construct containing the gene encoding the FAD-GLDH to obtain transformants overexpressing the gene encoding the FAD-GLDH.

Methods for inserting a gene encoding an FAD-GLDH into a host filamentous fungus such that the gene is expressed are not particularly limited and include, for example, a method of directly inserting the gene into the chromosome of a host organism by using homologous recombination; and a method of ligating the gene to a plasmid vector and introducing the vector to a host filamentous fungus.

In the method using homologous recombination, a DNA construct is inserted into the genome of the host filamentous fungus by ligating the DNA construct between sequences homologous to the upstream region and downstream region of a recombination site on the chromosome. By overexpressing the gene under control of its own high expression promotor in the host filamentous fungus a transformant by self-cloning can be obtained. Examples of the high expression promoter include, but are not particularly limited to, the promoter region of TEF1 gene (tefl) serving as a translation elongation factor, the promoter region of α-amylase gene (amy) and the promoter region of an alkaline protease gene (alp).

In the method of using a vector, a DNA construct is integrated into a plasmid vector used in transformation of filamentous fungi by a routine method and then the corresponding host filamentous fungus can be transformed (with the plasmid vector) with a routine method.

Such suitable vector-host system is not particularly limited as long as the FAD-GLDH can be produced in the host filamentous fungus and includes, for example, pUC19 and filamentous fungus system, pSTA14 (Mol. Gen. Genet. 218, 99-104, 1989) and filamentous fungus system.

It is preferable to use the DNA construct by introducing the same into the chromosome of a host filamentous fungus. As an alternative method, the DNA construct can be integrated into an autonomous replicating vector (Ozeki et al. Biosci. Biotechnol. Biochem. 59, 1133 (1995)) and in this manner, the DNA construct can be used without being introduced into the chromosome.

The DNA construct may comprise a marker gene which enables a transformed cell to be selected. Examples of the marker gene include, but are not particularly limited to, genes compensating auxotrophy of a host such as pyrG, niaD, adeA; and drug resistance genes against chemical agents such as pyrithiamine, hygromycin B and oligomycin. Further, the DNA construct preferably comprises a promoter enabling overexpression of the gene encoding the FAD-GLDH in the host cell, a terminator and other regulatory sequences (for example, enhancer, polyadenylation sequence and the like). Examples of the promoter include, but are not particularly limited to, suitable expression induction promoters and constitutive promoters, such as the tefl promoter, alp promoter, amy promoter and the like. Examples of the terminator include, but are not particularly limited to, the alp terminator, amy terminator and tefl terminator and the like.

In the DNA construct, if the DNA fragment containing the gene encoding the FAD-GLDH to be inserted has a sequence having expression regulating function, then an expression regulatory sequence for the gene encoding the FAD-GLDH need not be required. When transformation is carried out by a co-transformation method, the DNA construct need not have a marker gene in some cases.

One embodiment of the DNA construct is e.g., a DNA construct prepared by ligating tefl gene promoter, a gene encoding FAD-GLDH, alp gene terminator and pyrG marker gene to the In-Fusion Cloning Site present in the multiple cloning site of pUC19.

As a method for transforming filamentous fungi, methods known to those skilled in the art can appropriately be selected and, for example, a protoplast PEG method can be used, in which a protoplast of a host filamentous fungus is prepared, and then, polyethylene glycol and calcium chloride are used (see, for example, Mol. Gen. Genet. 218, 99-104,

1989, JP Patent Publication (Kokai) No. 2007-222055A). As the culture medium for regenerating a transformed filamentous fungus, an appropriate culture medium is used depending on the host filamentous fungus to be used and the transformation marker gene. For example, if *Aspergillus soya* is used as the host filamentous fungus and pyrG gene is used as the transformation marker gene, the transformed filamentous fungus can be regenerated in Czapek-Dox minimal medium (Difco) containing for example, 0.5 % agar and 1.2 M sorbitol.

For example, to obtain the transformed filamentous fungus of the present invention, the promoter of the gene encoding the FAD-GLDH that the host filamentous fungus originally has in the chromosome may be substituted with a high expression promoter such as tefl, by using homologous recombination. In this case, a transformation marker gene such as pyrG is preferably inserted in addition to the high expression promoter. For example, for this purpose, a transformation cassette consisting of an upstream region of a gene encoding FAD-GLDH-transformation marker gene-high expression promoter-all or part of gene encoding FAD-GLDH, can be used (see, Example 1 and Figure 1 in JP Patent Publication (Kokai) No. 2011-239681A). In this case, the upstream region of a gene encoding FAD-GLDH and all or part of the gene encoding FAD-GLDH are used for homologous recombination. As all or part of the gene encoding FAD-GLDH, a region containing the initiation codon up to a midstream region can be used. The length of the region suitable for homologous recombination is preferably 0.5 kb or more.

Whether the transformed filamentous fungus of the present invention was produced or not can be confirmed by culturing the transformed filamentous fungus of the present invention under conditions where FAD-GLDH enzyme activity can be confirmed and then confirming the FAD-GLDH activity in a culture obtained after culturing.

Further, whether the transformed filamentous fungus of the present invention was produced or not can be confirmed by extracting chromosomal DNA from a transformed filamentous fungus, subjecting the chromosomal DNA to PCR using the chromosomal DNA as the template and confirming production of a PCR product that can be amplified if transformation took place.

For example, PCR is carried out by using a forward primer to the nucleotide sequence of the applied promoter in combination with a reverse primer to the nucleotide sequence of the transformation marker gene, and then, whether or not a product having the estimated length is obtained, is confirmed.

The host may be a known microorganism, a known strain and identicals or equivalents of a known microorganism or strain described in the present specification. Identicals refers to a host that exerts identical functions with regard to recombinant protein expression. Equivalents include modified hosts prepared based on a host known at the time of filing of the present application and includes those developed after filing of the present application and hosts that have properties similar to those of a host known at the time of filing of the present application which are discovered after filing of the present application. With regard to the academic name or classification of a microorganism, the description of the present specification should prevail if the academic name, genus name, classification or the like is changed after filing of the present application. Such a name or the like is based on that at the time of filing of the present application.

(High throughput screening)

An FAD-GLDH can further be subjected to high throughput screening in order to obtain a functional FAD-GLDH mutant. For example, a library of transformed strains or transduced strains comprising mutated FAD-GLDH genes can be prepared and then the library may be subjected to high throughput screening based on a microtiter plate or to ultrahigh-throughput screening based on droplet microfluids. As an example, a combinatorial library of mutant genes encoding variants is constructed and then a large population of modified FAD-GLDHs is screened by using, e.g., phage display (for example, Chem. Rev. 105 (11): 4056-72, 2005); yeast display (for example, Comb Chem High Throughput Screen. 2008; 11 (2): 127-34); bacterial display (for example, Curr Opin Struct Biol 17: 474-80, 2007) and the like. Also see, Agresti, et al., "Ultrahigh-throughput screening in drop-based microfluidics for directed evolution" Proceedings of the National Academy of Sciences 107 (9): 4004-4009 (Mar, 2010). The contents of this document on the ultrahigh-throughput screening method that may be used for screening FAD-GLDH variants is incorporated herein by reference. For example, a library can be constructed by an error-prone PCR method. Further, saturation mutagenesis may be used to introduce mutations into the region(s) or position(s) described herein or the corresponding region(s) or position(s) thereto as the target to construct a library. Using such library, appropriate cells such as electrocompetent EBY-100 cells, can be transformed and about 10 to the power of seven (10,000,000) mutants can be obtained. Yeast cells transformed with said library can subsequently be subjected to cell sorting. A polydimethoxysiloxane (PDMS) microfluidic device prepared by a standard soft lithography method may be used. Monodispersed droplets can be formed using a flow focus device. Formed droplets containing individual mutants can be subjected to an appropriate sorting device. When screening cells, the presence or absence of FAD-GLDH activity can be utilized. For this purpose, the reaction solution having a composition capable of developing color if FAD-GLDH functions, may, for example, be used. For example, in the case of using DCIP, absorbance at 600 nm may be measured using a 96 well plate, a 192 well plate, a 384 well plate or a 9600 well plate and a plate reader. Mutation and screening can be repeated a plurality of times. The term mutation used here includes an amino acid substitution, insertion, deletion, and/or addition.

For example, 1 to 10 mutations can be introduced into an FAD-GLDH and FAD-GLDH activity may be confirmed. Next, an FAD-GLDH mutant confirmed to have activity can be used as the starting material, further 1 to 10 mutations can be introduced thereinto and activity may be confirmed. A series of high throughput screening procedures (for example, the above method in which about 10 to the power of seven mutants are obtained and screened) may be repeated by 2 or more rounds, 5 or more rounds, 10 or more rounds, 15 or more rounds, for example, 20 or more rounds. High throughput screening to introduce 1 or more mutations, 5 or more mutations, for example, 10 or more mutations at each round may be repeated, for example, by 10 rounds to rapidly obtain mutants having 10 or more mutations, 50 or more mutations, for example, 100 or more mutations introduced in the starting FAD-GLDH and also having activity. Such high throughput screening may be repeated by 20 rounds to rapidly obtain mutants having 20 or more mutations, 100 or more mutations, for example, 200 or more mutations introduced into the starting FAD-GLDH and also having activity. These procedures may be performed by using an automatic apparatus or repeating a routine process.

Mutation(s) may be introduced into any one or more positions from the first amino acid to the last amino acid of the full length amino acid sequence of the FAD-GLDH. However, regions important for enzyme functions, such as the active center, substrate recognition sites, coenzyme recognition motifs and their neighborhoods are excluded. FAD-GLODs have been known for a long time and those skilled in the art have a thorough knowledge on the regions important for enzyme functions, including an active center, substrate recognition site and coenzyme recognition motif. Therefore, these findings may also be applied to the FAD-GLDH of the present disclosure. In a particular embodiment, for example, one or more mutations may first be introduced into the part from the 1st to 10th positions of the full length sequence of the FAD-GLDH. Next, an FAD-GLDH mutant confirmed to have activity can be used as the starting material, and then one or more mutations can be introduced into the 11th to 20th positions and activity may be confirmed. This may be repeated n times (n $\leq$ 68). For example, at the 68th round, one or more mutations may be introduced into the 680th to 687th positions. Regions important for enzyme functions or regions not intended to undergo modification may appropriately be skipped during the process. For example, the 51st to 56th positions of SEQ ID NO: 58 can be skipped. With this, it is possible to introduce a mutation into any position in the full length sequence except for the regions important for enzyme functions and FAD-GLDH mutants having, for example, 5 or more, 10 or more, 20 or more, 30 or more, 40 or more, 50 or more, 60 or more, 70 or more, 80 or more, 90 or more, 100 or more, 110 or more, 120 or more, 130 or more, 140 or more, 150 or more, 160 or more, 170 or more, 180 or more, 190 or more, for example, 200 or more mutations and having activity may be obtained rapidly.

Mutation(s) may be introduced at random or may be introduced by rational design. In one embodiment, the mutation to be introduced by rational design or the mutation to be introduced at random may be a conservative amino acid substitution. Conservative amino acid substitutions include an amino acid substitution by which the amino acid before the substitution and the amino acid after the substitution have similar chemical characteristics (for example, Stryer et al., Biochemistry, vol. 5, 2002, p. 44-49). The conservative amino acid substitution may be selected from the group consisting of, for example, (i) a substitution of a basic amino acid with a different type of basic amino acid; (ii) a substitution of an acidic amino acid with a different type of acidic amino acid; (iii) a substitution of an aromatic amino acid with a different type of aromatic amino acid; (iv) a substitution of a nonpolar aliphatic amino acid with a different type of nonpolar aliphatic amino acid; and (v) a substitution of a polar uncharged amino acid with a different type of polar uncharged amino acid. The basic amino acid may be selected from, for example, arginine, histidine, and lysine. The acidic amino acid may be, for example, aspartic acid or glutamic acid. The aromatic amino acid may be selected from, for example, phenylalanine, tyrosine and tryptophan. The nonpolar aliphatic amino acid may be selected from, for example, glycine, alanine, valine, leucine, methionine, proline and isoleucine. The polar uncharged amino acid may be selected from, for example, serine, threonine, cysteine, asparagine and glutamine. With regard to the conservative amino acid substitution, the amino acid residue before the substitution and the amino acid residue after the substitution have similar chemical characteristics and positions at which the conservative amino acid substitution is performed are considered to exist at the same positions in the three-dimensional structures of protein. Therefore, it is highly plausible that variants having the conservative amino acid substitution maintain the three-dimensional structures and have activity.

In one embodiment, the mutation to be introduced by rational design or the mutation to be introduced at random includes a substitution between functionally similar amino acids. The list of functionally similar amino acids is well known in the art. In one embodiment, with regard to the substitution between functionally similar amino acids, the amino acid before the substitution and the amino acid after the substitution may fall into any category of amino acid classification defined as follows:

1) glycine (G), alanine (A);
2) aspartic acid (D), glutamic acid (E);
3) asparagine (N), glutamine (Q);
4) arginine (R), lysine (K), histidine (H);
5) isoleucine (I), leucine (L), valine (V), proline (P);
6) phenylalanine (F), tyrosine (Y), tryptophan (W);
7) serine (S), threonine (T); and

8) cysteine (C), methionine (M).

In contrast to the conservative amino acid substitution, a nonconservative amino acid substitution is any amino acid substitution that does not correspond to the conservative substitutions (i) to (v) summarized above, for an amino acid. In one embodiment, the amino acid substitution may be a nonconservative amino acid substitution. In this case, for example, whether dehydrogenase activity is maintained before and after introduction of the nonconservative amino acid substitution may be confirmed. If the activity is confirmed, the nonconservative amino acid substitution may be adopted.

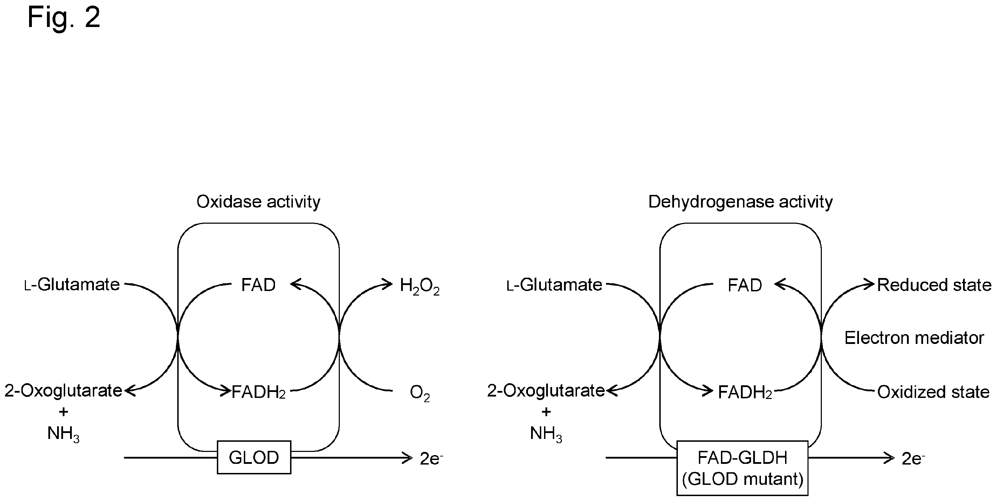

In one embodiment, the amino acid substitution may be a substitution between similar amino acids (similarity substitution). As used herein, the substitution between similar amino acids refers to a substitution between amino acids that are evaluated as positive values or neutral values (zero) in amino acid substitution matrices used in ClustalW software and Blosum62 algorithm, unless specified otherwise (see, for example, S. Heinkoff and J. G. Henikoff, Proc. Natl. Acad. Sci. USA, Vol. 89, pp. 10915-10919, 1992, particularly, Figure 2 therein, and Thompson, Nucleic Acid Research, 1994, Vol. 22, No. 22, pp. 4673-4680). The matrix list was generated from aligned sequence segments of about 2000 blocks of 500 or more related proteins. Iterative application leads to nearly the same set of scores, even starting with a unitary matrix or using a subset of the protein group. Therefore, it is believed that these substitution matrices are versatile. This is an approach used most widely, which exploits the fact that sequences having a homology are evolutionarily related. Therefore, it is highly plausible that variants having a similarity substitution introduced in a glutamate oxidase or glutamate dehydrogenase have activity. For example, in these substitution matrices, a substitution of serine with threonine is evaluated as the positive value "1" and thus, for example, a substitution of serine at the 212th position of SEQ ID NO: 58 with threonine corresponds to the substitution between similar amino acids. This position is threonine in a GLOD from *Streptomyces* sp. X-119-6 of SEQ ID NO: 1 and both enzymes have activity. Therefore, it is highly plausible that a S212T mutant of SEQ ID NO: 58 acts on glutamic acid. With regard to an FAD-GLDH having an amino acid substitution introduced at the 545th, 425th and/or 109th position of SEQ ID NO: 58, it is also highly plausible that a S212T mutant thereof acts on glutamic acid. The same applies to a mutant having T212S introduced in the FAD-GLDH having an amino acid substitution introduced at the 545th, 425th and/or 109th position of SEQ ID NO: 1. The same also applies to other similarity substitution variants.

In one embodiment, the conservative amino acid substitution or substitution between functionally similar amino acids does not reside in regions important for enzyme functions of the enzyme, such as the active center, substrate recognition sites, coenzyme recognition motifs and their neighborhoods and thus does not significantly influence the activity of the enzyme. In another embodiment, while the conservative amino acid substitution or substitution between functionally similar amino acids resides in the active center, substrate recognition sites, coenzyme recognition motifs and their neighborhoods, and the like in the enzyme, this does not substantially influence the activity of the enzyme.

The FAD-GLDH mutant may also comprise an insertion of an additional amino acid compared to a sequence before the mutation. In a typical embodiment, the amino acid insertion does not reside in regions important for enzyme functions, such as the active center, substrate recognition sites, coenzyme recognition motifs and their neighborhoods and thus does not significantly influence the activity of the enzyme. The FAD-GLDH mutant may also comprise an addition of an additional amino acid compared to a sequence before the mutation. In one embodiment, the amino acid addition is performed at the N terminus or C terminus of the FAD-GLDH and thus does not significantly influence the activity of the enzyme. Examples of addition include, but are not limited to, a short stretch of histidine residues (for example, 2 to 6 histidine residues) for assisting in the purification of the FAD-GLDH. Other examples of addition include, but are not limited to, an addition of a signal peptide for assisting in the expression of the FAD-GLDH. Examples of the signal peptide include known signal sequences or their functional equivalents.

The FAD-GLDH mutant may comprise a deletion of an amino acid compared to a sequence before the mutation. In a typical embodiment, the amino acid deletion does not reside in regions important for enzyme functions and thus does not significantly influence the activity of the enzyme. In one embodiment, the deletion may be a short deletion of 1 to 2 amino acids. In one embodiment, when the amino acid sequence of an FAD-GLDH is compared to the amino acid sequence of another FAD-GLDH and an amino acid is deleted in one of the sequences, this deletion may be introduced into the other FAD-GLDH. Since both the FAD-GLDHs exhibit activity, such a deletion is likely to have no significant impact on the activity of the enzyme. For example, when SEQ ID NO: 58 and SEQ ID NO: 1 are aligned, the 661st, 671st and 672nd positions of SEQ ID NO: 58 are deleted in SEQ ID NO: 1. Therefore, it is believed that deletion of the 661st, 671st and/or 672nd position from SEQ ID NO: 58 is likely to have no significant impact on the activity of the enzyme.

Mutation(s) may be introduced into an FAD-GLDH so as not to destroy a secondary structure or structural motif, such as an $\alpha$ helix structure or $\beta$ sheet structure. The region of the secondary structure may be identified with, for example, a secondary structure predicting algorithm. Examples of the predicting algorithm include, but are not limited to, NetSurfP - 2.0. The same applies to other structural motifs such as nest or niche.

In one embodiment, an amino acid residue or amino acid sequence motif essential for the activity of a glutamate dehydrogenase is not substituted. In another embodiment, even though a conservative amino acid substitution or similarity substitution can be performed at these positions, activity is confirmed with regard to a variant after the

substitution. In one embodiment, no amino acid is deleted or inserted at a position upstream or downstream of an amino acid residue essential for the activity of a glutamate dehydrogenase. The position upstream or downstream of an amino acid residue essential for the activity of a glutamate dehydrogenase refers to a position on an N-terminal or C-terminal side by one or two residues from the amino acid residue essential for the activity. In another embodiment, an amino acid can be deleted or inserted at a position upstream or downstream of an amino acid residue essential for the activity of a glutamate dehydrogenase. In this case, however, activity is confirmed with regard to a variant after the substitution. Whether or not the variant has activity may be routinely confirmed by, for example, high-throughput screening.

The present invention is carried out, unless specified otherwise, using chemical, biochemical, molecular biological, microbial, and immunological conventional techniques within the scope of an exercise of creativity of those skilled in the art. Such techniques are described in literatures. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D.

(1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press. These general texts are each incorporated herein by reference.

(Thermal stability improvement type mutation)

In one embodiment, the present disclosure provides an FAD-GLDH mutant having a thermal stability improvement type mutation introduced therein. The thermal stability of this mutant is improved compared to an FAD-GLDH before introduction of the mutation. In the present specification, with regard to the FAD-GLDH mutant of the present disclosure, improved thermal stability means that when an FAD-GLDH is subjected to heat treatment at a predetermined temperature for a predetermined time, the residual activity of the FAD-GLDH mutant after the heat treatment is improved compared to the residual activity after the heat treatment of the FAD-GLDH before introduction of the mutation.

In one embodiment, the FAD-GLDH mutant of the present disclosure comprises an amino acid substitution compared to SEQ ID NO: 58, at one or more positions, for example, 1, 2, 3, 4, 5, 6, 7, 8, 9, 10, 11, 12, or 13 positions, selected from the group consisting of

the position corresponding to the 186th position of SEQ ID NO: 58,
the position corresponding to the 87th position of SEQ ID NO: 58,
the position corresponding to the 103rd position of SEQ ID NO: 58,
the position corresponding to the 133rd position of SEQ ID NO: 58,
the position corresponding to the 297th position of SEQ ID NO: 58,
the position corresponding to the 376th position of SEQ ID NO: 58,
the position corresponding to the 393rd position of SEQ ID NO: 58,
the position corresponding to the 428th position of SEQ ID NO: 58,
the position corresponding to the 516th position of SEQ ID NO: 58,
the position corresponding to the 566th position of SEQ ID NO: 58,
the position corresponding to the 568th position of SEQ ID NO: 58,
the position corresponding to the 585th position of SEQ ID NO: 58, and
the position corresponding to the 615th position of SEQ ID NO: 58, and

the thermal stability of the mutant after the amino acid substitution is improved compared to the mutant before the substitution. With regard to the above positions, the phrase "amino acid is substituted (comprises an amino acid substitution)" means that the wild type amino acid is substituted with another amino acid and therefore, the wild type amino acid is excluded from amino acids after the substitution.

In one embodiment, with regard to the FAD-GLDH mutant of the present disclosure, the substituted amino acid (amino acid after the substitution) introduced into the position corresponding to the 186th position of SEQ ID NO: 58 may be selected from the group consisting of glycine, alanine, cysteine, leucine, methionine, phenylalanine, tyrosine, serine, threonine, asparagine, glutamine, histidine, aspartic acid and glutamic acid.

In one embodiment, with regard to the FAD-GLDH mutant of the present disclosure, the substituted amino acid (amino acid after the substitution) introduced into the position corresponding to the 87th position of SEQ ID NO: 58 may be tyrosine.

In one embodiment, with regard to the FAD-GLDH mutant of the present disclosure, the substituted amino acid introduced into the position corresponding to the 103rd position of SEQ ID NO: 58 may be selected from the group

consisting of phenylalanine, leucine, valine, isoleucine and methionine.

In one embodiment, with regard to the FAD-GLDH mutant of the present disclosure, the substituted amino acid introduced into the position corresponding to the 133rd position of SEQ ID NO: 58 may be selected from the group consisting of leucine and tyrosine.

In one embodiment, with regard to the FAD-GLDH mutant of the present disclosure, the substituted amino acid introduced into the position corresponding to the 297th position of SEQ ID NO: 58 may be selected from the group consisting of leucine, valine, isoleucine and methionine.

In one embodiment, with regard to the FAD-GLDH mutant of the present disclosure, the substituted amino acid introduced into the position corresponding to the 376th position of SEQ ID NO: 58 may be selected from the group consisting of phenylalanine, leucine, isoleucine and methionine.

In one embodiment, with regard to the FAD-GLDH mutant of the present disclosure, the substituted amino acid introduced into the position corresponding to the 393rd position of SEQ ID NO: 58 may be selected from the group consisting of leucine, valine, isoleucine and methionine.

In one embodiment, with regard to the FAD-GLDH mutant of the present disclosure, the substituted amino acid introduced into the position corresponding to the 428th position of SEQ ID NO: 58 may be selected from the group consisting of tyrosine and methionine.

In one embodiment, with regard to the FAD-GLDH mutant of the present disclosure, the substituted amino acid introduced into the position corresponding to the 516th position of SEQ ID NO: 58 may be phenylalanine.

In one embodiment, with regard to the FAD-GLDH mutant of the present disclosure, the substituted amino acid introduced into the position corresponding to the 566th position of SEQ ID NO: 58 may be selected from the group consisting of phenylalanine, leucine, valine and methionine.

In one embodiment, with regard to the FAD-GLDH mutant of the present disclosure, the substituted amino acid introduced into the position corresponding to the 568th position of SEQ ID NO: 58 may be selected from the group consisting of isoleucine and methionine.

In one embodiment, with regard to the FAD-GLDH mutant of the present disclosure, the substituted amino acid introduced into the position corresponding to the 585th position of SEQ ID NO: 58 may be selected from the group consisting of leucine and methionine.

In one embodiment, with regard to the FAD-GLDH mutant of the present disclosure, the substituted amino acid introduced into the position corresponding to the 615th position of SEQ ID NO: 58 may be leucine. The corresponding positions shall be described later.

In one embodiment, the thermal stability improvement type mutation above may be introduced into a wild type GLOD or conventional GLOD. A conventional GLOD, as mentioned herein, refers to a conventional type GLOD that requires protease treatment for expression of the active protein. It is believed that not only does a GLOD having the thermal stability improvement type mutation introduced therein exert activity after being treated with protease, as in the wild type GLOD or conventional type GLOD, but also thermal stability thereof is improved compared to the GLOD before introduction of the mutation. For convenience, such mutant may also be referred to as a GLOD-T in the present specification. Further, a plurality of such thermal stability improvement type mutations may be introduced. In the present specification, a GLOD having one thermal stability improvement type mutation above introduced therein may also be referred to as GLOD-T1. Likewise, a mutant having 2, 3, 4, 5, 6, 7, 8, 9, 10, 11, 12, or 13 such mutations introduced therein may also be referred to as GLOD-T2, GLOD-T3, GLOD-T4, GLOD-T5, GLOD-T6, GLOD-T7, GLOD-T8, GLOD-T9, GLOD-T10, GLOD-T11, GLOD-T12, or GLOD-T13, respectively.

In one embodiment, the amino acid substitution of the present disclosure, for example, the amino acid substitution at the position corresponding to the 545th, 425th and/or 109th position of SEQ ID NO: 58, may be introduced to a GLOD-T, thereby obtaining an FAD-GLDH-T with improved glutamate dehydrogenase activity. In another embodiment, the amino acid substitution of the present disclosure, for example, the amino acid substitution at the position corresponding to the 545th, 425th and/or 109th position of SEQ ID NO: 58, is introduced to a wild type GLOD or conventional type GLOD to obtain an FAD-GLDH, to which one or more thermal stability improvement type mutations may then be introduced to obtain an FAD-GLDH-T. It is believed that the thermal stability of an FAD-GLDH-T having a thermal stability improvement type mutation introduced therein is improved compared to an FAD-GLDH before introduction of the mutation. In the present specification, an FAD-GLDH having one thermal stability improvement type mutation above introduced therein may also be referred to as FAD-GLDH-T1. Likewise, a mutant having 2, 3, 4, 5, 6, 7, 8, 9, 10, 11, 12, or 13 such mutations introduced therein may also be referred to as FAD-GLDH-T2, FAD-GLDH-T3, FAD-GLDH-T4, FAD-GLDH-T5, FAD-GLDH-T6, FAD-GLDH-T7, FAD-GLDH-T8, FAD-GLDH-T9, FAD-GLDH-T10, FAD-GLDH-T11, FAD-GLDH-T12, or FAD-GLDH-T 13, respectively.

The present inventors confirmed that the thermal stability of a mutant having an amino acid substitution introduced at the position corresponding to the 186th, 87th, 103rd, 133rd, 297th, 376th, 393rd, 428th, 516th, 566th, 568th, 585th, or 615th position of SEQ ID NO: 58 is improved compared to an enzyme before modification. Based on these findings, those skilled in the art shall appreciate that the thermal stability of an FAD-GLDH having the mutation of the

present disclosure based on a GLOD of another origin will also be improved by introducing an identical amino acid substitution into the position corresponding to the 186th or 87th position or the like of SEQ ID NO: 58 and this FAD-GLDH can be used in various reactions.

In one embodiment, the thermal stability improvement type mutation above may be introduced into the amino acid sequence deletion type GLOD mutant (GLODΔ). The amino acid sequence deletion type GLOD mutant requires no protease treatment for expression of an active protein, unless otherwise specified. The thermal stability of the amino acid sequence deletion type GLOD mutant may be improved compared to the corresponding GLOD before the amino acid sequence deletion. The thermal stability improvement type mutation above may further be introduced into such a GLODΔ. For convenience, such a mutant may also be referred to as GLODΔ-T in the present specification. In the present specification, a GLODΔ having one thermal stability improvement type mutation introduced therein may also be referred to as GLODΔ-T1. Likewise, a GLODΔ mutant having 2, 3, 4, 5, 6, 7, 8, 9, 10, 11, 12, or 13 such mutations introduced therein may also be referred to as GLODΔ-T2, GLODΔ-T3, GLODΔ-T4, GLODΔ-T5, GLODΔ-T6, GLODΔ-T7, GLODΔ-T8, GLODΔ-T9, GLODΔ-T10, GLODΔ-T11, GLODΔ-T12, or GLODΔ-T13, respectively. The amino acid substitution of the present disclosure may be introduced to these enzymes to obtain an FAD-GLDH. A thermal stability improvement type mutation, amino acid deletion, and the amino acid substitution of the present disclosure may be introduced in any order to an enzyme.

In the present specification, an FAD-GLDHΔ having one thermal stability improvement type mutation introduced therein may also be referred to as FAD-GLDHΔ-T1. Likewise, an FAD-GLDHΔ mutant having 2, 3, 4, 5, 6, 7, 8, 9, 10, 11, 12, or 13 such mutations introduced therein may also be referred to as FAD-GLDHΔ-T2, FAD-GLDHΔ-T3, FAD-GLDHΔ-T4, FAD-GLDHΔ-T5, FAD-GLDHΔ-T6, FAD-GLDHΔ-T7, FAD-GLDHΔ-T8, FAD-GLDHΔ-T9, FAD-GLDHΔ-T10, FAD-GLDHΔ-T11, FAD-GLDHΔ-T12, or FAD-GLDHΔ-T13, respectively.

(Preliminary amino acid substitution)

In a preliminary embodiment, the FAD-GLDH, for example, a thermal stability improvement type mutant such as an FAD-GLDH-T or FAD-GLDH-T1 to FAD-GLDH-T13, an amino acid sequence deletion type FAD-GLDH mutant such as an FAD-GLDHΔ-T or FAD-GLDHΔ-T1 to FAD-GLDHΔ-T13, or an FAD-GLDH mutant having, for example, the amino acid sequence of SEQ ID NO: 12, 13, 58, 59 or 60 or an amino acid sequence having identity of 70% or more, 80% or more, or 90% or more with any of these may optionally have an amino acid substitution described in WO 2021/193598. For example, with regard to the FAD-GLDH mutant, Ala at the 106th position with reference to SEQ ID NO: 58 in the present specification may be substituted with Ser. This amino acid substitution is referred to as A106S in the present specification. Likewise, the FAD-GLDH mutant may also have one or more, for example, 1 to 25 amino acid substitutions selected from the group consisting of A106S, C210S, Q235E, D236E, D237E, P244H, T311S, W313F, Q333E, I334V, I334L, M336L, Q338E, R339K, T416S, A438P, K441E, Y455F, Q456R, Q457E, Q457K, L545I, P598A, C601S, and P609A with reference to SEQ ID NO: 58 in the present specification. The same applies to the positions corresponding to these positions.

For convenience, amino acid substitutions described in WO 2021/193598 may also be referred to as J for differentiating from the thermal stability improvement type amino acid deletion Δ of the present disclosure or the thermal stability improvement type mutation T of the present disclosure. That is, J is regarded as a set of the amino acid substitutions described in WO 2021/193598 and the order of the individual components is not limited.

J = {A106S, C210S, Q235E, D236E, D237E, P244H, T311S, W313F, Q333E, I334V, I334L, M336L, Q338E, R339K, T416S, A438P, K441E, Y455F, Q456R, Q457E, Q457K, L545I, P598A, C601S, P609A} [Mathematical formula 1]

A mutant having one of the mutations included in J is defined as J1 and mutants having n mutations are also defined as Jn ... and likewise, mutants up to J25 are defined in the same manner. In one embodiment, the present disclosure provides a combination mutant described below. Those skilled in the art are capable of preparing such finite combination mutants one by one and confirming their activity or thermal stability.

[Mathematical formula 2]

$$\text{FAD-GLDH-T} \cap \text{J}$$

[Mathematical formula 3]

$$\text{FAD-GLDH-T} \cap \{\text{J1 to J25}\}$$

[Mathematical formula 4]

$$\{\text{FAD-GLDH-T1 to FAD-GLDH-T13}\} \cap J$$

[Mathematical formula 5]

$$\{\text{FAD-GLDH-T1 to FAD-GLDH-T13}\} \cap \{\text{J1 to J25}\}$$

[Mathematical formula 6]

$$\text{FAD-GLDH}\Delta\text{-T} \cap J$$

[Mathematical formula 7]

$$\text{FAD-GLDH}\Delta\text{-T} \cap \{\text{J1 to J25}\}$$

[Mathematical formula 8]

$$\{\text{FAD-GLDH}\Delta\text{-T1 to FAD-GLDH}\Delta\text{-T13}\} \cap J$$

[Mathematical formula 9]

$$\{\text{FAD-GLDH}\Delta\text{-T1 to FAD-GLDH}\Delta\text{-T13}\} \cap \{\text{J1 to J25}\}$$

(GLOD mutant)

A GLODΔ mutant may be prepared by introducing the amino acid sequence deletion to a GLOD gene, and a GLOD, for example, a GLOD having high thermal stability, may be produced without carrying out protease treatment. A GLOD-T mutant may be prepared by introducing the amino acid substitution T to a GLOD gene, and a GLOD having high thermal stability may be produced. A GLODΔ-T mutant may be prepared by introducing the amino acid sequence deletion and amino acid substitution to a GLOD gene, and a GLOD, for example, a GLOD having high thermal stability, may be produced without carrying out protease treatment.

(FAD-GLDH mutant)

An FAD-GLDHΔ mutant may be prepared by introducing an amino acid sequence deletion to an FAD-GLDH gene, and an FAD-GLDH, for example, an FAD-GLDH having high thermal stability, may be produced without carrying out protease treatment. An FAD-GLDH-T mutant may be prepared by introducing an amino acid substitution T to an FAD-GLDH gene, and an FAD-GLDH having high thermal stability may be produced. An FAD-GLDHΔ-T mutant may be prepared by introducing an amino acid sequence deletion and amino acid substitution to an FAD-GLDH gene, and an FAD-GLDH, for example, an FAD-GLDH having high thermal stability, may be produced without carrying out protease treatment. A thermally stable GLOD may first be prepared, and the amino acid substitution of the present disclosure may be introduced to this.

In some limited embodiments, reverse mutations (back mutations) from the amino acid after substitution back to the amino acid in the sequence of the naturally occurring GLOD (i.e., the naturally occurring amino acid) are excluded. In other embodiments, the amino acid after substitution at the position corresponding to the 87th position or the like of SEQ ID NO: 58 can be identical to the amino acid at the position in the sequence of the naturally occurring GLOD (i.e., the naturally occurring amino acid).

(Corresponding position)

In the present specification, when a particular position in a reference amino acid sequence corresponds to a particular position in another amino acid sequence similar thereto, such position is referred to as a "corresponding position". Further, the amino acid at the corresponding position is referred to as the "corresponding amino acid." In the

present specification, for convenience, description shall be made with reference to the amino acid sequence of the putative glutamate oxidase from *Streptomyces* sp. MOE7 (M7GLOD) shown in SEQ ID NO: 58. In such a case, a "corresponding position" in an amino acid sequence is a position in the amino acid sequence of a GLOD derived from another organism species that corresponds to the particular position in the amino acid sequence of SEQ ID NO: 58.

A method of identifying a "corresponding position" of an amino acid sequence can be performed by, for example, comparing amino acid sequences using a known algorithm such as the Lipman-Pearson method to assign maximum identity to conserved amino acid residues present in the amino acid sequence of each GLOD. By aligning the amino acid sequences of the GLODs by such method, the positions of the homologous amino acid residues in each of the GLOD sequences can be determined, regardless of insertion or deletion of amino acid residues in the amino acid sequences. Corresponding positions (homologous positions) are considered to exist at the same positions in the three-dimensional structures, and amino acid residues at such homologous positions are expected to exert similar effects in terms of specific function of the GLOD of interest. An FAD-GLDH having the amino acid substitution of the present disclosure introduced in a GLOD can be similarly expected.

(Corresponding position of mutation)

In the present specification, the term "position corresponding to the 87th position of the amino acid sequence of SEQ ID NO: 58" refers to the position corresponding to the 87th position of SEQ ID NO: 58, when the amino acid sequence of the target polypeptide is compared with the amino acid sequence of SEQ ID NO: 58. The same applies to other positions, for example, the 103rd, 133rd, 186th, 297th, 376th, 393rd, 428th, 516th, 566th, 568th, 585th, 615th, 545th, 425th, and 109th positions, of SEQ ID NO: 58. The same applies to the 106th, 210th, 235th, 236th, 237th, 244th, 311th, 313th, 333rd, 334th, 336th, 338th, 339th, 416th, 438th, 441st, 455th, 456th, 457th, 545th, 598th, 601st, and 609th positions of SEQ ID NO: 58. For example, the position corresponding to the 87th position of the amino acid sequence of SEQ ID NO: 58 is the 87th position in SEQ ID NO: 1. Each sequence and its corresponding positional relationship are shown in Tables 14 and 15.

(Corresponding region)

The "corresponding region" in an amino acid sequence is also defined in a similar manner as described above with regard to a "corresponding position." For example, the region corresponding to the 459th to 507th positions of SEQ ID NO: 58 is from the 459th to 507th positions in SEQ ID NO: 1. The region corresponding to the 459th to 466th positions of SEQ ID NO: 58 is from the 459th to 466th positions in SEQ ID NO: 1. The region corresponding to the 671st to 690th positions of SEQ ID NO: 58 is from the 670th to 687th positions of SEQ ID NO: 1.

(Amino acid sequence homology, identity, or similarity)

Amino acid sequence homology, identity, or similarity can be calculated by a program such as Maximum Matching or Search Homology of GENETYX (manufactured by GENETYX), a program such as Maximum Matching or Multiple Alignment of DNASIS Pro (manufactured by Hitachi Solutions, Ltd.), or a program such as Multiple Alignment of CLUSTALW. In order to calculate amino acid sequence identity, two or more GLODs may be aligned, and the positions of identical amino acids in such two or more GLODs may be determined. The identical regions in amino acid sequences can be determined based on such information. The percent identity of two or more amino acid sequences is determined by subjecting two or more amino acid sequences to alignment using the algorithm such as Blosum62 by designating the total number of amino acids in the aligned region as the denominator and the number of identical amino acids relative to the total number as the numerator. As such, if no identity is found in parts of the two or more amino acid sequences, for example, an amino acid sequence comprises at its C terminus an additional sequence in which no identity is observed, in general, such regions cannot be aligned and, therefore, such regions are not used for calculation of the percent identity.

Further, positions of similar amino acids in two or more GLODs can be examined. For example, a plurality of amino acid sequences can be subjected to alignment with the use of CLUSTALW. In such a case, Blosum62 can be used as the algorithm and a plurality of amino acid sequences can be subjected to alignment. Amino acids determined to be similar as a result of alignment may be referred to as "similar amino acids." In the mutant of the present disclosure, amino acid substitution can be carried out between such similar amino acids. By way of such alignment, amino acid sequences composed of identical amino acids or similar amino acids among a plurality of amino acid sequences can be examined. Based on such information, homologous regions (conserved regions) in the amino acid sequences can be determined.

In one embodiment, the FAD-GLDH mutant of the present disclosure, when aligned with a GLOD having the amino acid sequence of SEQ ID NO: 58, has a full length amino acid sequence identity of 50% or more, 55% or more, 60% or more, 65% or more, 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94%

or more, 95% or more, 96% or more, 97% or more, 98% or more, for example, 99% or more, has an amino acid substitution at the position corresponding to the 545th, 425th and/or 109th position of SEQ ID NO: 58, and has an improved ratio of dehydrogenase activity to oxidase activity compared to a GLOD before modification.

In one embodiment, the FAD-GLDH variant of the present disclosure has an amino acid sequence comprising a modification or mutation, or a deletion, substitution, addition and/or insertion of one or several amino acids at a position other than the positions corresponding to the 186th, 87th, 103rd, 133rd, 297th, 376th, 393rd, 428th, 516th, 566th, 568th, 585th, and 615th positions of SEQ ID NO: 58, has an amino acid substitution at the position corresponding to the 545th, 425th and/or 109th position of SEQ ID NO: 58, and has an improved ratio of dehydrogenase activity to oxidase activity compared to a GLOD before modification. In the present specification, one or several amino acids refer to 1 to 20, 1 to 15, 1 to 10, 1 to 7, 1 to 5, 1 to 4, for example, 1 to 3, for example, 1 or 2 amino acids.

In one embodiment, the FAD-GLDHΔ has an amino acid substitution at the position corresponding to the 545th, 425th and/or 109th position of SEQ ID NO: 58, has an improved ratio of dehydrogenase activity to oxidase activity compared to a polypeptide before introduction of the amino acid substitution, and meets at least one of the following:

(i) when the amino acid sequence of the FAD-GLDHΔ is aligned with the amino acid sequence of SEQ ID NO: 58, the FAD-GLDHΔ comprises a deletion of the amino acid sequence of the region corresponding to the 459th to 507th positions of SEQ ID NO: 58, and optionally comprises a deletion of the amino acid sequence of the region corresponding to the 671st to 690th positions of SEQ ID NO: 58,
(ii) the FAD-GLDHΔ of said (i), wherein the FAD-GLDHΔ consists of an amino acid sequence comprising a substitution, deletion or addition of one or several amino acids at a position other than the region corresponding to the 459th to 507th positions of SEQ ID NO: 58, or consists of an amino acid sequence comprising a substitution, deletion or addition of one or several amino acids at a position other than the region corresponding to the 459th to 507th and 671st to 690th positions of SEQ ID NO: 58,
(iii) with regard to the FAD-GLDHΔ of said (i) or (ii), the full length amino acid sequence thereof has a sequence identity of 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, for example, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13 or SEQ ID NO: 59, or
(iv) with regard to the FAD-GLDHΔ of said (i) or (ii), the full length amino acid sequence thereof has a sequence identity of 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, for example, 90% or more, 91% or more, 92% or more, 93% or more, 94% or more, 95% or more, 96% or more, 97% or more, 98% or more, for example, 99% with the amino acid sequence of SEQ ID NO: 12, SEQ ID NO: 13 or SEQ ID NO: 59, the amino acid at the position corresponding to the 291st position of SEQ ID NO: 58 in the FAD-GLDHΔ is arginine, and the amino acid sequence of the positions corresponding to the 51st to 56th positions of SEQ ID NO: 58 is Gly-Xaa-Gly-Xaa-Xaa-Gly (wherein Xaa represents any amino acid).

In one embodiment, the FAD-GLDH-T has an amino acid substitution at the position corresponding to the 545th, 425th and/or 109th position of SEQ ID NO: 58, has an improved ratio of dehydrogenase activity to oxidase activity compared to a polypeptide before introduction of the amino acid substitution, and meets at least one of the following:

(i) when the amino acid sequence of the FAD-GLDH-T is aligned with the amino acid sequence of SEQ ID NO: 58, the FAD-GLDH-T comprises a substitution of the amino acid at the position corresponding to a position selected from the group consisting of the 186th, 87th, 103rd, 133rd, 297th, 376th, 393rd, 428th, 516th, 566th, 568th, 585th, and 615th positions of SEQ ID NO: 58,
(ii) the FAD-GLDH-T of said (i), wherein the FAD-GLDH-T consists of an amino acid sequence comprising a substitution, deletion or addition of one or several amino acids at a position other than the positions corresponding to the 186th, 87th, 103rd, 133rd, 297th, 376th, 393rd, 428th, 516th, 566th, 568th, 585th, and 615th positions of SEQ ID NO: 58,
(iii) with regard to the FAD-GLDH-T of said (i) or (ii), the full length amino acid sequence thereof has the amino acid sequence of SEQ ID NO: 58 or a sequence having identity of 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, for example, 90% or more, 91% or more, 92% or more, 93% or more, 94% or more, 95% or more, 96% or more, 97% or more, 98% or more, for example, 99% or more with SEQ ID NO: 58, or
(iv) with regard to the FAD-GLDH-T of said (i) or (ii), the full length amino acid sequence thereof has the amino acid sequence of SEQ ID NO: 58 or a sequence having identity of 70% or more, 71% or more, 72% or more, 73% or more,

74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, for example, 90% or more, 91% or more, 92% or more, 93% or more, 94% or more, 95% or more, 96% or more, 97% or more, 98% or more, for example, 99% or more with SEQ ID NO: 58, the amino acid at the position corresponding to the 291st position of SEQ ID NO: 58 in the FAD-GLDH-T is arginine, and the amino acid sequence of the positions corresponding to the 51st to 56th positions of SEQ ID NO: 58 is Gly-Xaa-Gly-Xaa-Xaa-Gly (wherein Xaa represents any amino acid).

In a particular embodiment, with regard to the FAD-GLDH-T mutant of the present disclosure, the substituted amino acid introduced into the position corresponding to the 186th position of SEQ ID NO: 58 may be selected from the group consisting of glycine, alanine, cysteine, leucine, methionine, phenylalanine, tyrosine, serine, threonine, asparagine, glutamine, histidine, aspartic acid and glutamic acid. The substituted amino acid introduced into the position corresponding to the 87th position of SEQ ID NO: 58 may be tyrosine. In a particular embodiment, with regard to the FAD-GLDH-T mutant of the present disclosure, the substituted amino acid introduced into the position corresponding to the 103rd position of SEQ ID NO: 58 may be selected from the group consisting of phenylalanine, leucine, valine, isoleucine and methionine. In a particular embodiment, with regard to the FAD-GLDH-T mutant of the present disclosure, the substituted amino acid introduced into the position corresponding to the 133rd position of SEQ ID NO: 58 may be selected from the group consisting of leucine and tyrosine. In a particular embodiment, with regard to the FAD-GLDH-T mutant of the present disclosure, the substituted amino acid introduced into the position corresponding to the 297th position of SEQ ID NO: 58 may be selected from the group consisting of leucine, valine, isoleucine and methionine. In a particular embodiment, with regard to the FAD-GLDH-T mutant of the present disclosure, the substituted amino acid introduced into the position corresponding to the 376th position of SEQ ID NO: 58 may be selected from the group consisting of phenylalanine, leucine, isoleucine and methionine. In a particular embodiment, with regard to the FAD-GLDH-T mutant of the present disclosure, the substituted amino acid introduced into the position corresponding to the 393rd position of SEQ ID NO: 58 may be selected from the group consisting of leucine, valine, isoleucine and methionine. In a particular embodiment, with regard to the FAD-GLDH-T mutant of the present disclosure, the substituted amino acid introduced into the position corresponding to the 428th position of SEQ ID NO: 58 may be selected from the group consisting of tyrosine and methionine. In a particular embodiment, with regard to the FAD-GLDH-T mutant of the present disclosure, the substituted amino acid introduced into the position corresponding to the 516th position of SEQ ID NO: 58 may be phenylalanine. In a particular embodiment, with regard to the FAD-GLDH-T mutant of the present disclosure, the substituted amino acid introduced into the position corresponding to the 566th position of SEQ ID NO: 58 may be selected from the group consisting of phenylalanine, leucine, valine and methionine. In a particular embodiment, with regard to the FAD-GLDH-T mutant of the present disclosure, the substituted amino acid introduced into the position corresponding to the 568th position of SEQ ID NO: 58 may be selected from the group consisting of isoleucine and methionine. In a particular embodiment, with regard to the FAD-GLDH-T mutant of the present disclosure, the substituted amino acid introduced into the position corresponding to the 585th position of SEQ ID NO: 58 may be selected from the group consisting of leucine, and methionine. In a particular embodiment, with regard to the FAD-GLDH-T mutant of the present disclosure, the substituted amino acid introduced into the position corresponding to the 615th position of SEQ ID NO: 58 may be leucine.

In one embodiment, the FAD-GLDHΔ-T of the present disclosure has an amino acid substitution at the position corresponding to the 545th, 425th and/or 109th position of SEQ ID NO: 58, has an improved ratio of dehydrogenase activity to oxidase activity compared to a polypeptide before introduction of the amino acid substitution, and meets at least one of the following:

(i) when the amino acid sequence of the FAD-GLDHΔ-T is aligned with the amino acid sequence of SEQ ID NO: 58, the FAD-GLDHΔ-T comprises a deletion of the amino acid sequence of the region corresponding to the 459th to 507th positions of SEQ ID NO: 58, and optionally comprises a deletion of the amino acid sequence of the region corresponding to the 671st to 690th positions of SEQ ID NO: 58, and
when the amino acid sequence of the FAD-GLDHΔ-T is aligned with the amino acid sequence of SEQ ID NO: 58, the FAD-GLDHΔ-T comprises a substitution of an amino acid, for example, a modification to an amino acid selected from the group consisting of tyrosine, phenylalanine, tryptophan, leucine, isoleucine, valine and methionine, at a position corresponding to a position selected from the group consisting of the 186th, 87th, 103rd, 133rd, 297th, 376th, 393rd, 428th, 516th, 566th, 568th, 585th, and 615th positions of SEQ ID NO: 58,
(ii) the FAD-GLDHΔ-T of said (i), wherein the FAD-GLDHΔ-T consists of an amino acid sequence comprising a substitution, deletion or addition of one or several amino acids at a position other than the positions corresponding to the 186th, 87th, 103rd, 133rd, 297th, 376th, 393rd, 428th, 516th, 566th, 568th, 585th, and 615th positions of SEQ ID NO: 58 and the region corresponding to the 459th to 507th positions of SEQ ID NO: 58, or consists of an amino acid sequence comprising a substitution, deletion or addition of one or several amino acids at a position other than the positions corresponding to the 186th, 87th, 103rd, 133rd, 297th, 376th, 393rd, 428th, 516th, 566th, 568th, 585th, and 615th positions of SEQ ID NO: 58 and the region corresponding to the 459th to 507th and 671st to 690th positions of

SEQ ID NO: 58,
(iii) with regard to the FAD-GLDHΔ-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13, SEQ ID NO: 59, or SEQ ID NO: 60, or
(iv) with regard to the FAD-GLDHΔ-T of said (i) or (ii), the full length amino acid sequence thereof has a sequence identity of 70% or more, 75% or more, 80% or more, 85% or more, 90% or more, 91% or more, 92% or more, 93% or more, 94% or more, 95% or more, 96% or more, 97% or more, 98% or more, for example, 99% or more with the amino acid sequence of SEQ ID NO: 12, SEQ ID NO: 13, SEQ ID NO: 59 or SEQ ID NO: 60, the amino acid at the position corresponding to the 291st position of SEQ ID NO: 58 in the FAD-GLDHΔ is arginine, and the amino acid sequence of the positions corresponding to the 51st to 56th positions of SEQ ID NO: 58 is Gly-Xaa-Gly-Xaa-Xaa-Gly (wherein Xaa represents any amino acid).

In one embodiment, the present disclosure provides an FAD-GLDH mutant having an amino acid sequence identity of 70% or more, 71% or more, 72% or more, 73% or more, 74% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, for example, 90% or more, 91% or more, 92% or more, 93% or more, 94% or more, 95% or more, 96% or more, 97% or more, 98% or more, for example, 99% or more with the amino acid sequence of SEQ ID NO: 60, comprising an amino acid substitution at the position corresponding to the 545th, 425th and/or 109th position of SEQ ID NO: 58, and comprising a deletion of the region corresponding to the 459th to 507th positions of SEQ ID NO: 58,

having an amino acid substitution compared to the amino acid sequence of SEQ ID NO: 58, at one or more positions selected from the group consisting of
the position corresponding to the 186th position of SEQ ID NO: 58,
the position corresponding to the 87th position of SEQ ID NO: 58,
the position corresponding to the 103rd position of SEQ ID NO: 58,
the position corresponding to the 133rd position of SEQ ID NO: 58,
the position corresponding to the 297th position of SEQ ID NO: 58,
the position corresponding to the 376th position of SEQ ID NO: 58,
the position corresponding to the 393rd position of SEQ ID NO: 58,
the position corresponding to the 428th position of SEQ ID NO: 58,
the position corresponding to the 516th position of SEQ ID NO: 58,
the position corresponding to the 566th position of SEQ ID NO: 58,
the position corresponding to the 568th position of SEQ ID NO: 58,
the position corresponding to the 585th position of SEQ ID NO: 58, and
the position corresponding to the 615th position of SEQ ID NO: 58, and
having glutamate dehydrogenase activity. However, in particular embodiments, wild type amino acids are excluded from the substituted amino acids. In a particular embodiment, in the mutant, the region corresponding to the 671st to 690th positions of SEQ ID NO: 58 may further be deleted.

In a particular embodiment, with regard to the FAD-GLDHΔ-T mutant of the present disclosure, the substituted amino acid introduced into the position corresponding to the 186th position of SEQ ID NO: 58 may be selected from the group consisting of glycine, alanine, cysteine, leucine, methionine, phenylalanine, tyrosine, serine, threonine, asparagine, glutamine, histidine, aspartic acid and glutamic acid. In a particular embodiment, with regard to the FAD-GLDHΔ-T mutant of the present disclosure, the substituted amino acid introduced into the position corresponding to the 87th position of SEQ ID NO: 58 may be tyrosine. In a particular embodiment, with regard to the FAD-GLDHΔ-T mutant of the present disclosure, the substituted amino acid introduced into the position corresponding to the 103rd position of SEQ ID NO: 58 may be selected from the group consisting of phenylalanine, leucine, valine, isoleucine and methionine. In a particular embodiment, with regard to the FAD-GLDHΔ-T mutant of the present disclosure, the substituted amino acid introduced into the position corresponding to the 133rd position of SEQ ID NO: 58 may be selected from the group consisting of leucine and tyrosine. In a particular embodiment, with regard to the FAD-GLDHΔ-T mutant of the present disclosure, the substituted amino acid introduced into the position corresponding to the 297th position of SEQ ID NO: 58 may be selected from the group consisting of leucine, valine, isoleucine and methionine. In a particular embodiment, with regard to the FAD-GLDHΔ-T mutant of the present disclosure, the substituted amino acid introduced into the position corresponding to the 376th position of SEQ ID NO: 58 may be selected from the group consisting of phenylalanine, leucine, isoleucine and methionine. In a particular embodiment, with regard to the FAD-GLDHΔ-T mutant of the present disclosure, the substituted amino acid introduced into the position corresponding to the 393rd position of SEQ ID NO: 58 may be selected from the group

consisting of leucine, valine, isoleucine and methionine. In a particular embodiment, with regard to the FAD-GLDHΔ-T mutant of the present disclosure, the substituted amino acid introduced into the position corresponding to the 428th position of SEQ ID NO: 58 may be selected from the group consisting of tyrosine and methionine. In a particular embodiment, with regard to the FAD-GLDHΔ-T mutant of the present disclosure, the substituted amino acid introduced into the position corresponding to the 516th position of SEQ ID NO: 58 may be phenylalanine. In a particular embodiment, with regard to the FAD-GLDHΔ-T mutant of the present disclosure, the substituted amino acid introduced into the position corresponding to the 566th position of SEQ ID NO: 58 may be selected from the group consisting of phenylalanine, leucine, valine and methionine. In a particular embodiment, with regard to the FAD-GLDHΔ-T mutant of the present disclosure, the substituted amino acid introduced into the position corresponding to the 568th position of SEQ ID NO: 58 may be selected from the group consisting of isoleucine and methionine. In a particular embodiment, with regard to the FAD-GLDHΔ-T mutant of the present disclosure, the substituted amino acid introduced into the position corresponding to the 585th position of SEQ ID NO: 58 may be selected from the group consisting of leucine and methionine. In a particular embodiment, with regard to the FAD-GLDHΔ-T mutant of the present disclosure, the substituted amino acid introduced into the position corresponding to the 615th position of SEQ ID NO: 58 may be leucine.

(Production of FAD-GLDH)

In one embodiment, the present invention provides a method for producing FAD-GLDH comprising a step of culturing a strain capable of producing FAD-GLDH under conditions where the FAD-GLDH can be expressed and a step of isolating FAD-GLDH from a culture product or culture solution. In such method, a host cell transformed with a vector comprising a gene encoding the FAD-GLDH of the present disclosure integrated therein can be used. The phrase conditions where the FAD-GLDH can be expressed refers to conditions where an FAD-GLDH gene is transcribed and translated, and a polypeptide encoded by such gene is produced. GLOD may be produced in the same manner as above.

Further, examples of media for culturing the strains mentioned above include media prepared by adding 1 or more inorganic salts selected from among, for example, sodium chloride, monopotassium phosphate, dipotassium phosphate, magnesium sulfate, magnesium chloride, ferric chloride, ferric sulfate, and manganese sulfate to 1 or more nitrogen sources, such as a yeast extract, tryptone, peptone, a meat extract, a corn steep liquor, or a leaching solution of soybean or wheat bran, and appropriately adding saccharine materials (sugar sources), vitamins, and the like thereto, where necessary.

Further, a substrate with which the FAD-GLDH can react or a compound similar thereto, such as a glycated protein, including a glycated amino acid, a glycated peptide, a degradation product of a glycated protein, glycated hemoglobin, or glycated albumin, may be added to the media, so as to increase the amount of the target enzyme to be produced.

It is appropriate to adjust the initial pH of the media to 7 to 9. Culture is preferably performed at 20°C to 42°C, and more preferably at about 25°C to 37°C for 4 to 24 hours, and further preferably at about 25°C to 37°C for 8 to 16 hours, by, for example, aeration spinner submerged culture, shake culture, or stationary culture.

Following the completion of culture, FAD-GLDH may be collected from the culture products with conventional enzyme collecting means. For example, a strain may be subjected to ultrasonication treatment or grinding treatment by a conventional method, the enzyme may be extracted using a lytic enzyme such as lysozyme, or bacteriolysis may be performed via shaking or allowing to stand still in the presence of toluene to excrete the enzyme from the microorganism body. The solution is filtered or centrifuged to remove solid content, and nucleic acids is removed with the aid of streptomycin sulfate, protamine sulfate, or manganese sulfate, according to need. Thereafter, ammonium sulfate, alcohol, acetone, or the like is added thereto, so as to fractionate the solution, and precipitates are then collected to obtain the crude enzymes.

A purified enzyme preparation can be obtained from the crude enzyme by a method appropriately selected from: gel filtration methods using Sephadex, Superdex, or Ultrogel; adsorption-elution methods using ion exchange carriers, hydrophobic carriers, or hydroxyapatite; electrophoretic methods using polyacrylamide gels, etc.; precipitation methods such as sucrose density-gradient centrifugation; affinity chromatographic methods; and fractionation methods using a molecular sieve membrane, a hollow-fiber membrane, etc. Alternatively, the methods mentioned above can be performed appropriately in combination and the purified FAD-GLDH preparation can be thus obtained.

In one embodiment, the FAD-GLDH mutant is capable of, for example,

(i) using FAD as a coenzyme,
(ii) recognizing glutamate as a substrate, and
(iii) acting so as to oxidize glutamate to generate 2-oxoglutaric acid and ammonia.

In one embodiment, the residual activity of the FAD-GLDH after being subjected to heat treatment at 30 to 40°C, for example, 35°C, for 30 minutes or 35 minutes may be 50% or more, 60% or more, 65% or more, 70% or more, 75% or

more, 80% or more, 85% or more, for example, 90% or more when its activity before the heat treatment is defined as 100%. In one embodiment, the residual activity of the FAD-GLDH after being subjected to heat treatment at, for example, 60°C, for 30 minutes or 35 minutes may be 50% or more, 60% or more, 65% or more, 70% or more, 75% or more, 80% or more, 85% or more, for example, 90% or more when its activity before the heat treatment is defined as 100%. In one embodiment, the residual activity of the FAD-GLDH after being subjected to heat treatment at, for example, 65°C, for 30 minutes or 35 minutes may be 50% or more, 60% or more, 65% or more, 70% or more, for example, 75% or more when its activity before the heat treatment is defined as 100%. In one embodiment, the residual activity of the FAD-GLDH after being subjected to heat treatment at, for example, 70°C, for 30 minutes or 35 minutes may be 10% or more, 15% or more, 20% or more, 25% or more, 30% or more, for example, 35% or more when its activity before the heat treatment is defined as 100%.

FAD-GLDHs that do not exhibit any activity on glutamate are excluded from the FAD-GLDH of the present disclosure.

(Composition, reagent, electrode, sensor, and kit)

In one embodiment, the present invention provides a kit, sensor, electrode, reagent for measurement, or reagent composition for measurement of glutamate, comprising an FAD-GLDH. The composition, reagent, electrode, sensor or kit may comprise reagents for measurement of a reduced compound, reagents for measurement of hydrogen peroxide, a buffer, a surfactant, a salt, a preservative, or the like. The composition, reagent, electrode, sensor or kit may be supplemented with, for example, a solubilizer, a stabilizer, a reaction-improving agent, a glycated hemoglobin denaturation agent, a reducing agent, bovine serum albumin, or a saccharide (e.g., glycerin, lactose, or sucrose) and the like. The composition, reagent, electrode, sensor or kit may be supplemented with other known stabilizers, or contaminant-eliminating systems, and the like, according to need. Techniques that are employed for various conventional reagents, electrodes, sensors, or kits may be modified appropriately, and be employed for the composition, reagent, electrode, sensor or kit of the present disclosure.

Examples of surfactants include nonionic surfactants and ionic surfactants, such as cationic surfactants, anionic surfactants, and amphoteric surfactants and the like.

Examples of nonionic surfactants include polyoxyethylene alkyl ether, sorbitan fatty acid ester, alkyl polyglucoside, fatty acid diethanol amide, and alkyl monoglyceryl ether.

Examples of cationic surfactants include alkyltrimethylammonium salt, dialkyldimethylammonium salt, alkylbenzyldimethylammonium salt, pyridinium salt, such as alkylpyridinium salt, phosphonium salt, such as alkylphosphonium salt, imidazolium salt, such as alkylimidazolium salt, and isoquinolinium salt, such as alkylisoquinolinium salt.

(Method for measurement of glutamate)

In one embodiment, the present disclosure provides a method for measurement of glutamate. The method for measurement of glutamate may be a qualitative or quantitative method. The quantitative method comprises a step of bringing a glutamate-containing sample into contact with the FAD-GLDH of the present disclosure and a step of measuring the amount of substances produced or consumed by the reaction. The term "contact" used in accordance with the quantifying method encompasses any aspect of physical contact between the FAD-GLDH and a sample, such that the FAD-GLDH can catalyze the dehydrogenation reaction of the glutamate. This includes, for example, not only cases in which a free enzyme is mixed with glutamate in a solution, but also cases in which a liquid sample comprising glutamate can be added or dropped (added dropwise) to the enzyme immobilized on a solid support. In one embodiment, in the case of measuring glutamate with the use of the dehydrogenase activity of the FAD-GLDH, hydrogen peroxide is excluded from the reaction product to be measured (that is, generated hydrogen peroxide is not measured). In one embodiment, in the case of measuring glutamate with the use of the dehydrogenase activity of the FAD-GLDH, water is excluded from the consumed product to be measured (that is, consumed water is not measured). In one embodiment, in the case of measuring glutamate with the use of the dehydrogenase activity of the FAD-GLDH, a reaction product to be measured comprises a reduced form mediator (that is, a generated reduced form mediator is measured). In one embodiment, in the case of measuring glutamate with the use of the dehydrogenase activity of the FAD-GLDH, a consumed product to be measured comprises an oxidized form mediator (that is, a consumed oxidized form mediator is measured).

A sample used for measurement can be any type of sample that can contain glutamate. A sample can appropriately be a processed sample.

When the amount of the enzyme used and the duration of the reaction are maintained at a constant level and the amount of glutamate to be added is altered, the range of glutamate concentration in which the absorbance of the detected chromogenic substrate or response current proportionally decreases as the amount of added glutamate decreases can be investigated in order to determine the lowest glutamate concentration that can be detected (detection limit concentration) using that FAD-GLDH. It is possible to configure the amount of enzyme and duration of reaction, so as to adjust the detection limit of glutamate to a level lower than the glutamate level in the sample or in the blood.

According to the quantitative method of measurement, a calibration curve can be prepared in advance by performing regression analysis such as the method of least squares based on the measured absorbance of the control sample comprising glutamate at a known concentration. The measured value of the sample containing glutamate at an unknown concentration may be plotted on the prepared calibration curve, to quantify the glutamate level in the sample.

The time FAD-GLDH is allowed to act on a sample containing glutamate may be 5 seconds or longer, 10 seconds or longer, 20 seconds or longer, 30 seconds or longer, or 1 minute or longer to less than 60 minutes, less than 30 minutes, less than 10 minutes, or less than 5 minutes, for example, 0.5 minutes or more to less than 60 minutes, 1 minute or more to less than 30 minutes, 1 minute or more to less than 20 minutes, 1 minute or more to less than 10 minutes, or 1 minute or more to less than 5 minutes. While the reaction temperature may vary depending on the optimal temperature of the enzyme being used, the reaction temperature may for example be from 20°C to 45°C, and a temperature that is generally employed for an enzymatic reaction can appropriately be selected.

While the amount of the FAD-GLDH to be used varies depending on the amount of the substrate contained in the sample solution, the enzyme can be added to the solution to a final concentration of, for example, 0.1 to 50 U/ml or for example 0.2 to 10 U/ml. The pH level at the time of reaction can be adjusted using a buffer by taking the pH levels at which the FAD-GLDH can act, for example the optimal pH level, into consideration. The reaction pH level is, for example, 3 to 11, 5 to 9, or 6 to 8.

(Method for measurement of glutamate oxidase activity (GLOD activity))

Hereinbelow, an example of a method for measurement of GLOD activity involving the use of glutamate as a substrate is provided. However, the method for measurement is not limited thereto. The glutamate may be a commercially available glutamate. In the present specification, regarding the enzyme titer, unless specified otherwise, the amount of the enzyme that generates 1 μmol of hydrogen peroxide per minute, when carrying out measurement at 30°C and pH 7.4 using glutamate as the substrate, is defined as 1 U.

A: Reagents for measurements of activity

(Reagent 1) 250 mM potassium phosphate buffer, pH 7.4
(Reagent 2) 30 mM 4-aminoantipyrine (4-AA) solution
(Reagent 3) 15 mM N-ethyl-N-(2-hydroxy-3-sulfopropyl)-3-methylaniline sodium salt (TOOS) solution
(Reagent 4) 300 U/ml horseradish peroxidase (POD) solution
(Reagent 5) 300 mM sodium hydrogen glutamate solution

B: Method for measurement of activity

300 μl of Reagent 1, 12.5 μl of Reagent 2, 25 μl of Reagent 3, 12.5 μl of Reagent 4, (375 - V) μl of deionized water, and V μl of the GLOD solution are mixed and kept at a temperature of 30°C for 5 minutes. Subsequently, 25 μl of Reagent 5 is added, the resultant is thoroughly mixed, and the light absorbance at a wavelength of 555 nm ($A_{555}$) is then measured using spectrophotometer U-3900 (manufactured by Hitachi High-Tech Science Corporation), while maintaining the cell holder thereof at a temperature of 30°C, to compute the change in $A_{555}$ per minute ($\Delta A_s$). Incidentally, as a control experiment, 25 μl of deionized water is added instead of 25 μl of Reagent 5, and the light absorbance at a wavelength of 555 nm ($A_{555}$) is then measured to compute the change in $A_{555}$ per minute ($\Delta A_0$).

Glutamate oxidase activity (U/ml) may be calculated according to the calculation formula given below. In the formula, "39.2" represents the millimolar extinction coefficient ($mM^{-1}cm^{-1}$) of quinoneimine dye formed by the condensation between 4-AA and TOOS, against light at a wavelength of 555 nm.

[Formula]

$$U/ml = (\Delta A_S - \Delta A_0) \times 600 \times df / (39.2 \times 0.5 \times V)$$

$$= 30.6 \times (\Delta A_S - \Delta A_0) \times df / V.$$

In the case of using different chromogenic reagents, wavelengths appropriate for the chromogenic reagents and millimolar extinction coefficients at the wavelengths may be used.

(Method for measurement of glutamate dehydrogenase activity (FAD-GLDH activity))

Hereinbelow, an example of a method for measurement of glutamate dehydrogenase activity involving the use of glutamate as a substrate is provided. However, the method for measurement is not limited thereto. The glutamate may be a commercially available glutamate. In the present specification, regarding the enzyme titer, unless specified otherwise, the amount of the enzyme that performs reaction with 1 μmol of the substrate glutamate per minute, when carrying out measurement at 30°C and pH 7.4 using glutamate as the substrate, is defined as 1 U.

A: Reagents for measurements of activity

(Reagent 1) 250 mM potassium phosphate buffer, pH 7.4
(Reagent 2) 3 mM dichloroindophenol (DCIP) solution
(Reagent 3) 30 mM 1-methoxy PMS (mPMS) solution
(Reagent 4) 300 mM sodium hydrogen glutamate solution

B: Method for measurement of activity

300 μl of Reagent 1, 25 μl of Reagent 2, 25 μl of Reagent 3, (375 - V) μl of deionized water, and V μl of the GLOD solution are mixed and kept at a temperature of 30°C for 5 minutes. Subsequently, 25 μl of Reagent 5 is added, the resultant is thoroughly mixed, and the light absorbance at a wavelength of 600 nm ($A_{600}$) is then measured using spectrophotometer U-3900 (manufactured by Hitachi High-Tech Science Corporation), while maintaining the cell holder thereof at a temperature of 30°C, to compute the decrease in $A_{600}$ per minute ($\Delta A_S$). As a control experiment, 25 μl of deionized water is added instead of 25 μl of Reagent 4, and the light absorbance at a wavelength of 600 nm ($A_{600}$) is then measured to compute the change in $A_{600}$ per minute ($\Delta A_0$).

Glutamate dehydrogenase activity (U/ml) may be calculated according to the calculation formula given below. In the formula, "21" represents the millimolar extinction coefficient ($mM^{-1}cm^{-1}$) at pH 7.4 of DCIP against light at a wavelength of 600 nm.

[Formula]

$$U/ml = -(\Delta A_S - \Delta A_0) \times 750 \times df / (21 \times V)$$
$$= -35.7 \times (\Delta A_S - \Delta A_0) \times df / V.$$

In the case of using different chromogenic reagents, wavelengths appropriate for the chromogenic reagents and millimolar extinction coefficients at the wavelengths may be used.

(Dry composition of GLOD or FAD-GLDH)

GLOD or FAD-GLDH can be allowed to coexist with a trisaccharide and dried, thereby maintaining enzyme activity even if the dried product is preserved over a long period of time. Examples of the trisaccharide used in the present invention include raffinose, kestose, maltotriose, maltotriulose, nigerotriose, and melezitose and the like. Particularly, raffinose is preferable. In a step of drying GLOD or FAD-GLDH, the GLOD or FAD-GLDH may be allowed to coexist with a plurality of trisaccharides and dried. A mixture of a dried GLOD or FAD-GLDH powder and a dried trisaccharide powder merely mixed uniformly is not included in the dry composition of the present invention.

The dry composition of GLOD or FAD-GLDH may further comprise, in addition to the trisaccharide, an additive (buffer, stabilizer, excipient, solubilizer, antiseptic, etc.) and may contain two or more additives.

The amount of the GLOD or FAD-GLDH contained in the dry composition of the present invention is preferably 0.1% by mass or more and less than 80% by mass, more preferably 1% by mass or more and less than 75% by mass, and further preferably 10% by mass or more and less than 70% by mass.

The amount of the trisaccharide contained in the dry composition of the present invention is preferably 1% by mass or more and less than 90% by mass, more preferably 10% by mass or more and less than 80% by mass, and further preferably 20% by mass or more and less than 75% by mass.

The amount of the trisaccharide contained in the dry composition can be 0.10 mg or more and less than 50 mg, 0.10 mg or more and less than 40 mg, 0.10 mg or more and less than 30 mg, 0.10 mg or more and less than 20 mg, for example, 0.10 mg or more and less than 10 mg per 100 U of the GLOD or FAD-GLDH. The amount of the trisaccharide

contained in the dry composition is preferably 0.10 mg or more and less than 5 mg, more preferably 0.10 mg or more and less than 2.5 mg, and further preferably 0.10 mg or more and less than 0.50 mg per 100 U of the GLOD or FAD-GLDH.

The stabilizing effect of the trisaccharide on the GLOD or FAD-GLDH is larger than that of a monosaccharide or sugar alcohol. More specifically, a monosaccharide D-glucose, when used in a GLOD dry composition, exhibited a decreased activity value after preservation at 37°C for 2 weeks. When a sugar alcohol xylitol was used in a GLOD dry composition, about 49% of an activity value remained after preservation at 37°C for 2 weeks.

Patent Literature 2 (JP Patent No. 6349452) discloses a disaccharide (lactose) as a compound that stabilizes a dried product of GLOD from *Streptomyces* sp. X-119-6. A freeze-dried product of GLOD from *Streptomyces* sp. X-119-6 supplemented with lactose had an activity value of 101.5% after being preserved at 37°C for 2 weeks. On the other hand, a freeze-dried product of GLOD from *Streptomyces* sp. X-119-6 supplemented with a sugar alcohol (mannitol) had an activity value of 50.5% after being preserved at 37°C for 2 weeks. The tendency of the compound that stabilizes such a dried product of GLOD from *Streptomyces* sp. X-119-6 is different from the tendency of the compound that stabilizes the dried product of GLOD or FAD-GLDH disclosed by the present invention. As such, the stabilizing effect of the trisaccharide, particularly, D-raffinose, on GLOD is a novel finding that is difficult to predict from conventional techniques.

The dry composition of GLOD or FAD-GLDH may be prepared by a freeze drying approach or may be prepared by a spray drying method.

The dry composition of GLOD or FAD-GLDH can be prepared by drying an aqueous composition containing the GLOD or FAD-GLDH and a trisaccharide. The pH of the aqueous composition may be adjusted with a buffer. Any buffer having a buffering ability in the range of pH 5.0 to 9.0 can be suitably used. Examples of such buffers include various carboxylates (acetate, citrate, ethylenediaminetetraacetate, phthalate, fumarate, malate, maleate, glutarate, etc.), phosphate, tris(hydroxymethyl)aminomethane, borate, and various Good's buffer solutions (MES, Bis-Tris, ADA, PIEPS, ACES, MOPSO, BES, MOPS, TES, HEPES, TAPSO, POPSO, HEPSO, EPPS, Tricine, Bicine, TAPS, CHES, etc.). It is possible to use only one of these buffers, or two or more thereof may be used in combination. Phosphate is preferable as the buffer for adjusting the pH of the aqueous composition containing the GLOD or FAD-GLDH and the trisaccharide.

The concentration of the buffer solution is not particularly limited and is, for example, 5 mM or more and 100 mM or less, and preferably 5 mM or more and 50 mM or less. The buffer solution component content of the dry composition is preferably 1 to 80% by mass.

The GLOD or FAD-GLDH content of the aqueous composition is preferably 10 U/mL or more and less than 500 U/mL, and more preferably 25 U/mL or more and less than 300 U/mL.

The trisaccharide content of the aqueous composition is, for example, 0.1 mg/mL or more and less than 100 mg/mL, preferably 0.5 mg/mL or more and less than 50 mg/mL, and more preferably 1 mg/mL or more and less than 10 mg/mL.

In one embodiment, the present disclosure provides a polynucleotide encoding a glutamate dehydrogenase. In one embodiment, the present disclosure provides a vector comprising such a polynucleotide. In one embodiment, the present disclosure provides a host cell transformed with such a vector, that is, a host cell comprising such vector. In one embodiment, the present disclosure provides a method for producing a glutamate dehydrogenase comprising the steps of: culturing such host cell to produce a glutamate dehydrogenase, and obtaining the produced glutamate dehydrogenase. In one embodiment, the present disclosure provides a dry composition comprising a glutamate oxidase or FAD-glutamate dehydrogenase, and a method for producing the same. In one embodiment, the present disclosure provides a method for bringing a glutamate dehydrogenase or a composition, reagent, electrode, sensor or kit comprising this into contact with a sample containing glutamic acid to oxidize the glutamic acid contained in the sample. In one embodiment, this method is capable of detecting glutamic acid. In one embodiment, this method is capable of measuring glutamic acid.

(Method for preparing further FAD-GLDH(s))

The present inventor prepared a GLODΔ having a particular amino acid sequence deletion introduced into GLOD. Further, a GLODΔ-T having a particular amino acid substitution introduced into GLODΔ was prepared. The present inventors further prepared an FAD-GLDHΔ having a particular amino acid sequence deletion introduced into GLODΔ. Further, an FAD-GLDHΔ-T having a particular amino acid substitution introduced into FAD-GLDHΔ was prepared. The amino acid substitution, deletion and mutation can appropriately be combined. Based on the description of the present specification, further mutants may be prepared. As such, in one embodiment, the present invention provides a method for preparing an FAD-GLDH, comprising the following steps:

(i) obtaining a GLOD gene or GLODΔ gene,
(ii) integrating the GLOD gene or GLODΔ gene into a vector, transforming a host cell, expressing the GLOD or GLODΔ, and isolating the expressed product,
(iii) measuring the activity of the expressed product,
(iv) subjecting the expressed product to heat treatment and then measuring the residual activity thereof,

(v) modifying said GLOD gene or GLODΔ gene such that, when the amino acid sequence of the GLOD or GLODΔ is aligned with the amino acid sequence of SEQ ID NO: 58, the amino acid at the position corresponding to a position selected from the group consisting of the 186th, 87th, 103rd, 133rd, 297th, 376th, 393rd, 428th, 516th, 566th, 568th, 585th, and 615th positions of SEQ ID NO: 58 is substituted with, for example, an amino acid selected from the group consisting of tyrosine, phenylalanine, tryptophan, leucine, isoleucine, valine and methionine,
(vi) integrating the modified gene into a vector, transforming a host cell, expressing the variant, and isolating the expressed product,
(vii) subjecting the expressed product of the variant to heat treatment, and then measuring the residual activity thereof, and comparing the measured value with the value measured in step (iv),
(viii) when the residual activity after the heat treatment of the modified variant is increased by 5%, 6%, 7%, 8%, 9%, or 10% or more compared to the residual activity after the heat treatment of the GLOD or GLODΔ before modification, designating the modified variant as a thermal stability improved type GLOD mutant,
(ix) optionally repeating steps (v) to (vii) on the variant of step (viii),
(x) introducing the amino acid substitution of the present disclosure into the gene encoding the thermal stability improvement type GLOD mutant of the step (viii) or (ix) to prepare an FAD-GLDH gene,
(xi) integrating the modified gene into a vector, transforming a host cell, expressing the variant, and isolating the expressed product,
(xii) measuring the ratio of dehydrogenase activity to oxidase activity of the expressed product of the variant, and comparing it to the ratio of dehydrogenase activity to oxidase activity of a GLOD mutant before introduction of the amino acid substitution of the present disclosure, and
(xiii) when the ratio of dehydrogenase activity to oxidase activity of the FAD-GLDH having the amino acid substitution of the present disclosure introduced therein is increased by 5% or more, for example, 10% or more, 20% or more, 30% or more, 40% or more, 50% or more, 100% or more, 150% or more, 200% or more, 300% or more, 400% or more, 500% or more, 1000% or more, 1500% or more, for example, 2000% or more compared to a GLOD mutant before modification, formulating the modified variant as an FAD-GLDH into a kit or composition.

In a particular embodiment, with regard to the FAD-GLDH mutant, for example, FAD-GLDHΔ or FAD-GLDHΔ-T, a peptide linker may be inserted into a region in which an amino acid sequence was deleted. The peptide linker may be, for example, a linker composed of 1 to 20 amino acid residues. The peptide linker may be composed of an amino acid sequence different from a partial amino acid sequence of an FAD-GLDH. The peptide linker may be composed of, for example, 2, 3, 4, 5, 6, 7, 8, 9, 10, 11, 12, 13, 14, 15, 16, 17, 18, 19 or 20 amino acid residues. The peptide linker may be composed of, for example, 2 to 19, 3 to 18, 4 to 17, 5 to 16, 6 to 15, for example, 7 to 14 amino acid residues. The amino acid residues constituting the peptide linker may be natural amino acids and glycine. Examples of the amino acid residue include Ala, Asn, Cys, Gln, Ile, Leu, Met, Phe, Pro, Ser, Thr, Trp, Tyr, Val, Asp, Glu, Arg, His, and Lys. Examples of the amino acid residue include Gly, Ala, Ser, and Thr. In one embodiment, the peptide linker may be GGGGS or a repeated sequence thereof. The number of repeats may be 2, 3, or 4. In one embodiment, the FAD-GLDH mutant, for example, FAD-GLDHΔ or FAD-GLDHΔ-T, has no peptide linker.

The present disclosure provides an FAD-GLDH. The FAD-GLDH is an unprecedented type of enzyme and is evidently differentiated from NAD-GLDH known by conventional techniques. The FAD-GLDH of the present disclosure has no or low amino acid sequence identity with conventional NAD-GLDHs. The FAD-GLDH of the present disclosure enables glutamic acid to be measured without externally adding NAD. With regard to a NAD type GLDH, it is known that NAD or NADH is liberated from the enzyme. Furthermore, reverse reaction may occur when the concentration of the liberated (free) NADH is elevated. With regard to an FAD type GLOD, FAD is not liberated or rarely liberated from the enzyme. With regard to the FAD-GLDH of the present disclosure, it is likewise believed that FAD is not liberated or rarely liberated from the enzyme. It is therefore believed that the FAD-GLDH of the present disclosure is unlikely to cause reverse reaction. This is advantageous for use as a sensor. Furthermore, the FAD-GLDH of the present disclosure is capable of reducing the influence of dissolved oxygen on measured values. This is also advantageous for use as a sensor.

In one embodiment, the present disclosure provides a method for measuring glutamic acid using an FAD-GLDH. This method does not require external addition of NAD. Furthermore, this method does not require adding peroxidase. Further, pretreatment with a protease is not necessary for preparing the FAD-GLDH. Further, the method for measuring glutamic acid using an FAD-GLDH can be combined with various mediators because NAD is not liberated from the enzyme. In one embodiment, in the method for measuring glutamic acid using an FAD-GLDH, NAD is not added (aspects in which NAD is added are excluded). In one embodiment, in the method for measuring glutamic acid using an FAD-GLDH, peroxidase is not added (except for an aspect in which peroxidase is added).

In another embodiment, the method for measuring glutamic acid using an FAD-GLDH according to the present disclosure can measure glutamic acid even in the presence of NAD or NADH in a measurement sample because liberation of NAD from the enzyme does not occur. In one embodiment, the present disclosure provides a reagent for measurement of glutamic acid comprising an FAD-GLDH. In one embodiment, this reagent for measurement comprises no NAD. In one

embodiment, this reagent for measurement comprises no peroxidase. However, this reagent for measurement can be used to measure glutamic acid with regard to a sample containing NAD.

Examples

The FAD-GLDH of the present disclosure will be further illustrated by way of the following Examples. However, these examples are provided merely for illustration and the present disclosure is not limited by these examples in any way.

[Example 1]

First, a glutamate oxidase (GLOD) was prepared. Subsequently, mutants of the GLOD were prepared and the dehydrogenase activity thereof was confirmed.

1. Construction of plasmid for glutamate oxidase (GLOD) expression

A plasmid (pKK223-3-StGLOD) for expression of a glutamate oxidase from *Streptomyces* sp. X-119-6 (StGLOD) having the amino acid sequence of SEQ ID NO: 1 was prepared using NEBuilder HiFi DNA assembly (manufactured by New England Biolabs Inc.).

StGLOD gene having the nucleotide sequence of SEQ ID NO: 2 was divided into 3 fragments of SEQ ID NO: 3 (StGLOD-f1), SEQ ID NO: 4 (StGLOD-f2) and SEQ ID NO: 5 (StGLOD-f3) and their synthesis was commissioned to Integrated DNA Technologies, Inc. Fifteen 3' terminal bases of StGLOD-f1 and Fifteen 5' terminal bases of StGLOD-f2 are overlapping sequences for gene assembly. Fifteen 3' terminal bases of StGLOD-f2 and Fifteen 5' terminal bases of StGLOD-f3 are overlapping sequences for gene assembly.

A plasmid fragment was prepared by PCR using pKK223 plasmid as the template and primers of SEQ ID NO: 6 (ggtcatttcattcatgaattctgtttcctgtgtgaaattg) and SEQ ID NO: 7 (gcgttaacttcttaagcttggctgttttggcggatgag). The PCR was carried out in accordance with the instruction manual of KOD One PCR Master Mix (manufactured by Toyobo Co., Ltd.). The primer of SEQ ID NO: 6 or SEQ ID NO: 7 had a sequence of 15 bases overlapping with StGLOD-f1 or StGLOD-f3, respectively, which was added to the 5' end of the sequence that anneals to pKK223-3. The solution after PCR was supplemented with 1.0 μl of DpnI (manufactured by New England BioLabs Inc.) and treated at 37°C for 1 hour, and then, the amplified fragment was purified using GFX PCR DNA and Gel Band Purification Kit (manufactured by Cytiva).

The plasmid (pKK223-3-StGLOD) for StGLOD expression was obtained through reaction at 50°C for 60 minutes according to the composition of Table 1. *E. coli* JM109 strain was transformed with the obtained plasmid. The obtained transformant was cultured, and the nucleotide sequence of the extracted plasmid was confirmed by DNA sequence analysis to be the intended sequence.

[Table 1]

| Reagent | Volume |
|---|---|
| NEBuilder HiFi DNA assembly | 10.0 μl |
| 32 ng/μl pKK223-3 fragment | 3.1 μl |
| 40 ng/μl StGLOD-f1 | 2.3 μl |
| 40 ng/μl StGLOD-f2 | 2.3 μl |
| 40 ng/μl StGLOD-f3 | 2.3 μl |
| Total volume | 20.0 μl |

2. Preparation of StGLOD deletion mutant

A plasmid carrying a gene encoding a StGLOD deletion mutant was obtained by site directed mutagenesis using pKK223-3-StGLOD as the template. The PCR reaction solution was prepared by mixing 10 μl of KOD One PCR Master Mix (manufactured by Toyobo Co., Ltd.), 3 μl of 2 μM Fw primer, 3 μl of 2 μM Rv primer, 0.5 μl of 40 μg/ml pKK223-3-StGLOD, and 3.5 μl of ion exchange water. A deletion mutant prepared using primers of SEQ ID NO: 8 (gataagaccgaagcgac caatgcgtacgga) and SEQ ID NO: 9 (cagcttacgataatagtcgtacagacccgg) was designated as StGLODΔ49Ai, and a deletion mutant prepared using primers of SEQ ID NO: 10 (gcagagccacctgcgaccaatgcgtacgga) and SEQ ID NO: 11 (cgcatcttga taatagtcgtacagacccgg) was designated as StGLODΔ49C. The amino acid sequence of StGLODΔ49Ai or StGLODΔ49C is shown in SEQ ID NO: 12 or SEQ ID NO: 13, respectively. PCR reaction conditions involved a cycle of "98°C for 10 sec →

55°C for 5 sec → 68°C for 35 sec" and this was repeated 7 times.

The solution after PCR was supplemented with 1 μl of DpnI and treated at 37°C for 1 hour to degrade the template pKK223-3-StGLOD. 2 μl of the obtained solution treated with DpnI was mixed with 5 μl of Ligation High Ver. 2 (manufactured by Toyobo Co., Ltd.), 1 μl of 5 U/ μl T4 polynucleotide kinase, and 7 μl of ion exchange water and reacted at 16°C for 1 hour, and then, *E. coli* JM109 strain was transformed with the reaction solution. The obtained transformant was cultured, and the nucleotide sequence of an extracted plasmid was confirmed by DNA sequence analysis to be the intended sequence.

The solution after PCR was supplemented with 1 μl of DpnI and treated at 37°C for 1 hour to degrade the template pKK223-3-StGLODΔ49C. *E. coli* JM109 strain was transformed with the obtained solution treated with DpnI. The obtained transformant was cultured, and the nucleotide sequence of the extracted plasmid was confirmed by DNA sequence analysis to be the intended sequence.

3. Recombinant production of GLOD

A GLOD producing strain was inoculated to 2.5 ml of LB-amp medium (ampicillin concentration: 50 μg/ml) added to a test tube, and cultured overnight at 160 rpm at 37°C. 2.5 ml of a seed culture solution was inoculated to 250 ml of LB-amp medium (ampicillin concentration: 50 μg/ml) containing 0.1 mM IPTG added to a Sakaguchi flask, and cultured at 130 rpm at 25°C for 16 hours.

The culture solution was centrifuged at 8,000 rpm for 10 minutes and the obtained pellets were resuspended in 4 ml of 10 mM potassium phosphate buffer (PPB), pH 7.5. The bacterial body suspension was ultrasonicated and then centrifuged at 15,000 rpm for 15 minutes, and the obtained supernatant was recovered as the GLOD crude enzyme solution.

4. Measurement of GLOD activity

4-Aminoantipyrine (4-AA) (manufactured by FUJIFILM Wako Pure Chemical Corporation), TOOS (manufactured by Dojindo Laboratories), and horseradish peroxidase (POD) (manufactured by Toyobo Co., Ltd.) were used as reagents for measurement of GLOD activity. The composition of the reagent for activity measurement is shown in Table 2. A GLOD solution was diluted with 10 mM PPB (pH 7.5) containing 0.15% bovine serum albumin (BSA, manufactured by Sigma-Aldrich).

[Table 2]

| Reagent | Volume | Final concentration |
|---|---:|---:|
| 250 mM PPB pH 7.4 | 300 μl | 100 mM |
| 30 mM 4-AA | 12.5 μl | 0.5 mM |
| 15 mM TOOS | 25 μl | 0.5 mM |
| 300 U/ml POD | 12.5 μl | 5 U/ml |
| Deionized water | (375 - V) μl | |
| GLOD solution | V μl | |
| Total volume | 725 μl | |

725 μl of the reagent of Table 2 was kept at a temperature of 30°C for 5 minutes and then supplemented and mixed with 25 μl of 300 mM sodium hydrogen glutamate (GluNa, manufactured by FUJIFILM Wako Pure Chemical Corporation) solution, and the light absorbance at a wavelength of 555 nm ($A_{555}$) was measured using spectrophotometer U-3900 (manufactured by Hitachi High-Tech Science Corporation), while maintaining the cell holder thereof at a temperature of 30°C, to compute the change in $A_{555}$ per minute ($\Delta A_S$). 20 μl of ion exchange water was added instead of the substrate solution (GluNa solution), and the same measurement as above was performed to compute the change in $A_{555}$ per minute ($\Delta A_0$).

Oxidase activity (U/ml) was calculated according to the calculation formula given below. In the formula, "39.2" represents the millimolar extinction coefficient ($mM^{-1}cm^{-1}$) of quinoneimine dye formed by the condensation between 4-AA and TOOS, against light at a wavelength of 555 nm.

[Formula]

$$U/ml = (\Delta A_S - \Delta A_0) \times 600 \times df / (39.2 \times 0.5 \times V)$$
$$= 30.6 \times (\Delta A_S - \Delta A_0) \times df / V.$$

5. Evaluation of thermal stability of GLOD

A GLOD crude enzyme solution was diluted with 10 mM PPB, pH 6.0 such that the final concentration of the GLOD was 0.05 U/ml. Next, 240 μl of 0.05 U/ml GLOD solution and 160 μl of 250 mM PPB, pH 6.0 were mixed and heated for 30 minutes or 35 minutes in a water bath kept at a predetermined temperature. The GLOD solution thus heated was immediately cooled on ice and activity was measured using 375 μl of the GLOD solution. The residual activity of the heated sample was calculated when the activity of a sample cooled on ice without being heated was defined as 1. The residual activity was calculated three times for each GLOD and thermal stability was evaluated from an average value thereof.
Table 3 shows the residual activity of StGLOD deletion mutants heated at 45°C for 30 minutes.

[Table 3]

| Mutant name | Residual activity |
|---|---|
| StGLOD | 0.57 |
| StGLODΔ49Ai | 0.68 |
| StGLODΔ49C | 0.94 |

The residual activities of StGLOD deletion mutants shown in Table 3 were increased by 0.11 to 0.37 compared to StGLOD and the thermal stabilities of all StGLOD deletion mutants were improved. In other words, the residual activities were improved by about 20% and about 65% compared to the wild type.

[Example 2]

6. Preparation of modified StGLODΔ49C

The expression and residual activity of StGLODΔ49C were confirmed. As such, modified mutants based on StGLODΔ49C were further prepared.
A plasmid harboring a gene encoding a modified StGLOD was obtained by site directed mutagenesis using a plasmid for StGLODΔ49C expression (pKK223-3-StGLODΔ49C) as the template. The PCR reaction solution was prepared by mixing 10 μl of KOD one PCR Master Mix (manufactured by Toyobo Co., Ltd.), 3 μl of 2 μM Fw primer, 3 μl of 2 μM Rv primer, 0.5 μl of 40 μg/ml pKK223-3-StGLOD, and 3.5 μl of ion exchange water. Table 4 shows the names of prepared mutants and combinations of Fw and Rv primers. PCR reaction conditions involved a cycle of "98°C for 10 sec → 55°C for 5 sec → 68°C for 35 sec" and this was repeated 15 times. A plasmid for expression of multiple mutation type StGLODΔ49C was constructed by repeating introduction of single mutations. For example, a plasmid for expression of a double mutation type StGLODΔ49C/F87Y/Y103I was constructed by PCR using pKK223-3-StGLODΔ49C/F87Y as the template and primers of SEQ ID NO: 20 and SEQ ID NO: 16.

[Table 4]

| Mutant name | Fw primer | Rv primer |
|---|---|---|
| **StGLODΔ49C/F87Y** | cgtatcaagacgtaccatgcaaaa aaagga (SEQ ID NO: 15) | cgtcttgatacgccctcccacacg gttggc (SEQ ID NO: 14) |
| **StGLODΔ49C/Y103F** | gatccagcgcagttcgcggaagct ggggcg (SEQ ID NO: 17) | ctgcgctggatcagcgaatggaga tggctc (SEQ ID NO: 16) |
| **StGLODΔ49C/Y103L** | gatccagcgcagttagcggaagct ggggcg (SEQ ID NO: 18) | ctgcgctggatcagcgaatggaga tggctc (SEQ ID NO: 16) |

(continued)

| Mutant name | Fw primer | Rv primer |
|---|---|---|
| **StGLODΔ49C/Y103V** | gatccagcgcaggtcgcggaagct ggggcg (SEQ ID NO: 19) | ctgcgctggatcagcgaatggaga tggctc (SEQ ID NO: 16) |
| **StGLODΔ49C/Y103I** | gatccagcgcagatcgcggaagct ggggcg (SEQ ID NO: 20) | ctgcgctggatcagcgaatggaga tggctc (SEQ ID NO: 16) |
| **StGLODΔ49C/Y103M** | gatccagcgcagatggcggaagct ggggcg (SEQ ID NO: 21) | ctgcgctggatcagcgaatggaga tggctc (SEQ ID NO: 16) |
| **StGLODΔ49C/F133Y** | cgccgcctgttttataatgtagat attgac (SEQ ID NO: 23) | aaacaggcggcgttttaagcccag cttgtc (SEQ ID NO: 22) |
| **StGLODΔ49C/F133L** | cgccgcctgtttttaaatgtagat attgac (SEQ ID NO: 24) | aaacaggcggcgttttaagcccag cttgtc (SEQ ID NO: 22) |
| **StGLODΔ49C/F297L** | caccttgcctttttacattcgttt ttgggc (SEQ ID NO: 26) | aaaggcaaggtgcaggcgcgaggt catgtt (SEQ ID NO: 25) |
| **StGLODΔ49C/F297V** | caccttgcctttgttcattcgttt ttgggc (SEQ ID NO: 27) | aaaggcaaggtgcaggcgcgaggt catgtt (SEQ ID NO: 25) |
| **StGLODΔ49C/F297I** | caccttgcctttattcattcgttt ttgggc (SEQ ID NO: 28) | aaaggcaaggtgcaggcgcgaggt catgtt (SEQ ID NO: 25) |
| **StGLODΔ49C/F297M** | caccttgcctttatgcattcgttt ttgggc (SEQ ID NO: 29) | aaaggcaaggtgcaggcgcgaggt catgtt (SEQ ID NO: 25) |
| **StGLODΔ49C/Y376F** | gagggagagcctttcgccgcaact caaacg (SEQ ID NO: 31) | aggctctccctctggaaccgtctg aatggc (SEQ ID NO: 30) |
| **StGLODΔ49C/Y376L** | gagggagagcctttagccgcaact caaacg (SEQ ID NO: 32) | aggctctccctctggaaccgtctg aatggc (SEQ ID NO: 30) |
| **StGLODΔ49C/Y376I** | gagggagagcctatcgccgcaact caaacg (SEQ ID NO: 33) | aggctctccctctggaaccgtctg aatggc (SEQ ID NO: 30) |
| **StGLODΔ49C/Y376M** | gagggagagcctatggccgcaact caaacg (SEQ ID NO: 34) | aggctctccctctggaaccgtctg aatggc (SEQ ID NO: 30) |
| **StGLODΔ49C/F393L** | gttactatccccttaagctcttta cgcttt (SEQ ID NO: 36) | ggggatagtaacaatcgccaggtc tcctgt (SEQ ID NO: 35) |
| **StGLODA49C/F393V** | gttactatccccgttagctcttta cgcttt (SEQ ID NO: 37) | ggggatagtaacaatcgccaggtc tcctgt (SEQ ID NO: 35) |
| **StGLODΔ49C/F393I** | gttactatccccattagctcttta cgcttt (SEQ ID NO: 38) | ggggatagtaacaatcgccaggtc tcctgt (SEQ ID NO: 35) |

(continued)

| Mutant name | Fw primer | Rv primer |
|---|---|---|
| **StGLODΔ49C/F393M** | gttactatccccatgagctcttta cgcttt (SEQ ID NO: 39) | ggggatagtaacaatcgccaggtc tcctgt (SEQ ID NO: 35) |
| **StGLODΔ49C/F428Y** | gtcttacttgaatatagccgtcgt tggtgg (SEQ ID NO: 41) | ttcaagtaagactttggtggcctg atcata (SEQ ID NO: 40) |
| **StGLODΔ49C/F428M** | gtcttacttgaaatgagccgtcgt tggtgg (SEQ ID NO: 42) | ttcaagtaagactttggtggcctg atcata (SEQ ID NO: 40) |
| **StGLODΔ49C/Y467F** | gcgaccaatgcgttcggaggggt agtacg (SEQ ID NO: 44) | cgcattggtcgcaggtggctctgc cgcatc (SEQ ID NO: 43) |
| **StGLODΔ49C/Y517F** | gatgctgagcgctttggctatgcg cttgaa (SEQ ID NO: 46) | gcgctcagcatcgtcaaaagaatc ccaacg (SEQ ID NO: 45) |
| **StGLODΔ49ClY517L** | gatgctgagcgcttaggctatgcg cttgaa (SEQ ID NO: 47) | gcgctcagcatcgtcaaaagaatc ccaacg (SEQ ID NO: 45) |
| **StGLODΔ49C/Y517V** | gatgctgagcgcgttggctatgcg cttgaa (SEQ ID NO: 48) | gcgctcagcatcgtcaaaagaatc ccaacg (SEQ ID NO: 45) |
| **StGLODΔ49C/Y517M** | gatgctgagcgcatgggctatgcg cttgaa (SEQ ID NO: 49) | gcgctcagcatcgtcaaaagaatc ccaacg (SEQ ID NO: 45) |
| **StGLODΔ49C/Y519I** | gagcgctatggcattgcgcttgaa aactta (SEQ ID NO: 51) | gccatagcgctcagcatcgtcaaa agaatc (SEQ ID NO: 50) |
| **StGLODΔ49C/Y519M** | gagcgctatggcatggcgcttgaa aactta (SEQ ID NO: 52) | gccatagcgctcagcatcgtcaaa agaatc (SEQ ID NO: 50) |
| **StGLODΔ49C/Y536L** | attgaggtgttcttaacaggagcg ggacag (SEQ ID NO: 54) | gaacacctcaatacgacggccgtg gacaga (SEQ ID NO: 53) |
| **StGLODΔ49C/Y536M** | attgaggtgttcatgacaggagcg ggacag (SEQ ID NO: 55) | gaacacctcaatacgacggccgtg gacaga (SEQ ID NO: 53) |
| **StGLODΔ49C/F566L** | caaatgaccgcgttacacttagat gtggtg (SEQ ID NO: 57) | cgcggtcatttgatggggtgtata gacagc (SEQ ID NO: 56) |

PCR, recombinant production of GLOD, activity measurement and thermal stability evaluation of the prepared mutants were all carried out in the same manner as in the above StGLODΔ49C. Table 5 shows the residual activity of modified StGLODΔ49C heated at 50°C for 35 minutes.

[Table 5]

| Mutant name | Residual activity |
|---|---|
| StGLODΔ49C | 0.22 |
| StGLODΔ49C/F87Y | 0.61 |
| StGLODΔ49C/Y103F | 0.26 |

(continued)

| Mutant name | Residual activity |
|---|---|
| StGLODΔ49C/Y103L | 0.36 |
| StGLODΔ49C/Y103V | 0.33 |
| StGLODΔ49C/Y1031 | 0.32 |
| StGLODΔ49C/Y103M | 0.32 |
| StGLODΔ49C/F133Y | 0.37 |
| StGLODΔ49C/F133L | 0.24 |
| StGLODΔ49C/F297L | 0.59 |
| StGLODΔ49C/F297V | 0.53 |
| StGLODΔ49C/F297I | 0.75 |
| StGLODΔ49C/F297M | 0.74 |
| StGLODΔ49C/Y376F | 0.27 |
| StGLODΔ49C/Y376L | 0.28 |
| StGLODΔ49C/Y376I | 0.25 |
| StGLODΔ49C/Y376M | 0.27 |
| StGLODΔ49C/F393L | 0.55 |
| StGLODΔ49C/F393V | 0.26 |
| StGLODΔ49C/F393I | 0.25 |
| StGLODΔ49C/F393M | 0.24 |
| StGLODΔ49C/F428Y | 0.79 |
| StGLODΔ49C/F428M | 0.29 |
| StGLODΔ49C/Y467F | 0.25 |
| StGLODΔ49C/Y517F | 0.92 |
| StGLODΔ49C/Y517L | 0.74 |
| StGLODΔ49C/Y517V | 0.94 |
| StGLODΔ49C/Y517M | 0.87 |
| StGLODΔ49C/Y519I | 0.31 |
| StGLODΔ49C/Y519M | 0.26 |
| StGLODΔ49C/Y536L | 0.51 |
| StGLODΔ49C/Y536M | 0.25 |
| StGLODΔ49C/F566L | 0.43 |

The residual activities of the modified StGLODΔ49C shown in Table 5 were increased by 0.02 to 0.72 compared to StGLODΔ49C and the thermal stabilities of all the modified StGLODΔ49Cs were improved. In other words, the residual activities of the prepared modified enzymes were improved by about 9% or more to about 325% or more compared to StGLODΔ49C before modification.

An octuple mutant was prepared by combining 8 types of amino acid substitutions (F87Y, Y103I, F133Y, F297M, F393L, F428Y, Y517F, Y536L), which contributed to improvement of thermal stability of StGLODΔ49C, and this was heated under more harsh heat treatment conditions (60°C, 65°C or 70°C for 35 min) than those of Table 5. Table 6 shows the residual activity of the StGLOD deletion mutant after such heat treatment.

[Table 6]

| Heat treatment condition | Residual activity |
| --- | --- |
| 60°C, 35 min | 0.90 |
| 65°C, 35 min | 0.78 |
| 70°C, 35 min | 0.36 |

The octuple mutant of StGLODΔ49C shown in Table 6 was stable even when heated at 60°C for 35 minutes and 1/3 or more of the activity remained even when the mutant was heated at 70°C for 35 minutes. It became evident that the thermal stability of StGLODΔ49C was drastically improved by combining the amino acid substitutions shown in Table 5.

7. Construction of plasmid for expression of StGLOD homolog(s) having thermal stability improving mutation

A putative glutamate oxidase from *Streptomyces* sp. MOE7 (M7GLOD) having the amino acid sequence of SEQ ID NO: 58 was used and designed so as to have an amino acid sequence having the same deletion as that of StGLODΔ49C (M7GLODΔ49C, SEQ ID NO: 59) and further designed so as to have an amino acid sequence having 8 types of amino acid substitutions (F87Y, Y103I, F133Y, F297M, F393L, F428Y, Y517F, Y536L) introduced therein (M7GLODΔ49C-T8, SEQ ID NO: 60).
A plasmid (pET22b-M7GLODΔ49C-T8) for M7GLODΔ49C-T8 expression was prepared using In-Fusion HD Cloning Kit (manufactured by Clontech Laboratories, Inc.). A GLOD gene having the nucleotide sequence of SEQ ID NO: 61 was divided into 3 fragments of SEQ ID NO: 62 (M7GLODΔ49C-T8-f1), SEQ ID NO: 63 (M7GLODA49C-T8-f2) and SEQ ID NO: 64 (M7GLODΔ49C-T8-f3) and their synthesis was commissioned to Integrated DNA Technologies, Inc. Fifteen 5' terminal bases of M7GLODΔ49C-T8-fl are an overlapping sequence for assembly with pET-22b(+). Fifteen 3' terminal bases of M7GLODΔ49C-T8-f1 and fifteen 5' terminal bases of M7GLODΔ49C-T8-f2 are overlapping sequences for gene assembly. Fifteen 3' terminal bases of M7GLODΔ49C-T8-f2 and fifteen 5' terminal bases of M7GLODΔ49C-T8-f3 are overlapping sequences for gene assembly. Fifteen 3' terminal bases of M7GLODΔ49C-T8-f3 are an overlapping sequence for assembly with pET-22b(+).
A plasmid fragment was prepared by PCR using pET-22b(+) plasmid as the template and primers of SEQ ID NO: 65 (catatgtatatctccttcttaaag) and SEQ ID NO: 66 (taacaaagcccgaaaggaag). The solution after PCR was supplemented with 1.0 μl of DpnI (manufactured by New England BioLabs Inc.) and treated at 37°C for 1 hour, and then, the amplified fragment was purified using GFX PCR DNA and Gel Band Purification Kit (manufactured by Cytiva).
The plasmid (pET22b-M7GLODΔ49C-T8) for M7GLODΔ49C-T8 expression was obtained through reaction at 50°C for 15 minutes according to the composition of Table 7. E. *coli* JM109 strain was transformed with the obtained plasmid. The obtained transformant was cultured, and the nucleotide sequence of an extracted plasmid was confirmed by DNA sequence analysis to be the intended sequence. Then, *E. coli* BL21 (DE3) strain was transformed with pET22b-M7GLODΔ49C-T8 to prepare a M7GLODΔ49C-T8 producing strain.

[Table 7]

| Reagent | Volume |
| --- | --- |
| 5×In-Fusion HD Enzyme Premix | 2 μl |
| 50 ng/μl pET-22b(+) fragment | 2 μl |
| 10 ng/μl M7GLODΔ49C-8T-f1 | 2 μl |
| 10 ng/μl M7GLODΔ49C-8T-f2 | 2 μl |
| 10 ng/μl M7GLODΔ49C-8T-f3 | 2 μl |
| Total volume | 10 μl |

8. Recombinant production of M7GLODΔ49C-T8

A M7GLODΔ49C-T8 producing strain was inoculated to 2.5 ml of LB-amp medium (ampicillin concentration: 50 μg/ml) added to a test tube, and cultured overnight at 180 rpm at 37°C. 2.5 ml of a seed culture solution was inoculated to 250 ml of LB-amp medium (ampicillin concentration: 100 μg/ml) added to a Sakaguchi flask, and cultured at 130 rpm at 37°C. At the stage where OD600 of the culture solution reached 0.6 to 1.0, isopropyl-β-D-thiogalactopyranoside (IPTG) was added at a final concentration of 1 mM, followed by culture at 130 rpm at 15°C for 16 hours.

The culture solution was centrifuged at 8,000 rpm for 10 minutes and the obtained pellets were resuspended in 4 ml of 10 mM potassium phosphate buffer (PPB), pH 6.0. The bacterial body suspension was ultrasonicated and then centrifuged at 15,000 rpm for 15 minutes, and the obtained supernatant was recovered to prepare a M7GLODΔ49C-T8 crude enzyme solution.

M7GLODΔ49C-T8 was heated at 60°C or 65°C for 35 minutes and the residual activity thereof was calculated in accordance with the above method (Table 8).

[Table 8]

| Heat treatment condition | Residual activity |
|---|---|
| 60°C, 35 min | 0.88 |
| 65°C, 35 min | 0.55 |

As shown in Table 8, M7GLODΔ49C-T8 heated at 60°C for 35 minutes was stable and 1/2 or more of the activity remained after heating at 65°C for 35 minutes, indicating high thermal stability. It was demonstrated that the thermal stability of GLOD was improved by combining the amino acid substitutions shown in Table 4, without being limited to StGLOD.

9. Preparation of FAD-GLDH based on GLOD

Next, FAD-GLDH was prepared by introducing an amino acid substitution to GLOD.

A mutation in which leucine at the 545th position of SEQ ID NO: 58 is substituted with another amino acid was introduced to a gene encoding GLOD. A plasmid carrying a gene encoding mutation type M7GLODΔ49C-T8 was obtained by site directed mutagenesis with a plasmid for M7GLODΔ49C-T8 expression (pET22b-M7GLODΔ49C-T8) as the template. The PCR reaction solution was prepared by mixing 10 μl of KOD One PCR Master Mix (manufactured by Toyobo Co., Ltd.), 3 μl of 2 μM Fw primer, 3 μl of 2 μM Rv primer, 0.5 μl of 40 μg/ml pET22b-M7GLODΔ49C-T8, and 3.5 μl of ion exchange water. Table 9 shows the names of prepared mutants and combinations of Fw and Rv primers. Leucine at the 545th position of SEQ ID NO: 58 corresponds to leucine at the 496th position in M7GLODΔ49C-T8 (SEQ ID NO: 60). PCR reaction conditions involved a cycle of "98°C for 10 sec → 55°C for 5 sec → 68°C for 35 sec" and this was repeated 15 times. A plasmid for expression of multiple mutation type M7GLODΔ49C was constructed by repeating introduction of single mutations. For example, a plasmid for expression of a double mutation type pET22b-M7GLODΔ49C-T8/L496A/L425Q was constructed by PCR using pET22b-M7GLODΔ49C-T8/L496A as the template and primers of SEQ ID NO: 75 and SEQ ID NO: 77.

Likewise, a mutation in which methionine at the 109th position of SEQ ID NO: 58 is substituted with another amino acid was introduced to a gene encoding GLOD. The template plasmid, PCR reaction solution composition, and reaction conditions for site-directed mutagenesis were the same as those for the substitution of leucine at the 545th position (in M7GLODΔ49C-T8, leucine at the 496th position). Table 9 shows the names of prepared mutants and combinations of Fw and Rv primers.

[Table 9]

| Mutant name | Fw primer | Rv primer |
|---|---|---|
| M7GLODΔ49C-T8/L496A | GGTGGTGTGGTTgcgGCCGCGTAT AGTTGG (SEQ ID NO: 67) | AACCACACCACCCTTAGAAC (SEQ ID NO: 76) |
| M7GLODΔ49C-T8/L496G | GGTGGTGTGGTTggtGCCGCGTAT AGTTGG (SEQ ID NO: 68) | AACCACACCACCCTTAGAAC (SEQ ID NO: 76) |
| M7GLODΔ49C-T8/L496V | GGTGGTGTGGTTgtgGCCGCGTAT AGTTGG (SEQ ID NO: 69) | AACCACACCACCCTTAGAAC (SEQ ID NO: 76) |
| M7GLODΔ49C-T8/L496M | GGTGGTGTGGTTatgGCCGCGTAT AGTTGG (SEQ ID NO: 70) | AACCACACCACCCTTAGAAC (SEQ ID NO: 76) |
| M7GLODΔ49C-T8/L496N | GGTGGTGTGGTTaacGCCGCGTAT AGTTGG (SEQ ID NO: 71) | AACCACACCACCCTTAGAAC (SEQ ID NO: 76) |

(continued)

| Mutant name | Fw primer | Rv primer |
|---|---|---|
| M7GLODΔ49C-T8/L496C | GGTGGTGTGGTTtgcGCCGCGTAT AGTTGG (SEQ ID NO: 72) | AACCACACCACCCTTAGAAC (SEQ ID NO: 76) |
| M7GLODΔ49C-T8/L496S | GGTGGTGTGGTTtcgGCCGCGTAT AGTTGG (SEQ ID NO: 73) | AACCACACCACCCTTAGAAC (SEQ ID NO: 76) |
| M7GLODΔ49C-T8/L496T | GGTGGTGTGGTTacgGCCGCGTAT AGTTGG (SEQ ID NO: 74) | AACCACACCACCCTTAGAAC (SEQ ID NO: 76) |
| M7GLODΔ49C-T8/L425Q | GCAACAAAAGTGcagTTAGAGTAT TCTCGT (SEQ ID NO: 75) | CACTTTTGTTGCCTGATC (SEQ ID NO: 77) |
| M7GLODΔ49C-T8/M109A | GAGGCGGGAGCTGCGCGTTTGCCT TCATTC (SEQ ID NO: 78) | AGCTCCCGCCTCTGCAATTTGAG CCGG (SEQ ID NO: 88) |
| M7GLODΔ49C-T8/M109C | GAGGCGGGAGCTTGCCGTTTGCCT TCATTC (SEQ ID NO: 79) | AGCTCCCGCCTCTGCAATTTGAG CCGG (SEQ ID NO: 88) |
| M7GLODΔ49C-T8/M109V | GAGGCGGGAGCTGTGCGTTTGCCT TCATTC (SEQ ID NO: 80) | AGCTCCCGCCTCTGCAATTTGAG CCGG (SEQ ID NO: 88) |
| M7GLODΔ49C-T8/M109F | GAGGCGGGAGCTTTTCGTTTGCCT TCATTC (SEQ ID NO: 81) | AGCTCCCGCCTCTGCAATTTGAG CCGG (SEQ ID NO: 88) |
| M7GLODΔ49C-T8/M109Y | GAGGCGGGAGCTTATCGTTTGCCT TCATTC (SEQ ID NO: 82) | AGCTCCCGCCTCTGCAATTTGAG CCGG (SEQ ID NO: 88) |
| M7GLODΔ49C-T8/M109W | GAGGCGGGAGCTTGGCGTTTGCCT TCATTC (SEQ ID NO: 83) | AGCTCCCGCCTCTGCAATTTGAG CCGG (SEQ ID NO: 88) |
| M7GLODΔ49C-T8/M109S | GAGGCGGGAGCTTCGCGTTTGCCT TCATTC (SEQ ID NO: 84) | AGCTCCCGCCTCTGCAATTTGAG CCGG (SEQ ID NO: 88) |
| M7GLODΔ49C-T8/M109T | GAGGCGGGAGCTACGCGTTTGCCT TCATTC (SEQ ID NO: 85) | AGCTCCCGCCTCTGCAATTTGAG CCGG (SEQ ID NO: 88) |
| M7GLODΔ49C-T8/M109Q | GAGGCGGGAGCTCAGCGTTTGCCT TCATTC (SEQ ID NO: 86) | AGCTCCCGCCTCTGCAATTTGAG CCGG (SEQ ID NO: 88) |
| M7GLODΔ49C-T8/M109H | GAGGCGGGAGCTCATCGTTTGCCT TCATTC (SEQ ID NO: 87) | AGCTCCCGCCTCTGCAATTTGAG CCGG (SEQ ID NO: 88) |

The solution after PCR was supplemented with 1 μl of DpnI and treated at 37°C for 1 hour to degrade the template plasmid. *E. coli* JM109 strain was transformed with the obtained solution treated with DpnI. The obtained transformant was cultured, and the nucleotide sequence of the extracted plasmid was confirmed by DNA sequence analysis to be the intended sequence. Then, *E. coli* BL21 (DE3) strain was transformed with the obtained plasmid to prepare a mutation type M7GLODΔ49C-T8 producing strain.

The prepared mutation type M7GLODΔ49C-T8 was recombinantly produced by the same method as in the above M7GLODΔ49C-T8.

The oxidase activity (OD) and dehydrogenase activity (DH) of the mutant after substitution were measured. OD was evaluated in the same manner as in the case for M7GLODΔ49C-T8 above.

10. Measurement of DH

Dichloroindophenol (DCIP) (manufactured by FUJIFELM Wako Pure Chemical Corporation) and 1-methoxy PMS (mPMS) (manufactured by Dojindo Laboratories) were used as reagents for measurement of GLDH activity. The composition of the reagents for activity measurement is shown in Table 10. A GLOD solution was diluted with 10 mM PPB (pH 7.4) containing 0.15% bovine serum albumin (BSA, manufactured by Sigma-Aldrich).

[Table 10]

| Reagent | Volume | Final concentration |
|---|---|---|
| 250 mM KPB pH 7.4 | 300 μl | 100 mM |
| 30 mM mPMS | 25 μl | 1 mM |
| 3 mM DCIP | 25 μl | 0.1 mM |
| Deionized water | (375 - V) μl | |
| GLOD solution | V μl | |
| Total volume | 725 μl | |

725 μl of the reagent of Table 10 was kept at a temperature of 30°C for 5 minutes and then supplemented and mixed with 25 μl of 300 mM sodium hydrogen glutamate (GluNa, manufactured by FUJIFILM Wako Pure Chemical Corporation) solution, and the light absorbance at a wavelength of 600 nm ($A_{600}$) was measured using spectrophotometer U-3900 (manufactured by Hitachi High-Tech Science Corporation), while maintaining the cell holder thereof at a temperature of 30°C, to compute the decrease in $A_{600}$ per minute ($\Delta A_s$). 25 μl of ion exchange water was added instead of the substrate solution (GluNa solution), and the same measurement as above was performed to compute the change in $A_{600}$ per minute ($\Delta A_0$).

Dehydrogenase (DH) activity (U/ml) was calculated according to the calculation formula given below. In the formula, "21" represents an estimated value of the millimolar extinction coefficient ($mM^{-1}cm^{-1}$) of DCIP at pH 7.4 against light at a wavelength of 600 nm.

[Formula]

$$U/ml = -(\Delta A_S - \Delta A_0) \times 750 \times df / (21 \times V)$$

$$= -35.7 \times (\Delta A_S - \Delta A_0) \times df / V.$$

The ratio of OD to DH (OD/DH) of each GLOD was calculated and is shown in Table 11 below. The OD/DH of GLOD before mutation was 32.4, whereas, surprisingly, the OD/DH of a mutant having a substitution added at the position of L545 was decreased and the dehydrogenase activity per oxidase activity was greatly improved. In terms of the ratio (DH/OD) of dehydrogenase activity against oxidase activity, particularly, improvement was seen by about 22 fold in the L496A mutant and by about 65 fold in the L496G mutant. Further, marked improvement was seen in the double mutant L496A/L425Q. It is believed that when L425Q is combined with other mutations, it is highly plausible that similar improvement is brought about.

Surprisingly, the OD/DH of a mutant having a substitution added at the position of M109 was decreased and the dehydrogenase activity per oxidase activity was significantly improved. In terms of the ratio (DH/OD) of dehydrogenase activity against oxidase activity, improvement was seen by about 29, 77, 163, 195, 284, 364, 470, 1080 or 2310 fold in a mutant having a substitution of M109 with F, C, A, V, Q, T, H, Y or S. In Particular, for mutant M109W, the oxidase activity disappeared completely and the converted mutant exhibited only dehydrogenase activity.

[Table 11]

| | OD/DH |
|---|---|
| M7GLODΔ49C-T8 | 32.4 |
| M7GLODΔ49C-T8/L496A | 1.42 |
| M7GLODΔ49C-T8/L496G | 0.493 |
| M7GLODΔ49C-T8/L496V | 18.6 |

(continued)

| | OD/DH |
|---|---|
| M7GLODΔ49C-T8/L496M | 9.03 |
| M7GLODΔ49C-T8/L496N | 5.69 |
| M7GLODΔ49C-T8/L496C | 1.71 |
| M7GLODΔ49C-T8/L496S | 1.59 |
| M7GLODΔ49C-T8/L496T | 6.07 |
| M7GLODΔ49C-T8/L496A/L425Q | 0.194 |
| M7GLODΔ49C-T8/M109A | 0.199 |
| M7GLODΔ49C-T8/M109C | 0.422 |
| M7GLODΔ49C-T8/M109V | 0.166 |
| M7GLODΔ49C-T8/M109F | 1.13 |
| M7GLODΔ49C-T8/M109Y | 0.030 |
| M7GLODΔ49C-T8/M109W | 0.000 |
| M7GLODΔ49C-T8/M109S | 0.014 |
| M7GLODΔ49C-T8/M109T | 0.089 |
| M7GLODΔ49C-T8/M109Q | 0.114 |
| M7GLODΔ49C-T8/M109H | 0.069 |

By aligning the amino acid sequences of the GLODs using a known algorithm such as the Lipman-Pearson method, the positions of the homologous amino acid residues in each of the GLOD sequences can be determined, regardless of insertion or deletion of amino acid residues in the amino acid sequences. Corresponding positions (homologous positions) are considered to exist at the same positions in the three-dimensional structures, and amino acid residues at such homologous positions are believed to exert similar roles in the respective GLODs. The present inventors prepared a glutamate dehydrogenase using FAD as a coenzyme by substituting the amino acid at the position corresponding to the 545th position of SEQ ID NO: 58 as to a GLOD using FAD as a coenzyme. A glutamate dehydrogenase using FAD as a coenzyme was further prepared by substituting the amino acid at the position corresponding to the 425th position of SEQ ID NO: 58. In addition, a glutamate dehydrogenase using FAD as a coenzyme was further prepared by substituting the amino acid at the position corresponding to the 109th position of SEQ ID NO: 58. Thus, based on these findings, it is believed that it is highly plausible that an enzyme having dehydrogenase activity can also be obtained by introducing an amino acid substitution to a corresponding position as to other GLODs.

11. Improvement in heat resistance of M7GLODΔ49C-T8

A plasmid (pET22b-M7GLODΔ49C-T7) carrying a gene encoding M7GLODΔ49C-T8 not comprising the amino acid substitution Y536L was obtained in accordance with the method described in "6. Preparation of modified StGLODΔ49C" using pET22b-M7GLODΔ49C-T8 as the template and primers of SEQ ID NO: 89 and SEQ ID NO: 90. M7GLODΔ49C-T8 not comprising the amino acid substitution Y536L was designated as M7GLODΔ49C-T7 (SEQ ID NO: 91).

A plasmid for expression of each modified M7GLODΔ49C-T7 was constructed with the same method as in "6. Preparation of modified StGLODΔ49C" using pET22b-M7GLODΔ49C-T7 as the template and primers of Table 12. Table 12 shows the names of prepared mutants and combinations of Fw and Rv primers.

[Table 12]

| Mutant name | Fw primer | Rv primer |
|---|---|---|
| M7GLODΔ49C-T7/R186G | attcgtaccaatggcactcaagtacgtcgc (SEQ ID NO: 92) | attggtacgaatccaagtgtggttacg (SEQ ID NO: 106) |
| M7GLODΔ49C-T7/R186A | attcgtaccaatgcgactcaagtacgtcgc (SEQ ID NO: 93) | attggtacgaatccaagtgtggttacg (SEQ ID NO: 106) |

(continued)

| Mutant name | Fw primer | Rv primer |
|---|---|---|
| M7GLODΔ49C-T7/R186C | attcgtaccaattgcactcaagtacgtcgc (SEQ ID NO: 94) | attggtacgaatccaagtgtggttacg (SEQ ID NO: 106) |
| M7GLODΔ49C-T7/R186L | attcgtaccaatctgactcaagtacgtcgc (SEQ ID NO: 95) | attggtacgaatccaagtgtggttacg (SEQ ID NO: 106) |
| M7GLODΔ49C-T7/R186M | attcgtaccaatatgactcaagtacgtcgc (SEQ ID NO: 96) | attggtacgaatccaagtgtggttacg (SEQ ID NO: 106) |
| M7GLODΔ49C-T7/R186F | attcgtaccaattttactcaagtacgtcgc (SEQ ID NO: 97) | attggtacgaatccaagtgtggttacg (SEQ ID NO: 106) |
| M7GLODΔ49C-T7/R186Y | attcgtaccaattatactcaagtacgtcgc (SEQ ID NO: 98) | attggtacgaatccaagtgtggttacg (SEQ ID NO: 106) |
| M7GLODΔ49C-T7/R186S | attcgtaccaatagcactcaagtacgtcgc (SEQ ID NO: 99) | attggtacgaatccaagtgtggttacg (SEQ ID NO: 106) |
| M7GLODΔ49C-T7/R186T | attcgtaccaataccactcaagtacgtcgc (SEQ ID NO: 100) | attggtacgaatccaagtgtggttacg (SEQ ID NO: 106) |
| M7GLODΔ49C-T7/R186N | attcgtaccaataacactcaagtacgtcgc (SEQ ID NO: 101) | attggtacgaatccaagtgtggttacg (SEQ ID NO: 106) |
| M7GLODΔ49C-T7/R186Q | attcgtaccaatcagactcaagtacgtcgc (SEQ ID NO: 102) | attggtacgaatccaagtgtggttacg (SEQ ID NO: 106) |
| M7GLODΔ49C-T7/R186H | attcgtaccaatcatactcaagtacgtcgc (SEQ ID NO: 103) | attggtacgaatccaagtgtggttacg (SEQ ID NO: 106) |
| M7GLODΔ49C-T7/R186D | attcgtaccaatgatactcaagtacgtcge (SEQ ID NO: 104) | attggtacgaatccaagtgtggttacg (SEQ ID NO: 106) |
| M7GLODΔ49C-T7/R186E | attcgtaccaatgaaactcaagtacgtcge (SEQ ID NO: 105) | attggtacgaatccaagtgtggttacg (SEQ ID NO: 106) |

Next, *E. coli* BL21 (DE3) strain was transformed with pET22b-M7GLODΔ49C-T7 or the plasmid for expression of each modified M7GLODΔ49C-T7 to prepare strains producing M7GLODΔ49C-T7 or each modified M7GLODΔ49C-T7.

12. Recombinant production of M7GLODΔ49C-T7 and mutant thereof, and thermal stability evaluation

Strains producing a M7GLODΔ49C-T7 or modified M7GLODΔ49C-T7 was inoculated to 2.5 ml of LB-amp medium (ampicillin concentration: 50 μg/ml) added to a test tube, and cultured overnight at 180 rpm at 37°C. 2.5 ml of a seed culture solution was inoculated to 250 ml of LB-amp medium (ampicillin concentration: 100 μg/ml) added to a Sakaguchi flask, and cultured at 130 rpm at 37°C. At the stage where OD600 of the culture solution reached 0.6 to 1.0, isopropyl-β-D-thiogalactopyranoside (IPTG) was added at a final concentration of 1 mM or 0.1 mM, followed by culture at 130 rpm at 15°C for 16 hours.

The culture solution was centrifuged at 8,000 rpm for 10 minutes and the obtained pellets were resuspended in 4 to 25 ml of 10 mM potassium phosphate buffer (PPB), pH 6.0. The bacterial body suspension was ultrasonicated and then centrifuged at 15,000 rpm for 15 minutes, and the obtained supernatant was recovered to prepare a M7GLODΔ49C-T7 or modified M7GLODΔ49C-T7 crude enzyme solution.

A crude enzyme solution of M7GLODΔ49C-T7 or modified M7GLODΔ49C-T7 was diluted with 10 mM PPB, pH 6.0 such that the final concentration of the GLOD was 0.05 U/ml. Next, 240 μl of 0.05 U/ml GLOD solution and 160 μl of 250 mM PPB, pH 6.0 were mixed and heated for 30 minutes in a water bath kept at a predetermined temperature. The residual activity thereof was calculated in accordance with the above method (Table 13).

The residual activity of M7GLODΔ49C-T7 after being heated at 60°C for 30 minutes was 0.93, and the residual activity of M7GLODΔ49C-T7 after being heated at 65°C for 30 minutes was 0.69, indicating high thermal stability.

[Table 13]

| Mutant name | Residual activity after treatment at 70°C for 30 min |
|---|---|
| M7GLODΔ49C-T7 | 0.17 |
| M7GLODΔ49C-T7/R186G | 0.33 |
| M7GLODΔ49C-T7/R186A | 0.43 |
| M7GLODΔ49C-T7/R186C | 0.26 |
| M7GLODΔ49C-T7/R186L | 0.29 |
| M7GLODΔ49C-T7/R186M | 0.29 |
| M7GLODΔ49C-T7/R186F | 0.22 |
| M7GLODΔ49C-T7/R186Y | 0.56 |
| M7GLODΔ49C-T7/R186S | 0.37 |
| M7GLODΔ49C-T7/R186T | 0.25 |
| M7GLODΔ49C-T7/R186N | 0.54 |
| M7GLODΔ49C-T7/R186Q | 0.54 |
| M7GLODΔ49C-T7/R186H | 0.34 |
| M7GLODΔ49C-T7/R186D | 0.66 |
| M7GLODΔ49C-T7/R186E | 0.75 |

Furthermore, the residual activities of each modified M7GLODΔ49C-T7 were improved by 0.05 to 0.58 compared to M7GLODΔ49C-T7 and the thermal stabilities of all modified M7GLODΔ49C-T7 were improved. In other words, the residual activities of the prepared modified enzymes were improved by about 29% to about 341% compared to M7GLODΔ49C-T7 before modification. It is believed that when these amino acid substitutions are introduced to GLDH, it is highly plausible that the thermal stability of the enzyme after modification also improves compared to GLDH before modification.

13. Freeze drying of GLOD

A solution of M7GLODΔ49C-T7/R186E in a 5 mM potassium phosphate buffer, pH 6.0 was prepared such that the amount of GLOD enzyme activity per mL was 240 U/mL. The enzyme solution was supplemented with each 30 mg/mL solution of the trisaccharide D-raffinose, the monosaccharide D-glucose, or the sugar alcohol xylitol dissolved in the same buffer as above to adjust the final concentration of GLOD to 200 U/mL and the final concentration of each stabilizer to 5 mg/mL. This was dispensed at 50 μL/tube to 200 μL tubes for PCR and freeze-dried. Three samples were prepared as to each concentration.

This freeze-dried product was stored at 37°C for 2 weeks and then thawed in water to adjust the putative concentration to 5 U/mL. The resulting product was further diluted with a 10 mM potassium phosphate buffer, pH 7.4, containing 0.1% BSA to attain a putative concentration of 0.25 U/mL and the activity thereof was measured.

In a composition supplemented with D-raffinose, no reduction in GLOD activity was seen and the residual activity ratio after storage at 37°C for 2 weeks was 106.5%, indicating very favorable stability. On the other hand, with regard to a composition supplemented with D-glucose, the residual activity ratio was 8.5%; with regard to a composition supplemented with xylitol, the residual activity ratio was 48.9%; and it was found that these compositions were unstable. For GLDH, it is believed that it is highly plausible that by adding D-raffinose to the composition, the residual activity of the enzyme after long term storage is maintained or is unlikely to be reduced compared to a composition not comprising raffinose or a composition comprising D-glucose or xylitol.

Industrial Applicability

The present disclosure provides an unprecedented type of glutamate dehydrogenase. This can be utilized for detection of glutamic acid or dehydrogenation reaction. Furthermore, large scale production of the glutamate dehydrogenase is possible.

Various literatures including patent applications and manufacturers' manuals are cited in the present specification. The disclosure of these literatures should not be interpreted as being related to the patentability of the present

disclosure and however, is incorporated herein by reference in their entirety. More specifically, all references are incorporated herein by reference, as in the case of specifically and individually stating that each literature is incorporated by reference.

A corresponding position in each sequence is shown in the tables below.

[Table 14]

| Name | StGLOD | StGLODΔ49Ai | StGLODΔ49C | M7GLOD | M7GLODΔ49C | M7GLODΔ49C-T8 | M7GLODΔ49C-T7 |
|---|---|---|---|---|---|---|---|
| Origin | *Streptomyces* sp. X-119-6 | *Streptomyces* sp. X-119-6 | *Streptomyces* sp. X-119-6 | *Streptomyces* sp. MOE7 | *Streptomyces* sp. MOE7 | *Streptomyces* sp. MOE7 | *Streptomyces* sp. MOE7 |
| SEQ ID NO / aa position | SEQ 1 | SEQ 12 | SEQ 13 | SEQ 58 | SEQ 59 | SEQ 60 | SEQ 91 |
| 87 | F87 | F87 | F87 | F87 | F87 | F87 | F87 |
| 103 | Y103 | Y103 | Y103 | Y103 | Y103 | I103 | I103 |
| 133 | F133 | F133 | F133 | F133 | F133 | Y133 | Y133 |
| 186 | R186 | R186 | R186 | R186 | R186 | R186 | R186 |
| 297 | F297 | F297 | F297 | F297 | F297 | M297 | M297 |
| 376 | Y376 | Y376 | Y376 | S376 | S376 | S376 | S376 |
| 393 | F393 | F393 | F393 | F393 | F393 | L393 | L393 |
| 428 | F428 | F428 | F428 | F428 | F428 | Y428 | Y428 |
| 516 | Y516 | Y467 | Y467 | H516 | H467 | H467 | H467 |
| 566 | Y566 | Y517 | Y517 | Y566 | Y517 | F517 | F517 |
| 568 | Y568 | Y519 | Y519 | Y568 | Y519 | Y519 | Y519 |
| 585 | Y585 | Y536 | Y536 | Y585 | Y536 | L536 | Y536 |
| 615 | F615 | F566 | F566 | F615 | F566 | F566 | F566 |
| 545 | L545 | L496 | L496 | L545 | L496 | L496 | L496 |
| 425 | L425 | L425 | L425 | L425 | L425 | L425 | L425 |
| 109 | M109 | M109 | M109 | M109 | M109 | M109 | M109 |

[Table 15]

| Name | StGLOD | StGLODΔ49Ai | StGLODΔ49C | M7GLOD | M7GLODΔ49C | M7GLODΔ49C-T8 | M7GLODΔ49C-T7 |
|---|---|---|---|---|---|---|---|
| Origin | *Streptomyces* sp. X-119-6 | *Streptomyces* sp. X-119-6 | *Streptomyces* sp. X-119-6 | *Streptomyces* sp. MOE7 | *Streptomyces* sp. MOE7 | *Streptomyces sp.* MOE7 | *Streptomyces sp.* MOE7 |
| SEQ ID NO / aa position | SEQ 1 | SEQ 12 | SEQ 13 | SEQ 58 | SEQ 59 | SEQ 60 | SEQ 91 |
| 106 | A106 | A106 | A106 | A106 | A106 | A106 | A106 |
| 210 | C210 | C210 | C210 | C210 | C210 | C210 | C210 |
| 235 | Q235 | Q235 | Q235 | Q235 | Q235 | Q235 | Q235 |
| 236 | D236 | D236 | D236 | S236 | S236 | S236 | S236 |
| 237 | D237 | D237 | D237 | D237 | D237 | D237 | D237 |

| | | | | | | | |
|---|---|---|---|---|---|---|---|
| 244 | P244 | P244 | P244 | P244 | P244 | P244 | P244 |
| 311 | T311 | T311 | T311 | T311 | T311 | T311 | T311 |
| 313 | W313 | W313 | W313 | W313 | W313 | W313 | W313 |
| 333 | Q333 | Q333 | Q333 | Q333 | Q333 | Q333 | Q333 |
| 334 | I334 | I334 | I334 | I334 | I334 | I334 | I334 |
| 336 | M336 | M336 | M336 | M336 | M336 | M336 | M336 |
| 338 | Q338 | Q338 | Q338 | Q338 | Q338 | Q338 | Q338 |
| 339 | R339 | R339 | R339 | R339 | R339 | R339 | R339 |
| 416 | T416 | T416 | T416 | T416 | T416 | T416 | T416 |
| 438 | A438 | A438 | A438 | E438 | E438 | E438 | E438 |
| 441 | K441 | K441 | K441 | K441 | K441 | K441 | K441 |
| 455 | Y455 | Y455 | Y455 | Y455 | Y455 | Y455 | Y455 |
| 456 | Q456 | R456 | Q456 | Q456 | Q456 | Q456 | Q456 |
| 457 | Q457 | K457 | D457 | R457 | D457 | D457 | D457 |
| 545 | L545 | L496 | L496 | L545 | L496 | L496 | L496 |
| 598 | P598 | P549 | P549 | P598 | P549 | P549 | P549 |
| 601 | C601 | C552 | C552 | C601 | C552 | C552 | C552 |
| 609 | P609 | P560 | P560 | P609 | P560 | P560 | P560 |

[Sequence Listing]

SEQ ID NO: 1 Amino acid sequence of the glutamate oxidase from *Streptomyces* sp. X-119-6 (StGLOD)

MNEMTYEQLARELLLVGPAPTNEDLKLRYLDVLIDNGLNPPGPPKRILIVGAGIAGLV AG

DLLTRAGHDVTILEANANRVGGRIKTFHAKKGEPSPFADPAQYAEAGAMRLPSFHPL TLA

LIDKLGLKRRLFFNVDIDPQTGNQDAPVPPVFYKSFKDGKTWTNGAPSPEFKEPDKRN HT

WIRTNREQVRRAQYATDPSSINEGFHLTGCETRLTVSDMVNQALEPVRDYYSVKQDD GTR

VNKPFKEWLAGWADVVRDFDGYSMGRFLREYAEFSDEAVEAIGTIENMTSRLHLAFF HSF

LGRSDIDPRATYWEIEGGSRMLPETLAKDLRDQIVMGQRMVRLEYYDPGRDGHHGE LTGP

GGPAVAIQTVPEGEPYAATQTWTGDLAIVTIPFSSLRFVKVTPPFSYKKRRAVIETHYD Q

ATKVLLEFSRRWWEFTEADWKRELDAIAPGLYDYYQQWGEDDAEAALALPQSVRNLPTGL

LGAHPSVDESRIGEEQVEYYRNSELRGGVRPATNAYGGGSTTDNPNRFMYYPSHPVPGTQ

GGVVLAAYSWSDDAARWDSFDDAERYGYALENLQSVHGRRIEVFYTGAGQTQSWLRDPYA

CGEAAVYTPHQMTAFHLDVVRPEGPVYFAGEHVSLKHAWIEGAVETAVRAAIAVNEAPVG

DTGVTAAAGRRGAAAATEPMREEALTS

SEQ ID NO: 2 Nucleotide sequence of the glutamate oxidase from *Streptomyces* sp. X-119-6 (StGLOD)

atgaatgaaatgacctacgagcaattggcccgcgaattgttattggttggtccggccccgacgaacgaggatcttaaattacgctatttgg

atgtgctgattgataacgggctgaacccgccgggtccgcctaagcgtatcttgatcgtcggggctggtattgcaggccttgttgccggag

atttgttgacccgcgctgggcatgatgttactattcttgaagctaatgccaaccgtgtgggagggcgtatcaagacgttccatgcaaaaaa

aggagagccatctccattcgctgatccagcgcagtacgcggaagctggggcgatgcgtcttccttcctttcacccacttaccttggcactt

atcgacaagctgggcttaaaacgccgcctgtttttaatgtagatattgacccacaaactggaaatcaagatgctccagtcccaccggtctt

ctataagtcttttaaggacggcaagacgtggacaaatggagccccgagccctgagtttaaagagcccgataaacgtaatcacacctgga

tccgtacgaatcgcgaacaagtacgccgcgcccaatacgccacagacccgagctccatcaatgaaggcttccatctgacgggatgtga

gacccgccttactgtctccgacatggttaaccaagcgcttgaaccggtacgcgactattattcagtcaagcaggacgatggaactcgtgt

gaataagcctttcaaagaatggcttgcagggtgggccgacgtcgtgcgtgatttcgacggttattctatgggccgcttcttacgtgaatatg

ccgagttttcggatgaggcagtggaagcaattggcacaattgaaaacatgacctcgcgcctgcaccttgcctttttcattcgtttttgggcc

gttccgatatcgatccgcgcgccacttactgggagatcgagggggggtcacgcatgcttcctgaaacactggcaaaggacctgcgcga

tcagatcgttatgggtcagcgcatggtacgcttagagtactatgacccgggccgcgatggccatcatggtgagcttactgggcctgggg

gtccggccgtggccattcagacggttccagagggagagccttacgccgcaactcaaacgtggacaggagacctggcgattgttactat

cccctttagctctttacgctttgttaaagtaacgccacccttctcttataaaaagcgccgcgcggtaattgagacccattatgatcaggccac

caaagtcttacttgaatttagccgtcgttggtgggaattcacagaggcggactggaagcgtgagcttgatgctatcgccccgggtctgtac

gactattatcaacagtggggtgaagacgacgccgaagcagcgcttgcactgccgcagtcagtccgcaacttgcctaccggcttgcttgg

ggcccacccaagcgtcgacgaatcgcgtatcggagaggagcaagttgagtattatcgtaacagcgaactgcgtggcggagtgcgccc

tgcgaccaatgcgtacggagggggtagtacgaccgataatcctaatcgtttcatgtactatccctcccaccccgttccaggcacgcagg

ggggggtcgtacttgcagcatacagttggagcgatgatgcagctcgttgggattcttttgacgatgctgagcgctatggctatgcgcttga

aaacttacagtctgtccacggccgtcgtattgaggtgttctatacaggagcgggacagacccaatcatggctgcgcgatccgtacgcatg

cggcgaggcagctgtctatacaccccatcaaatgaccgcgtttcacttagatgtggtgcgtcccgaggggcccgtctattttgcgggtga

gcacgtttcattaaaacatgcctggatcgagggggcggtggaaacagccgttcgtgcggcgatcgctgtaaatgaagcccccgtaggt

gacactggggtcacagccgctgctgggcgccgtggggcggccgccgctactgagcctatgcgcgaggaggcgttaacttcttaa

SEQ ID NO: 3 StGLOD-f1

atgaatgaaatgacctacgagcaattggcccgcgaattgttattggttggtccggccccgacgaacgaggatcttaaattacgctatttgg

atgtgctgattgataacgggctgaacccgccgggtccgcctaagcgtatcttgatcgtcggggctggtattgcaggccttgttgccggag

atttgttgacccgcgctgggcatgatgttactattcttgaagctaatgccaaccgtgtgggagggcgtatcaagacgttccatgcaaaaaa
aggagagccatctccattcgctgatccagcgcagtacgcggaagctggggcgatgcgtcttccttcctttcacccacttaccttggcactt
atcgacaagctgggcttaaaacgccgcctgttttttaatgtagatattgacccacaaactggaaatcaagatgctccagtcccaccggtctt
ctataagtcttttaaggacggcaagacgtggacaaatggagccccgagccctgagtttaaagagcccgataaacgtaatcacacctgga
tccgtacgaatcgcgaacaagtacgccgcgcccaatacgccacagacccgagctccatcaatgaaggcttccatctgacgggatgtga
gacccgccttactgtctccgacatggttaaccaagcgcttgaaccggtacgcgactattattcagtcaagcaggac

SEQ ID NO: 4 StGLOD-f2

tcagtcaagcaggacgatggaactcgtgtgaataagcctttcaaagaatggcttgcagggtgggccgacgtcgtgcgtgatttcgacgg
ttattctatgggccgcttcttacgtgaatatgccgagttttcggatgaggcagtggaagcaattggcacaattgaaaacatgacctcgcgcc
tgcaccttgcctttttcattcgtttttgggccgttccgatatcgatccgcgcgccacttactgggagatcgagggggggtcacgcatgcttc
ctgaaacactggcaaaggacctgcgcgatcagatcgttatgggtcagcgcatggtacgcttagagtactatgacccgggccgcgatgg
ccatcatggtgagcttactgggcctgggggtccggccgtggccattcagacggttccagagggagagccttacgccgcaactcaaacg
tggacaggagacctggcgattgttactatcccctttagctctttacgctttgttaaagtaacgccacccttctcttataaaaagcgccgcgcg
gtaattgagacccattatgatcaggccaccaaagtcttacttgaatttagccgtcgttggtgggaattcacagaggcggactggaagcgtg
agcttgatgctatcgccccgggtctgtacgactattatcaacagtggggtgaagac

SEQ ID NO: 5 StGLOD-f3

cagtggggtgaagacgacgccgaagcagcgcttgcactgccgcagtcagtccgcaacttgcctaccggcttgcttggggcccaccca
agcgtcgacgaatcgcgtatcggagaggagcaagttgagtattatcgtaacagcgaactgcgtggcggagtgcgccctgcgaccaat
gcgtacggagggggtagtacgaccgataatcctaatcgtttcatgtactatccctcccaccccgttccaggcacgcaggggggggtcgt
acttgcagcatacagttggagcgatgatgcagctcgttgggattcttttgacgatgctgagcgctatggctatgcgcttgaaaacttacagt
ctgtccacggccgtcgtattgaggtgttctatacaggagcgggacagacccaatcatggctgcgcgatccgtacgcatgcggcgaggc
agctgtctatacaccccatcaaatgaccgcgtttcacttagatgtggtgcgtcccgaggggcccgtctattttgcgggtgagcacgtttcat
taaaacatgcctggatcgagggggcggtggaaacagccgttcgtgcggcgatcgctgtaaatgaagcccccgtaggtgacactgggg
tcacagccgctgctgggcgccgtggggcggccgccgctactgagcctatgcgcgaggaggcgttaacttcttaa

SEQ ID NO: 6 Primer sequence ggtcatttca ttcatgaatt ctgtttcctg tgtgaaattg
SEQ ID NO: 7 Primer sequence gcgttaactt cttaagcttg gctgttttgg cggatgag
SEQ ID NO: 8 Primer sequence gataagaccg aagcgaccaa tgcgtacgga
SEQ ID NO: 9 Primer sequence cagcttacga taatagtcgt acagacccgg
SEQ ID NO: 10 Primer sequence gcagagccac ctgcgaccaa tgcgtacgga
SEQ ID NO: 11 Primer sequence cgcatcttga taatagtcgt acagacccgg
SEQ ID NO: 12 Amino acid sequence of StGLODΔ49Ai

MNEMTYEQLARELLLVGPAPTNEDLKLRYLDVLIDNGLNPPGPPKRILIVGAGIAGLV
AGDLLTRAGHDVTILEANANRVGGRIKTFHAKKGEPSPFADPAQYAEAGAMRLPSFH
PLTLALIDKLGLKRRLFFNVDIDPQTGNQDAPVPPVFYKSFKDGKTWTNGAPSPEFKE
PDKRNHTWIRTNREQVRRAQYATDPSSINEGFHLTGCETRLTVSDMVNQALEPVRDY
YSVKQDDGTRVNKPFKEWLAGWADVVRDFDGYSMGRFLREYAEFSDEAVEAIGTIE
NMTSRLHLAFFHSFLGRSDIDPRATYWEIEGGSRMLPETLAKDLRDQIVMGQRMVRL
EYYDPGRDGHHGELTGPGGPAVAIQTVPEGEPYAATQTWTGDLAIVTIPFSSLRFVKV
TPPFSYKKRRAVIETHYDQATKVLLEFSRRWWEFTEADWKRELDAIAPGLYDYYRKL
DKTEATNAYGGGSTTDNPNRFMYYPSHPVPGTQGGVVLAAYSWSDDAARWDSFDD
AERYGYALENLQSVHGRRIEVFYTGAGQTQSWLRDPYACGEAAVYTPHQMTAFHLD
VVRPEGPVYFAGEHVSLKHAWIEGAVETAVRAAIAVNEAPVGDTGVTAAAGRRGAA
AATEPMREEALTS

SEQ ID NO: 13 Amino acid sequence of StGLODΔ49C

MNEMTYEQLARELLLVGPAPTNEDLKLRYLDVLIDNGLNPPGPPKRILIVGAGIAGLV
AGDLLTRAGHDVTILEANANRVGGRIKTFHAKKGEPSPFADPAQYAEAGAMRLPSFH
PLTLALIDKLGLKRRLFFNVDIDPQTGNQDAPVPPVFYKSFKDGKTWTNGAPSPEFKE
PDKRNHTWIRTNREQVRRAQYATDPSSINEGFHLTGCETRLTVSDMVNQALEPVRDY
YSVKQDDGTRVNKPFKEWLAGWADVVRDFDGYSMGRFLREYAEFSDEAVEAIGTIE
NMTSRLHLAFFHSFLGRSDIDPRATYWEIEGGSRMLPETLAKDLRDQIVMGQRMVRL
EYYDPGRDGHHGELTGPGGPAVAIQTVPEGEPYAATQTWTGDLAIVTIPFSSLRFVKV
TPPFSYKKRRAVIETHYDQATKVLLEFSRRWWEFTEADWKRELDAIAPGLYDYYQD
AAEPPATNAYGGGSTTDNPNRFMYYPSHPVPGTQGGVVLAAYSWSDDAARWDSFD
DAERYGYALENLQSVHGRRIEVFYTGAGQTQSWLRDPYACGEAAVYTPHQMTAFHL
DVVRPEGPVYFAGEHVSLKHAWIEGAVETAVRAAIAVNEAPVGDTGVTAAAGRRGA
AAATEPMREEALTS

SEQ ID NO: 14 Primer sequence cgtcttgata cgccctccca cacggttggc
SEQ ID NO: 15 Primer sequence cgtatcaaga cgtaccatgc aaaaaaagga
SEQ ID NO: 16 Primer sequence ctgcgctgga tcagcgaatg gagatggctc
SEQ ID NO: 17 Primer sequence gatccagcgc agttcgcgga agctggggcg
SEQ ID NO: 18 Primer sequence gatccagcgc agttagcgga agctggggcg
SEQ ID NO: 19 Primer sequence gatccagcgc aggtcgcgga agctggggcg
SEQ ID NO: 20 Primer sequence gatccagcgc agatcgcgga agctggggcg
SEQ ID NO: 21 Primer sequence gatccagcgc agatggcgga agctggggcg
SEQ ID NO: 22 Primer sequence aaacaggcgg cgttttaagc ccagcttgtc

SEQ ID NO: 23 Primer sequence cgccgcctgt tttataatgt agatattgac
SEQ ID NO: 24 Primer sequence cgccgcctgt ttttaaatgt agatattgac
SEQ ID NO: 25 Primer sequence aaaggcaagg tgcaggcgcg aggtcatgtt
SEQ ID NO: 26 Primer sequence caccttgcct ttttacattc gtttttgggc
SEQ ID NO: 27 Primer sequence caccttgcct ttgttcattc gtttttgggc
SEQ ID NO: 28 Primer sequence caccttgcct ttattcattc gtttttgggc
SEQ ID NO: 29 Primer sequence caccttgcct ttatgcattc gtttttgggc
SEQ ID NO: 30 Primer sequence aggctctccc tctggaaccg tctgaatggc
SEQ ID NO: 31 Primer sequence gagggagagc ctttcgccgc aactcaaacg
SEQ ID NO: 32 Primer sequence gagggagagc ctttagccge aactcaaacg
SEQ ID NO: 33 Primer sequence gagggagagc ctatcgccgc aactcaaacg
SEQ ID NO: 34 Primer sequence gagggagagc ctatggccgc aactcaaacg
SEQ ID NO: 35 Primer sequence ggggatagta acaatcgcca ggtctcctgt
SEQ ID NO: 36 Primer sequence gttactatcc ccttaagctc tttacgcttt
SEQ ID NO: 37 Primer sequence gttactatcc ccgttagctc tttacgcttt
SEQ ID NO: 38 Primer sequence gttactatcc ccattagctc tttacgcttt
SEQ ID NO: 39 Primer sequence gttactatcc ccatgagctc tttacgcttt
SEQ ID NO: 40 Primer sequence ttcaagtaag actttggtgg cctgatcata
SEQ ID NO: 41 Primer sequence gtcttacttg aatatagccg tcgttggtgg
SEQ ID NO: 42 Primer sequence gtcttacttg aaatgagccg tcgttggtgg
SEQ ID NO: 43 Primer sequence cgcattggtc gcaggtggct ctgccgcatc
SEQ ID NO: 44 Primer sequence gcgaccaatg cgttcggagg gggtagtacg
SEQ ID NO: 45 Primer sequence gcgctcagca tcgtcaaaag aatcccaacg
SEQ ID NO: 46 Primer sequence gatgctgagc getttggeta tgcgcttgaa
SEQ ID NO: 47 Primer sequence gatgctgagc gettaggeta tgcgcttgaa
SEQ ID NO: 48 Primer sequence gatgctgagc gcgttggcta tgcgcttgaa
SEQ ID NO: 49 Primer sequence gatgctgagc gcatgggcta tgcgcttgaa
SEQ ID NO: 50 Primer sequence gccatagcgc tcagcatcgt caaaagaatc
SEQ ID NO: 51 Primer sequence gagcgctatg gcattgcgct tgaaaactta
SEQ ID NO: 52 Primer sequence gagcgctatg gcatggcgct tgaaaactta
SEQ ID NO: 53 Primer sequence gaacacctca atacgacggc cgtggacaga
SEQ ID NO: 54 Primer sequence attgaggtgt tcttaacagg agcgggacag
SEQ ID NO: 55 Primer sequence attgaggtgt tcatgacagg agcgggacag
SEQ ID NO: 56 Primer sequence cgcggtcatt tgatggggtg tatagacagc
SEQ ID NO: 57 Primer sequence caaatgaccg cgttacactt agatgtggtg
SEQ ID NO: 58 Amino acid sequence of putative glutamate oxidase from *Streptomyces* sp. MOE7

MDDKTYQQLARELLLVGPEPANEDLKLRYLDVLIDNGLEPPVDRKRILIVGAGIAGLV
AGHLLTRAGHDVTILEANANRVGGRIKTFHAKKGEPAPFTDPAQYAEAGAMRLPSFH
PLTLALIDKLGLKRRLFFNVDIDPKTGNQGAALPPVVYKSFKDGKTWTYGKPSPEFRE
PDKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFFHSFLGRSDIDPSATYWEIEGGSRQLPEALAKDLRDQIVMGQRMVRLE
YYDPGRDGHHGGLAGPSGPAVAIETVPENEPSAEPQTWTADLAIVTVPFSSLRFVAVT

PPFSYKKRRAVIETHYDQATKVLLEFSRRWWEFTEEDWKRELDAIAPGLYEYYQRW
GEDDAEAALTVPESVRNLPTGLLGAHPSVDEQLIDDEQVEYLRNSTLRGGVRPATQV
HGGGSTTDNPNRFMYYPSHAVPGSKGGVVLAAYSWSDDAARWDSFDDAERYGYAL
ENLQSVHGRRIEVFYTGAGQTQSWLRDPYACGEAAVYTPHQMTSFHLDVVRPEGPV
YFAGEHVSLKHAWIEGAVETAVRAAIAVNEAPVPYDTAAARAEAPRERAGTASATR
TREKAVTS

SEQ ID NO: 59 Amino acid sequence of deletion type M7GLOD (M7GLODΔ49C)

MDDKTYQQLARELLLVGPEPANEDLKLRYLDVLIDNGLEPPVDRKRILIVGAGIAGLV
AGHLLTRAGHDVTILEANANRVGGRIKTFHAKKGEPAPFTDPAQYAEAGAMRLPSFH
PLTLALIDKLGLKRRLFFNVDIDPKTGNQGAALPPVVYKSFKDGKTWTYGKPSPEFRE
PDKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFFHSFLGRSDIDPSATYWEIEGGSRQLPEALAKDLRDQIVMGQRMVRLE
YYDPGRDGHHGGLAGPSGPAVAIETVPENEPSAEPQTWTADLAIVTVPFSSLRFVAVT
PPFSYKKRRAVIETHYDQATKVLLEFSRRWWEFTEEDWKRELDAIAPGLYEYYQDAA
EPPATQVHGGGSTTDNPNRFMYYPSHAVPGSKGGVVLAAYSWSDDAARWDSFDDA
ERYGYALENLQSVHGRRIEVFYTGAGQTQSWLRDPYACGEAAVYTPHQMTSFHLDV
VRPEGPVYFAGEHVSLKHAWIEGAVETAVRAAIAVNEAPVPYDTAAARAEAPRERA
GTASATRTREKAVTS

SEQ ID NO: 60 Amino acid sequence of M7GLODΔ49C having 8 types of mutations for thermal stability introduced therein (M7GLODΔ49C-T8)

MDDKTYQQLARELLLVGPEPANEDLKLRYLDVLIDNGLEPPVDRKRILIVGAGIAGLV
AGHLLTRAGHDVTILEANANRVGGRIKTYHAKKGEPAPFTDPAQIAEAGAMRLPSFH
PLTLALIDKLGLKRRLFYNVDIDPKTGNQGAALPPVVYKSFKDGKTWTYGKPSPEFRE
PDKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFMHSFLGRSDIDPSATYWEIEGGSRQLPEALAKDLRDQIVMGQRMVRLE

YYDPGRDGHHGGLAGPSGPAVAIETVPENEPSAEPQTWTADLAIVTVPLSSLRFVAVT
PPFSYKKRRAVIETHYDQATKVLLEYSRRWWEFTEEDWKRELDAIAPGLYEYYQDA
AEPPATQVHGGGSTTDNPNRFMYYPSHAVPGSKGGVVLAAYSWSDDAARWDSFDD
AERFGYALENLQSVHGRRIEVFLTGAGQTQSWLRDPYACGEAAVYTPHQMTSFHLD
VVRPEGPVYFAGEHVSLKHAWIEGAVETAVRAAIAVNEAPVPYDTAAARAEAPRER
AGTASATRTREKAVTS

SEQ ID NO: 61 Nucleotide sequence of M7GLODΔ49C-T8

atggacgacaagacctatcagcagttagcacgcgaactgctgcttgtagggcccgagccagcgaatgaagatcttaaattgcgctattta
gacgtacttattgataacgggctggagcccccagtcgatcgtaaacgtatcttaattgtgggggcagggatcgccggcctggtcgcggg
acatttgttgacacgcgcgggacacgatgtcaccatcctggaggcgaatgcaaatcgcgttggaggtcgcattaagacatatcatgcaa
agaaaggcgaaccggccccccttcaccgacccggctcaaattgcagaggcgggagctatgcgtttgccttcattccatcccttaacgttag
cgctgattgataaattagggttgaagcgtcgcttgttctataatgttgacattgaccctaagacgggcaatcaaggtgcagcattgccgcct
gtcgtttacaagagctttaaggacggtaaaacttggacgtatggtaaaccttctccggaattccgtgaaccagacaaacgtaaccacactt
ggattcgtaccaatcgcactcaagtacgtcgcgcccagtatgtgaaggacccgagtgcgattaacgaagggttccatttaaccgggtgc
gagtcgcgtttgacagtttctgatatggtgaaccaagctttagaaccggttcgtgactactattctgtattgcagagcgacgggcgccgcgt
aaataagccatttaaggagtggttagacggctgggccggtgtaatccgcgatttcgacggattctcaatgggacgttttctgcgcgagtac
gctgggttttcggacgaggcggtagaagcaatcggtaccatcgagaatatgacaagtcgtttgcatttggcatttatgcacagcttcttagg
tcgcagtgacattgaccccagcgcgacatattgggagattgaaggcggtagccgtcagctgcctgaggctctggcaaaagacctgcgt
gatcagattgtaatgggccaacgcatggtgcgtttagagtactacgacccaggacgtgatggacaccatggaggtctggcaggaccct
ctggtcctgcggttgcaatcgaaactgtacccgagaacgagccaagtgcagagccgcagacgtggactgcggacctggcgattgtca
ctgtaccactttcgtctcttcgctttgttgcggtgactcccccattcagttataaaaaacgtcgtgcagttatcgaaactcactatgatcaggc
aacaaaagtgctgttagagtattctcgtcgctggtgggagtttacagaagaggactggaagcgtgagttggacgcgattgcccccggcc
tgtatgaatactaccaggatgctgcagaaccgccggcaactcaagttcatggaggaggttcaacaactgacaatccaaaccgttttatgta
ttatccgagtcacgcggtgcctggttctaagggtggtgtggttttggccgcgtatagttggagtgacgatgcagcgcgttgggattcctttg
atgacgccgaacgttttggctacgccctggaaaaccttcaatcggtccatggccgccgcatcgaggtctttttaactggagctggacaaa
ctcagtcatggttgcgcgacccctacgcctgcggtgaggccgcagtttacacaccacatcagatgacatcttttcacttagacgtagtgcg
cccggaggggccagtatactttgcgggagagcatgtctctcttaagcatgcgtggatcgagggggccgttgaaaccgcggtccgtgct
gcgatcgcggttaacgaagcccccgtaccatacgacacagctgctgctcgtgcggaggcccctcgcgaacgtgccggtacagcatca
gccactcgcacgcgcgagaaggccgtgacttcctaa

SEQ ID NO: 62 M7GLODΔ49C-T8-f1

ggagatatacatatggacgacaagacctatcagcagttagcacgcgaactgctgcttgtagggcccgagccagcgaatgaagatcttaa
attgcgctatttagacgtacttattgataacgggctggagcccccagtcgatcgtaaacgtatcttaattgtgggggcagggatcgccggc
ctggtcgcgggacatttgttgacacgcgcgggacacgatgtcaccatcctggaggcgaatgcaaatcgcgttggaggtcgcattaaga
catatcatgcaaagaaaggcgaaccggccccccttcaccgacccggctcaaattgcagaggcgggagctatgcgtttgccttcattccat
cccttaacgttagcgctgattgataaattagggttgaagcgtcgcttgttctataatgttgacattgaccctaagacgggcaatcaaggtgc
agcattgccgcctgtcgtttacaagagctttaaggacggtaaaacttggacgtatggtaaaccttctccggaattccgtgaaccagacaaa
cgtaaccacacttggattcgtaccaatcgcactcaagtacgtcgcgcccagtatgtgaaggacccgagtgcgattaacgaagggttccat
ttaaccgggtgcgagtcgcgtttga

SEQ ID NO: 63 M7GLODΔ49C-T8-f2

gcgagtcgcgtttgacagtttctgatatggtgaaccaagctttagaaccggttcgtgactactattctgtattgcagagcgacgggcgccg
cgtaaataagccatttaaggagtggttagacggctgggccggtgtaatccgcgatttcgacggattctcaatgggacgttttctgcgcgag
tacgctgggttttcggacgaggcggtagaagcaatcggtaccatcgagaatatgacaagtcgtttgcatttggcatttatgcacagcttctt
aggtcgcagtgacattgaccccagcgcgacatattgggagattgaaggcggtagccgtcagctgcctgaggctctggcaaaagacctg
cgtgatcagattgtaatgggccaacgcatggtgcgtttagagtactacgacccaggacgtgatggacaccatggaggtctggcaggac
cctctggtcctgcggttgcaatcgaaactgtacccgagaacgagccaagtgcagagccgcagacgtggactgcggacctggcgattgt
cactgtaccactttcgtctcttcgctttgttgcggtgactcccccattcagttataaaaaacgtcgtgcagttatcgaaactcactatgatcag
gcaacaaaagtgctgttagagtattctcgtcgctggtg

SEQ ID NO: 64 M7GLODΔ49C-T8-f3

ttctcgtcgctggtgggagtttacagaagaggactggaagcgtgagttggacgcgattgcccccggcctgtatgaatactaccaggatg
ctgcagaaccgccggcaactcaagttcatggaggaggttcaacaactgacaatccaaaccgttttatgtattatccgagtcacgcggtgc
ctggttctaagggtggtgtggttttggccgcgtatagttggagtgacgatgcagcgcgttgggattcctttgatgacgccgaacgttttggc
tacgccctggaaaaccttcaatcggtccatggccgccgcatcgaggtctttttaactggagctggacaaactcagtcatggttgcgcgac
ccctacgcctgcggtgaggccgcagtttacacaccacatcagatgacatcttttcacttagacgtagtgcgcccggagggggccagtata
ctttgcgggagagcatgtctctcttaagcatgcgtggatcgagggggccgttgaaaccgcggtccgtgctgcgatcgcggttaacgaag
cccccgtaccatacgacacagctgctgctcgtgcggaggcccctcgcgaacgtgccggtacagcatcagccactcgcacgcgcgag
aaggccgtgacttcctaacaaagcccgaaa

SEQ ID NO: 65 Primer sequence catatgtata tctccttctt aaag
SEQ ID NO: 66 Primer sequence taacaaagcc cgaaaggaag
SEQ ID NO: 67 Primer sequence ggtggtgtgg ttgcggccgc gtatagttgg
SEQ ID NO: 68 Primer sequence ggtggtgtgg ttggtgccgc gtatagttgg
SEQ ID NO: 69 Primer sequence ggtggtgtgg ttgtggccgc gtatagttgg
SEQ ID NO: 70 Primer sequence ggtggtgtgg ttatggccgc gtatagttgg
SEQ ID NO: 71 Primer sequence ggtggtgtgg ttaacgccgc gtatagttgg

SEQ ID NO: 72 Primer sequence ggtggtgtgg tttgcgccgc gtatagttgg
SEQ ID NO: 73 Primer sequence ggtggtgtgg tttcggccgc gtatagttgg
SEQ ID NO: 74 Primer sequence ggtggtgtgg ttacggccgc gtatagttgg
SEQ ID NO: 75 Primer sequence gcaacaaaag tgcagttaga gtattctcgt
SEQ ID NO: 76 Primer sequence aaccacacca cccttagaac
SEQ ID NO: 77 Primer sequence cacttttgtt gcctgatc
SEQ ID NO: 78 Primer sequence gaggcgggag ctgcgcgttt gccttcattc
SEQ ID NO: 79 Primer sequence gaggcgggag cttgccgttt gccttcattc
SEQ ID NO: 80 Primer sequence gaggcgggag ctgtgcgttt gccttcattc
SEQ ID NO: 81 Primer sequence gaggcgggag cttttcgttt gccttcattc
SEQ ID NO: 82 Primer sequence gaggcgggag cttatcgttt gccttcattc
SEQ ID NO: 83 Primer sequence gaggcgggag cttggcgttt gccttcattc
SEQ ID NO: 84 Primer sequence gaggcgggag cttcgcgttt gccttcattc
SEQ ID NO: 85 Primer sequence gaggcgggag ctacgcgttt gccttcattc
SEQ ID NO: 86 Primer sequence gaggcgggag ctcagcgttt gccttcattc
SEQ ID NO: 87 Primer sequence gaggcgggag ctcatcgttt gccttcattc
SEQ ID NO: 88 Primer sequence agctcccgcc tctgcaattt gagccgg
SEQ ID NO: 89 Primer sequence atcgaggtct tttatactgg agctggacaa
SEQ ID NO: 90 Primer sequence aaagacctcg atgcggcggc catggaccga
SEQ ID NO: 91 Amino acid sequence of M7GLODΔ49C (M7GLODΔ49C-T7)

MDDKTYQQLARELLLVGPEPANEDLKLRYLDVLIDNGLEPPVDRKRILIVGAGIAGLV
AGHLLTRAGHDVTILEANANRVGGRIKTYHAKKGEPAPFTDPAQIAEAGAMRLPSFH
PLTLALIDKLGLKRRLFYNVDIDPKTGNQGAALPPVVYKSFKDGKTWTYGKPSPEFRE
PDKRNHTWIRTNRTQVRRAQYVKDPSAINEGFHLTGCESRLTVSDMVNQALEPVRDY
YSVLQSDGRRVNKPFKEWLDGWAGVIRDFDGFSMGRFLREYAGFSDEAVEAIGTIEN
MTSRLHLAFMHSFLGRSDIDPSATYWEIEGGSRQLPEALAKDLRDQIVMGQRMVRLE
YYDPGRDGHHGGLAGPSGPAVAIETVPENEPSAEPQTWTADLAIVTVPLSSLRFVAVT
PPFSYKKRRAVIETHYDQATKVLLEYSRRWWEFTEEDWKRELDAIAPGLYEYYQDA
AEPPATQVHGGGSTTDNPNRFMYYPSHAVPGSKGGVVLAAYSWSDDAARWDSFDD
AERFGYALENLQSVHGRRIEVFYTGAGQTQSWLRDPYACGEAAVYTPHQMTSFHLD
VVRPEGPVYFAGEHVSLKHAWIEGAVETAVRAAIAVNEAPVPYDTAAARAEAPRER
AGTASATRTREKAVTS

SEQ ID NO: 92 Primer sequence attcgtacca atggcactca agtacgtcgc
SEQ ID NO: 93 Primer sequence attcgtacca atgcgactca agtacgtcgc
SEQ ID NO: 94 Primer sequence attcgtacca attgcactca agtacgtcgc
SEQ ID NO: 95 Primer sequence attcgtacca atctgactca agtacgtcgc
SEQ ID NO: 96 Primer sequence attcgtacca atatgactca agtacgtcgc
SEQ ID NO: 97 Primer sequence attcgtacca attttactca agtacgtcgc
SEQ ID NO: 98 Primer sequence attcgtacca attatactca agtacgtcgc
SEQ ID NO: 99 Primer sequence attcgtacca atagcactca agtacgtcgc
SEQ ID NO: 100 Primer sequence attcgtacca ataccactca agtacgtcgc
SEQ ID NO: 101 Primer sequence attcgtacca ataacactca agtacgtcgc
SEQ ID NO: 102 Primer sequence attcgtacca atcagactca agtacgtcgc
SEQ ID NO: 103 Primer sequence attcgtacca atcatactca agtacgtcgc
SEQ ID NO: 104 Primer sequence attcgtacca atgatactca agtacgtcgc
SEQ ID NO: 105 Primer sequence attcgtacca atgaaactca agtacgtcgc

SEQ ID NO: 106 Primer sequence attggtacga atccaagtgt ggttacg

## Claims

1. A flavin adenine dinucleotide (FAD) type glutamate dehydrogenase, wherein the amino acid at the position corresponding to the 109th and/or 545th position of SEQ ID NO: 58 is substituted and the ratio (OD/DH) of oxidase activity (OD) to dehydrogenase activity (DH) of the polypeptide after the amino acid substitution is reduced compared to the polypeptide prior to the amino acid substitution.

2. The FAD type glutamate dehydrogenase of claim 1, wherein

   the amino acid at the position corresponding to the 109th position of SEQ ID NO: 58 is substituted with an amino acid residue selected from the group consisting of serine, tryptophan, glutamine, alanine, aspartic acid, glutamic acid, phenylalanine, arginine, histidine, lysine, threonine, asparagine, cysteine, glycine, proline, valine, leucine, isoleucine, and tyrosine, and/or
   the amino acid at the position corresponding to the 545th position of SEQ ID NO: 58 is substituted with an amino acid residue selected from the group consisting of alanine, aspartic acid, glutamic acid, phenylalanine, arginine, histidine, lysine, serine, threonine, asparagine, glutamine, cysteine, glycine, proline, valine, isoleucine, methionine, tyrosine and tryptophan.

3. The FAD type glutamate dehydrogenase of claim 1 or 2, wherein the amino acid at the position corresponding to the 425th position of SEQ ID NO: 58 is further substituted with an amino acid residue selected from the group consisting of glutamine, alanine, aspartic acid, glutamic acid, phenylalanine, arginine, histidine, lysine, serine, threonine, asparagine, cysteine, glycine, proline, valine, isoleucine, methionine, tyrosine and tryptophan.

4. The FAD type glutamate dehydrogenase of any one of claims 1 to 3, wherein the amino acid sequence of the region corresponding to the 459th to 507th positions of SEQ ID NO: 58 is further deleted and the FAD type glutamate dehydrogenase comprises glutamate dehydrogenase activity.

5. The FAD type glutamate dehydrogenase of any one of claims 1 to 4, wherein the amino acid sequence of the polypeptide prior to the amino acid substitution comprises an amino acid sequence identity of 90% or more with the amino acid sequence of SEQ ID NO: 58, SEQ ID NO: 59, SEQ ID NO: 12 or SEQ ID NO: 13.

6. The FAD type glutamate dehydrogenase of any one of claims 1 to 5, wherein the FAD type glutamate dehydrogenase further comprises an amino acid substitution compared to the amino acid sequence of SEQ ID NO: 58, at one or more positions selected from the group consisting of

   the position corresponding to the 186th position of SEQ ID NO: 58,
   the position corresponding to the 87th position of SEQ ID NO: 58,
   the position corresponding to the 103rd position of SEQ ID NO: 58,
   the position corresponding to the 133rd position of SEQ ID NO: 58,
   the position corresponding to the 297th position of SEQ ID NO: 58,
   the position corresponding to the 376th position of SEQ ID NO: 58,
   the position corresponding to the 393rd position of SEQ ID NO: 58,
   the position corresponding to the 428th position of SEQ ID NO: 58,
   the position corresponding to the 516th position of SEQ ID NO: 58,
   the position corresponding to the 566th position of SEQ ID NO: 58,
   the position corresponding to the 568th position of SEQ ID NO: 58,
   the position corresponding to the 585th position of SEQ ID NO: 58, and
   the position corresponding to the 615th position of SEQ ID NO: 58.

7. The FAD type glutamate dehydrogenase of claim 6, wherein

   the amino acid substitution at the position corresponding to the 186th position of SEQ ID NO: 58 is substitution with an amino acid selected from the group consisting of glycine, alanine, cysteine, leucine, methionine, phenylalanine, tyrosine, serine, threonine, asparagine, glutamine, histidine, aspartic acid and glutamic acid,
   the amino acid substitution at the position corresponding to the 87th position of SEQ ID NO: 58 is substitution with

tyrosine,
the amino acid substitution at the position corresponding to the 103rd position of SEQ ID NO: 58 is substitution with an amino acid selected from the group consisting of phenylalanine, leucine, valine, isoleucine and methionine,
the amino acid substitution at the position corresponding to the 133rd position of SEQ ID NO: 58 is substitution with an amino acid selected from the group consisting of leucine and tyrosine,
the amino acid substitution at the position corresponding to the 297th position of SEQ ID NO: 58 is substitution with an amino acid selected from the group consisting of leucine, valine, isoleucine and methionine,
the amino acid substitution at the position corresponding to the 376th position of SEQ ID NO: 58 is substitution with an amino acid selected from the group consisting of phenylalanine, leucine, isoleucine and methionine,
the amino acid substitution at the position corresponding to the 393rd position of SEQ ID NO: 58 is substitution with an amino acid selected from the group consisting of leucine, valine, isoleucine and methionine,
the amino acid substitution at the position corresponding to the 428th position of SEQ ID NO: 58 is substitution with an amino acid selected from the group consisting of tyrosine and methionine,
the amino acid substitution at the position corresponding to the 516th position of SEQ ID NO: 58 is substitution with phenylalanine,
the amino acid substitution at the position corresponding to the 566th position of SEQ ID NO: 58 is substitution with an amino acid selected from the group consisting of phenylalanine, leucine, valine and methionine,
the amino acid substitution at the position corresponding to the 568th position of SEQ ID NO: 58 is substitution with an amino acid selected from the group consisting of isoleucine and methionine,
the amino acid substitution at the position corresponding to the 585th position of SEQ ID NO: 58 is substitution with an amino acid selected from the group consisting of leucine and methionine, or
the amino acid substitution at the position corresponding to the 615th position of SEQ ID NO: 58 is substitution with leucine.

**8.** The FAD type glutamate dehydrogenase of claim 7, wherein

the FAD type glutamate dehydrogenase comprises an amino acid sequence identity of 90% or more with the amino acid sequence of SEQ ID NO: 60,
the amino acid sequence of the region corresponding to the 459th to 507th positions of SEQ ID NO: 58 is deleted, and
the FAD type glutamate dehydrogenase comprises an amino acid substitution compared to the amino acid sequence of SEQ ID NO: 58, at one or more positions selected from the group consisting of
the position corresponding to the 186th position of SEQ ID NO: 58,
the position corresponding to the 87th position of SEQ ID NO: 58,
the position corresponding to the 103rd position of SEQ ID NO: 58,
the position corresponding to the 133rd position of SEQ ID NO: 58,
the position corresponding to the 297th position of SEQ ID NO: 58,
the position corresponding to the 376th position of SEQ ID NO: 58,
the position corresponding to the 393rd position of SEQ ID NO: 58,
the position corresponding to the 428th position of SEQ ID NO: 58,
the position corresponding to the 516th position of SEQ ID NO: 58,
the position corresponding to the 566th position of SEQ ID NO: 58,
the position corresponding to the 568th position of SEQ ID NO: 58,
the position corresponding to the 585th position of SEQ ID NO: 58, and
the position corresponding to the 615th position of SEQ ID NO: 58,
wherein the amino acid substitution is any of the amino acid substitutions described in claim 7, and
wherein the FAD type glutamate dehydrogenase comprises glutamate dehydrogenase activity.

**9.** The FAD type glutamate dehydrogenase of any one of claims 1 to 8, wherein the amino acid sequence of the region corresponding to the 671st to 690th positions of SEQ ID NO: 58 is further deleted.

**10.** A composition, reagent, electrode, sensor or kit comprising the glutamate dehydrogenase of any one of claims 1 to 9.

**11.** A polynucleotide encoding the glutamate dehydrogenase of any one of claims 1 to 9.

**12.** A vector comprising the polynucleotide of claim 11.

**13.** A host cell comprising the vector of claim 12.

**14.** A method for producing a glutamate dehydrogenase, comprising the steps of:

culturing the host cell of claim 13 to produce the glutamate dehydrogenase of any one of claims 1 to 9, and obtaining the produced glutamate dehydrogenase.

**15.** A method for bringing the glutamate dehydrogenase of any one of claims 1 to 9 or the composition, reagent, electrode, sensor or kit of claim 10 into contact with a sample containing glutamic acid to oxidize the glutamic acid contained in the sample.

**16.** The method of claim 15, comprising detecting glutamic acid.

**17.** An FAD type glutamate dehydrogenase, wherein

the amino acid at the position corresponding to the 109th, 545th and/or 425th position of SEQ ID NO: 58 is substituted and the ratio (OD/DH) of oxidase activity (OD) to dehydrogenase activity (DH) of the polypeptide after the amino acid substitution is reduced compared to the polypeptide prior to the amino acid substitution,
wherein the FAD type glutamate dehydrogenase further comprises a thermal stability improving mutation, whereby the thermal stability of the polypeptide after the amino acid substitution is improved compared to the polypeptide before the substitution, and
the FAD type glutamate dehydrogenase meets a condition selected from the group consisting of the following:

(i) when the amino acid sequence of the glutamate dehydrogenase is aligned with the amino acid sequence of SEQ ID NO: 58, the glutamate dehydrogenase comprises a substitution of the amino acid at the position corresponding to a position selected from the group consisting of the 186th, 87th, 103rd, 133rd, 297th, 376th, 393rd, 428th, 516th, 566th, 568th, 585th, and 615th positions of SEQ ID NO: 58,
(ii) the glutamate dehydrogenase of said (i), wherein the glutamate dehydrogenase consists of an amino acid sequence comprising a substitution, deletion or addition of one or several amino acids at a position other than the positions corresponding to the 186th, 87th, 103rd, 133rd, 297th, 376th, 393rd, 428th, 516th, 566th, 568th, 585th, and 615th positions of SEQ ID NO: 58,
(iii) with regard to the glutamate dehydrogenase of said (i) or (ii), the full length amino acid sequence thereof comprises an amino acid sequence identity of 70% or more, 80% or more, or 90% or more with the amino acid sequence of SEQ ID NO: 58 or SEQ ID NO: 1, and
(iv) with regard to the glutamate dehydrogenase of said (i) or (ii), the full length amino acid sequence thereof comprises an amino acid sequence identity of 70% or more, 80% or more, or 90% or more with the amino acid sequence of SEQ ID NO: 58 or SEQ ID NO: 1, the amino acid at the position corresponding to the 291st position of SEQ ID NO: 58 in the glutamate dehydrogenase is arginine, and the amino acid sequence of the positions corresponding to the 51st to 56th positions of SEQ ID NO: 58 is Gly-Xaa-Gly-Xaa-Xaa-Gly (wherein Xaa represents any amino acid).

**18.** The FAD type glutamate dehydrogenase of claim 17, wherein

the amino acid at the position corresponding to the 109th position of SEQ ID NO: 58 is substituted with an amino acid residue selected from the group consisting of serine, tryptophan, glutamine, alanine, aspartic acid, glutamic acid, phenylalanine, arginine, histidine, lysine, threonine, asparagine, cysteine, glycine, proline, valine, leucine, isoleucine, and tyrosine, and/or
the amino acid at the position corresponding to the 545th position of SEQ ID NO: 58 is substituted with an amino acid residue selected from the group consisting of alanine, aspartic acid, glutamic acid, phenylalanine, arginine, histidine, lysine, serine, threonine, asparagine, glutamine, cysteine, glycine, proline, valine, isoleucine, methionine, tyrosine and tryptophan.

**19.** The FAD type glutamate dehydrogenase of claim 17 or 18, wherein the amino acid at the position corresponding to the 425th position of SEQ ID NO: 58 is further substituted with an amino acid residue selected from the group consisting of glutamine, alanine, aspartic acid, glutamic acid, phenylalanine, arginine, histidine, lysine, serine, threonine, asparagine, cysteine, glycine, proline, valine, isoleucine, methionine, tyrosine and tryptophan.

**20.** The FAD type glutamate dehydrogenase of any one of claims 17 to 19, wherein

the amino acid substitution at the position corresponding to the 186th position of SEQ ID NO: 58 is substitution with an amino acid selected from the group consisting of glycine, alanine, cysteine, leucine, methionine, phenylalanine, tyrosine, serine, threonine, asparagine, glutamine, histidine, aspartic acid and glutamic acid,
the amino acid substitution at the position corresponding to the 87th position of SEQ ID NO: 58 is substitution with tyrosine,
the amino acid substitution at the position corresponding to the 103rd position of SEQ ID NO: 58 is substitution with an amino acid selected from the group consisting of phenylalanine, leucine, valine, isoleucine and methionine,
the amino acid substitution at the position corresponding to the 133rd position of SEQ ID NO: 58 is substitution with an amino acid selected from the group consisting of leucine and tyrosine,
the amino acid substitution at the position corresponding to the 297th position of SEQ ID NO: 58 is substitution with an amino acid selected from the group consisting of methionine, leucine, valine, and isoleucine,
the amino acid substitution at the position corresponding to the 376th position of SEQ ID NO: 58 is substitution with an amino acid selected from the group consisting of phenylalanine, leucine, isoleucine and methionine,
the amino acid substitution at the position corresponding to the 393rd position of SEQ ID NO: 58 is substitution with an amino acid selected from the group consisting of leucine, valine, isoleucine and methionine,
the amino acid substitution at the position corresponding to the 428th position of SEQ ID NO: 58 is substitution with an amino acid selected from the group consisting of tyrosine and methionine,
the amino acid substitution at the position corresponding to the 516th position of SEQ ID NO: 58 is substitution with phenylalanine,
the amino acid substitution at the position corresponding to the 566th position of SEQ ID NO: 58 is substitution with an amino acid selected from the group consisting of methionine, phenylalanine, leucine, and valine,
the amino acid substitution at the position corresponding to the 568th position of SEQ ID NO: 58 is substitution with an amino acid selected from the group consisting of isoleucine and methionine,
the amino acid substitution at the position corresponding to the 585th position of SEQ ID NO: 58 is substitution with an amino acid selected from the group consisting of leucine and methionine, and
the amino acid substitution at the position corresponding to the 615th position of SEQ ID NO: 58 is substitution with leucine.

**21.** A dry composition comprising an FAD type glutamate dehydrogenase or GLOD and a trisaccharide.

**22.** The dry composition of claim 21, wherein the trisaccharide content per 100 U of the FAD type glutamate dehydrogenase or GLOD is 0.10 mg or more and less than 50 mg.

**23.** The dry composition of claim 21, wherein the amount of the trisaccharide contained in the dry composition is 1% by mass or more and less than 90% by mass.

**24.** The dry composition of any one of claims 21 to 23, wherein the trisaccharide is raffinose.

# Fig. 1-1

```
                            10        20        30        40        50
1_StGLOD     1    MNEMTYEQLARELLLVGPAPTNEDLKLRYLDVLIDNGLNPPGPPKRILIV   50
58_M7GLOD    1    MDDKTYQQLARELLLVGPEPANEDLKLRYLDVLIDNGLEPPVDRKRILIV   50

                            60        70        80        90       100
1_StGLOD     51   GAGIAGLVAGDLLTRAGHDVTILEANANRVGGRIKTFHAKKGEPSPFADP   100
58_M7GLOD    51   GAGIAGLVAGHLLTRAGHDVTILEANANRVGGRIKTFHAKKGEPAPFTDP   100

                           110       120       130       140       150
1_StGLOD     101  AQYAEAGAMRLPSFHPLTLALIDKLGLKRRLFFNVDIDPQTGNQDAPVPP   150
58_M7GLOD    101  AQYAEAGAMRLPSFHPLTLALIDKLGLKRRLFFNVDIDPKTGNQGAALPP   150

                           160       170       180       190       200
1_StGLOD     151  VFYKSFKDGKTWTNGAPSPEFKEPDKRNHTWIRTNREQVRRAQYATDPSS   200
58_M7GLOD    151  VVYKSFKDGKTWTYGKPSPEFREPDKRNHTWIRTNRTQVRRAQYVKDPSA   200

                           210       220       230       240       250
1_StGLOD     201  INEGFHLTGCETRLTVSDMVNQALEPVRDYYSVKQDDGTRVNKPFKEWLA   250
58_M7GLOD    201  INEGFHLTGCESRLTVSDMVNQALEPVRDYYSVLQSDGRRVNKPFKEWLD   250

                           260       270       280       290       300
1_StGLOD     251  GWADVVRDFDGYSMGRFLREYAEFSDEAVEAIGTIENMTSRLHLAFFHSF   300
58_M7GLOD    251  GWAGVIRDFDGFSMGRFLREYAGFSDEAVEAIGTIENMTSRLHLAFFHSF   300

                           310       320       330       340       350
1_StGLOD     301  LGRSDIDPRATYWEIEGGSRMLPETLAKDLRDQIVMGQRMVRLEYYDPGR   350
58_M7GLOD    301  LGRSDIDPSATYWEIEGGSRQLPEALAKDLRDQIVMGQRMVRLEYYDPGR   350
```

Fig. 1-2

```
                   360       370       380       390       400
1_StGLOD    351  DGHHGELTGPGGPAVAIQTVPEGEPYAATQTWTGDLAIVTIPFSSLRFVK  400
58_M7GLOD   351  DGHHGGLAGPSGPAVAIETVPENEPSAEPQTWTADLAIVTVPFSSLRFVA  400

                   410       420       430       440       450
1_StGLOD    401  VTPPFSYKKRRAVIETHYDQATKVLLEFSRRWWEFTEADWKRELDAIAPG  450
58_M7GLOD   401  VTPPFSYKKRRAVIETHYDQATKVLLEFSRRWWEFTEEDWKRELDAIAPG  450

                   460       470       480       490       500
1_StGLOD    451  LYDYYQQWGEDDAEAALALPQSVRNLPTGLLGAHPSVDESRIGEEQVEYY  500
58_M7GLOD   451  LYEYYQRWGEDDAEAALTVPESVRNLPTGLLGAHPSVDEQLIDDEQVEYL  500

                   510       520       530       540       550
1_StGLOD    501  RNSELRGGVRPATNAYGGGSTTDNPNRFMYYPSHPVPGTQGGVVLAAYSW  550
58_M7GLOD   501  RNSTLRGGVRPATQVHGGGSTTDNPNRFMYYPSHAVPGSKGGVVLAAYSW  550

                   560       570       580       590       600
1_StGLOD    551  SDDAARWDSFDDAERYGYALENLQSVHGRRIEVFYTGAGQTQSWLRDPYA  600
58_M7GLOD   551  SDDAARWDSFDDAERYGYALENLQSVHGRRIEVFYTGAGQTQSWLRDPYA  600

                   610       620       630       640       650
1_StGLOD    601  CGEAAVYTPHQMTAFHLDVVRPEGPVYFAGEHVSLKHAWIEGAVETAVRA  650
58_M7GLOD   601  CGEAAVYTPHQMTSFHLDVVRPEGPVYFAGEHVSLKHAWIEGAVETAVRA  650

                   660       670       680       690
1_StGLOD    651  AIAVNEAPVG-DTGVTAAAG--RRGAAAATEPMREEALTS  687
58_M7GLOD   651  AIAVNEAPVPYDTAAARAEAPRERAGTASATRTREKAVTS  690
```

Fig. 2

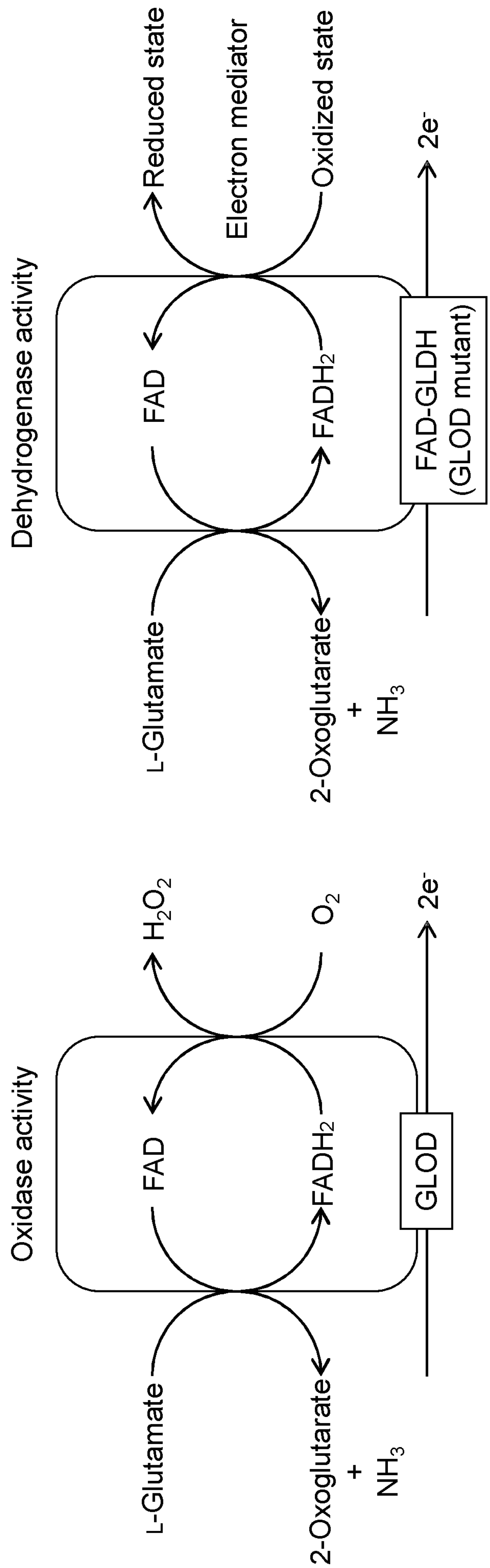
Oxidase activity
L-Glutamate
2-Oxoglutarate
+
$NH_3$
FAD
$FADH_2$
$H_2O_2$
$O_2$
GLOD
2e-
Dehydrogenase activity
L-Glutamate
2-Oxoglutarate
+
$NH_3$
FAD
$FADH_2$
Reduced state
Electron mediator
Oxidized state
FAD-GLDH
(GLOD mutant)
2e-

Fig. 3

MDDKTYQQLARELLLVGPEPANEDLKLRYLDVLIDNGLEPPVDRKRILIVGAGIAGLVAG
HLLTRAGHDVTILEANANRVGGRIKTFHAKKGEPAPFTDPAQYAEAGAMRLPSFHPLTLA
LIDKLGLKRRLFFNVDIDPKTGNQGAALPPVVYKSFKDGKTWTYGKPSPEFREPDKRNHT
WIRTNRTQVRRAQYVKDPSAINEGFHLTGCESRLTVSDMVNQALEPVRDYYSVLQSDGRR
VNKPFKEWLDGWAGVIRDFDGFSMGRFLREYAGFSDEAVEAIGTIENMTSRLHLAFFHSF
LGRSDIDPSATYWEIEGGSRQLPEALAKDLRDQIVMGQRMVRLEYYDPGRDGHHGGLAGP
SGPAVAIETVPENEP - SAEPQTWTADLAIVTVPFSSLRFVAVTPPFSYKKRRAVIETH
YDQATKVLLEFSRRWWEFTEEDWKRELDAIAPGLYEYYQRWGEDDAEAA - LTVPESVR
NLPTGLLGAHPSVDEQLIDDEQVEYLRNSTLR - GGVRPATQVHGGGSTTDNPNRFMYY
PSHAVPGSKGGVVLAAYSWSDDAARWDSFDDAERYGYALENLQSVHGRRIEVFYTGAGQT
QSWLRDPYACGEAAVYTPHQMTSFHLDVVRPEGPVYFAGEHVSLKHAWIEGAVETAVRAA
IAVNEAPVPYDTAAARAEA - PRERAGTASATRTREKAVTS

## INTERNATIONAL SEARCH REPORT

International application No.
**PCT/JP2023/038908**

### A. CLASSIFICATION OF SUBJECT MATTER

***C12N 15/53***(2006.01)i; ***C12M 1/40***(2006.01)i; ***C12N 1/15***(2006.01)i; ***C12N 1/19***(2006.01)i; ***C12N 1/21***(2006.01)i; ***C12N 5/10***(2006.01)i; ***C12N 9/06***(2006.01)i; ***C12N 11/00***(2006.01)i; ***C12N 15/63***(2006.01)i; ***C12Q 1/00***(2006.01)i; ***C12Q 1/26***(2006.01)i
FI: C12N15/53; C12N11/00; C12N15/63 Z; C12N1/15; C12N1/21; C12N1/19; C12N5/10; C12Q1/00 B; C12Q1/26; C12M1/40 B; C12N9/06 Z ZNA

According to International Patent Classification (IPC) or to both national classification and IPC

### B. FIELDS SEARCHED

Minimum documentation searched (classification system followed by classification symbols)

C12N15/53; C12M1/40; C12N1/15; C12N1/19; C12N1/21; C12N5/10; C12N9/06; C12N11/00; C12N15/63; C12Q1/00; C12Q1/26

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

### C. DOCUMENTS CONSIDERED TO BE RELEVANT

| Category* | Citation of document, with indication, where appropriate, of the relevant passages | Relevant to claim No. |
|---|---|---|
| A | JP 2-163080 A (TOYO JOZO COMPANY, LIMITED) 22 June 1990 (1990-06-22) entire text, all drawings | 1-24 |
| A | JP 2014-153243 A (TANITA CORPORATION) 25 August 2014 (2014-08-25) entire text, all drawings | 1-24 |
| A | CN 109266664 A (NANJING INDUSTRIAL UNIVERSITY) 25 January 2019 (2019-01-25) entire text, all drawings | 1-24 |
| A | WO 2021/193598 A1 (AJINOMOTO INCORPORATED COMPANY) 30 September 2021 (2021-09-30) entire text, all drawings | 1-24 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 December 2023** | **19 December 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2023/038908

C. DOCUMENTS CONSIDERED TO BE RELEVANT

| Category* | Citation of document, with indication, where appropriate, of the relevant passages | Relevant to claim No. |
|---|---|---|
| A | HUGHES, G. et al. The design, development and application of electrochemical glutamate biosensors. Trends in Analytical Chemistry. vol. 79, 2016, pp. 106-113, doi:10.1016/j.trac.2015.10.020<br>entire text, all drawings | 1-24 |
| P, A | WO 2023/140286 A1 (KIKKOMAN CORPORATION) 27 July 2023 (2023-07-27)<br>entire text, all drawings | 1-24 |

Form PCT/ISA/210 (second sheet) (January 2015)

**INTERNATIONAL SEARCH REPORT**

International application No.
**PCT/JP2023/038908**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   "In the form of an Annex C/ST.25 text file" above should be understood as "in ST.26 format".

Form PCT/ISA/210 (continuation of first sheet) (January 2015)

**INTERNATIONAL SEARCH REPORT**
**Information on patent family members**

International application No.
**PCT/JP2023/038908**

| Patent document cited in search report | Publication date (day/month/year) | Patent family member(s) | Publication date (day/month/year) |
| --- | --- | --- | --- |
| JP 2-163080 A | 22 June 1990 | (Family: none) | |
| JP 2014-153243 A | 25 August 2014 | US 2014/0224671 A1<br>entire text, all drawings<br>EP 2765412 A1<br>CN 103983668 A | |
| CN 109266664 A | 25 January 2019 | (Family: none) | |
| WO 2021/193598 A1 | 30 September 2021 | US 2023/0203456 A1<br>entire text, all drawings<br>EP 4130023 A1 | |
| WO 2023/140286 A1 | 27 July 2023 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021193598 A
- JP 2019097420 A, Kokai
- JP 6349452 B
- JP 2022172293 A
- JP 2007222055 A, Kokai
- JP 2011239681 A, Kokai


### Non-patent literature cited in the description


- **KUSAKABE H et al.** *Agric. Biol. Chem*, 1983, vol. 47, 1323-1328
- **ARIMA J et al.** *J. Biochem.*, 2003, vol. 134, 805-812
- **KRAMER**. *Nucleic Acids Res.*, 1984, vol. 12, 9441-9456
- **ECKSTEIN**. *Nucleic Acids Res.*, 1985, vol. 13, 8749-8764
- *Nucleic Acids Res.*, 1985, vol. 13, 8765
- *Nucleic Acids Res*, 1986, vol. 14, 9679
- **KUNKEL**. *Proc. Natl. Acid. Sci. U.S.A.*, 1985, vol. 82, 488-492
- *Mol. Gen. Genet*, 1989, vol. 218, 99-104
- **OZEKI et al.** *Biosci. Biotechnol. Biochem.*, 1995, vol. 59, 1133
- *Chem. Rev.*, 2005, vol. 105 (11), 4056-72
- *Comb Chem High Throughput Screen*, 2008, vol. 11 (2), 127-34
- *Curr Opin Struct Biol*, 2007, vol. 17, 474-80
- **AGRESTI et al.** Ultrahigh-throughput screening in drop-based microfluidics for directed evolution. *Proceedings of the National Academy of Sciences*, March 2010, vol. 107 (9), 4004-4009
- **STRYER et al.** *Biochemistry*, 2002, vol. 5, 44-49
- **S. HEINKOFF** ; **J. G. HENIKOFF**. *Proc. Natl. Acad. Sci. USA*, 1992, vol. 89, 10915-10919
- **THOMPSON**. *Nucleic Acid Research*, 1994, vol. 22 (22), 4673-4680
- **SAMBROOK, J** ; **FRITSCH, E.F** ; **MANIATIS, T**. Molecular Cloning: A Laboratory Manual. Cold Spring Harbor Laboratory Press, 1989
- **AUSUBEL, F.M et al.** Current Protocols in Molecular Biology. John Wiley & Sons, 1995
- **ROE, B.** ; **CRABTREE, J.** ; **KAHN, A** ; **POLAK, J.M** ; **MCGEE, J.O'D**. DNA Isolation and Sequencing: Essential Techniques. John Wiley & Sons, 1996
- Situ Hybridization: Principles and Practice. Oxford University Press, 1990
- **GAIT, M.J**. Oligonucleotide Synthesis: A Practical Approach. IRL Press, 1984
- **LILLEY, D.M** ; **DAHLBERG, J.E**. Methods in Enzymology: DNA Structures Part A: Synthesis and Physical Analysis of DNA. Academic Press, 1992